# FASCICULE DE BREVET EUROPEEN

(11) **EP 2 391 611 B1**
(45) Date de publication et mention de la délivrance du brevet: **30.04.2014**
(21) Numéro de dépôt: 10707597.0
(22) Date de dépôt: 27.01.2010
(51) Int. Cl.: C07D 271/06, C07D 285/12, A61K 31/4245, A61K 31/433

(54) **DÉRIVÉS DE THIADIAZOLES ET D'OXADIAZOLES, LEUR PRÉPARATION ET LEUR APPLICATION EN THÉRAPEUTIQUE**
THIADIAZOL- UND OXADIAZOLDERIVATE, DEREN HERSTELLUNG UND DEREN THERAPEUTISCHE VERWENDUNG
THIADIAZOLE AND OXADIAZOLE DERIVATIVES, PREPARATION THEREOF, AND THERAPEUTIC USE THEREOF

(30) Priorité: 28.01.2009 FR 0900359; 01.09.2009 FR 0904137
(43) Date de publication de la demande: 07.12.2011
(73) Titulaire: SANOFI, 75008 Paris (FR)
(72) Inventeur: FETT, Eykmar, F-75013 Paris (FR); MOUGENOT, Patrick, F-75013 Paris (FR); NAMANE, Claudie, F-75013 Paris (FR); NICOLAI, Eric, F-75013 Paris (FR); PHILIPPO, Christophe, F-75013 Paris (FR)
(74) Mandataire: Löwrick, Oliver
(86) Numéro de dépôt international: PCT/FR2010/050124
(87) Numéro de publication internationale: WO 2010/086551

(56) Documents cités:
- WO-A-2005/065683
- WO-A-2006/134317
- WO-A-2007/115935
- US-A1- 2004 063 700
- US-A1- 2005 143 384

## Description

La présente invention se rapporte à des dérivés de thiadiazoles et d'oxadiazoles, à leur préparation et à leur application en thérapeutique.

Les triacylglycerides représentent la principale forme de stockage de l'énergie chez les eucaryotes, et peuvent être aussi à l'origine de troubles ou de déséquilibre dans le métabolisme des triacylglycérides, impliqués dans la pathogenèse et l'augmentation du risque de plusieurs pathologies tels que l'obésité, la résistance à l'insuline, le diabète de type 2 (Reasner CA. J Cardiovasc Pharmacol. 52:136-44, 2008) et les complications découlant de cette pathologie (Krane et Wanner, Minerva Urol Nefrol. 59(3):299-316, 2007 ; King GL, J Periodontol. 79:1527-34, 2008), la dyslipidemie qui se caractérise par des niveaux élevés de triglycérides plasmatiques, des faibles niveaux de lipoprotéines de haute densité (HDL), et l'apparition de petites et denses lipoprotéines de basse densité (sdLDL) et une lipidémie postprandiale excessive (Ginsberg et al. Obesity (Silver Spring). 14: 41 S-49S, 2006, Adiels et al. Curr Opin Lipidol. 17: 238-246, 2006, Adiels et al. ATVB 28 :1225-1236,2008), les conditions d'altération de glucose à jeun, l'acidose métabolique, la cétose, le syndrome métabolique (Eschwège E. Diabetes Metab. 29:6S19-27, 2003), la stéatose hépatique (Parekh et Anania, Gastroenterology 132:2191-2207, 2007), les maladies coronariennes (Lewis, et al. Endocrine Review 23:701, 2002 ; Ridker et Silvertown, J Periodontol. 79:1544-51, 2008 ; McBride P. Curr Atheroscler Rep.10:386-90, 2008), les maladies de la peau (Chen et al. J Clin Invest. 109:175-81, 2002 ; Yosipovitch et al. J Am Acad Dermatol. 56:901-16, 2007), la maladie d'Alzheimer, différents maladies immunomodulatrices (Pahan K. Cell Mol Life Sci. 63:1165-78, 2006), l'infection par le VIH (Kotler DP. J Acquir Immune Defic Syndr. 49:S79-85 2008), le syndrome inflammatoire de l'intestin (Schäffler et al. Nat Clin Pract Gastroenterol Hepatol. 2:103-11, 2005). Le stockage en excès des triacylglycérides dans les tissus maigres, tels que le foie, les muscles, et d'autres tissus périphériques, conduit à un dysfonctionnement dans ces tissus; alors que la réduction de l'accumulation de ces graisses dans ces tissus périphériques semble être bénéfique dans le traitement de la lipotoxicité (Unger, Endocrinology, 144: 5 159-5 165, 2003). L'accumulation et l'excès de triacylglycérides dans le tissus adipeux (WAT) conduit à l'obésité, une condition qui est associée à une diminution de la durée de vie, du diabète de type II, des maladies coronariennes, de l'hypertension, des accidents vasculaires cérébraux, et le développement de certains cancers (Grundy, Endocrine 13 (2): 155-165, 2000).

Le document US2005/143384 décrit des composés qui inhibent l'activité de la inhibiteur de l'activateur du plasminogène (PAI-1) et qui sont donc utiles dans le traitement des troubles thromboemboliques.

Le document WO2006/134317 décrit des composés qui inhibent l'activité de la diacylglycerol acyltransferase (DGAT1) et qui sont donc utiles dans le traitement des troubles ou de déséquilibre dans le métabolisme des triglycérides.

La diacylglycerol acyltransferase (DGAT), catalyse la formation des triglycérides à partir du diacylglycerol et de l'acyl-CoA dans les cellules animales ou humaines, les triglycérides représentant la principale forme de stockage de l'énergie.

L'obésité, le diabète de type 2 et leurs complications sont des pathologies très répandues dans la société moderne et actuellement les options de traitement pharmacologique sont limitées, d'où la nécessité de développer des agents pharmaceutiques pour le traitement, pour la prévention, le retard ou le traitement des troubles liés à l'obésité, au diabète de type 2 et leurs complications, qui soient sûrs et efficaces.

Les composés de l'invention inhibent la biosynthèse des triglycérides et sont utiles pour le traitement des pathologies dans lesquelles une telle inhibition est bénéfique, comme dans le cas de l'obésité, de la dyslipidémie, de la stéatose hépatique, du diabète de type 2, du syndrome métabolique et des maladies coronariennes.

La présente invention a pour objet des composés répondant à la formule (I) 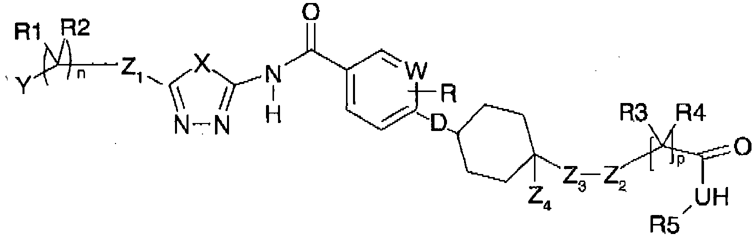 dans laquelle
- **U** représente un atome d'oxygène ou un atome d'azote, sachant que lorsque **U** représente un atome d'oxygène alors **R5** est absent ;
- **n** est égale à 1, 2 ou 3 ;
- **p** est égal à 0, 1 ou 2 ;
- **D** représente un atome d'oxygène, un groupe -NH- ou une liaison ;
- **W** représente un atome de carbone ou d'azote ;
- **X** représente un hétéroatome choisi parmi un atome d'oxygène ou un atome de soufre ;
- **R1, R2, R3 et R4** représentent, indépendamment l'un de l'autre,
   o un atome d'hydrogène,
   o un groupe -(C1-C6)alkyle ou bien,
   o (i) R1 et R2 peuvent former avec l'atome de carbone auquel ils sont rattachés un groupe -(C3-C10)cycloalkyle- et/ou (ii) R3 et R4 peuvent former avec l'atome de carbone auquel ils sont rattachés un groupe - (C3-C10)cycloalkyle- ;
- **Y** représente un atome d'hydrogène, un groupe -(C1-C6)alkyle, -(C3-C10)cycloalkyle-, (C3-C10)cycloalkyloxy-, (C3-C10)cycloalkyl-(C1-C6)alkyloxy-, heterocycloalkyle-(C1-C6)alkyloxy-, un groupe -COOR1, aryle, arylalkyle, hétéroaryle, hétérocycloalkyle, aryloxy, -C(O)-hétérocycloalkyle, -C(O)aryle, -CH(OH)aryle, -NH-cycloalkyle lesdits groupes étant éventuellement substitués par un ou plusieurs substituants choisis parmi un atome d'halogène, un groupe hydroxyl, un groupe (C1-C6) alkyle, (C1-C6) alcoxy, hétérocycloalkyle ou un groupe aryloxy ;
- **R** représente un atome d'hydrogène ou d'halogène ;
- **Z1** est absent ou représente un atome de soufre, une fonction-NH-, -NHC(O)-, un groupe -S(O)-CH₂-, -SCH₂-, méthylène ou un groupe éthylène ;
- **Z2** est absent ou représente un groupe méthylène, un groupe 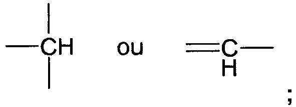
- **Z3** est absent ou représente un atome d'oxygène ou un groupe méthylène, un groupe 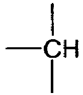 ou 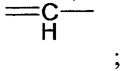 Sachant que Z2 ne représente un groupe 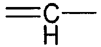 que lorsque **Z3** est présent et qu'il représente un groupe 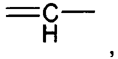 et réciproquement, **Z2 et Z3** formant ainsi une double liaison ;
   Sachant que **Z2** et **Z3,** lorsqu'ils sont présents, peuvent être compris dans un groupe cycloalkyle ;
- **Z4** est
   o un atome d'hydrogène,
   o un atome de carbone formant éventuellement avec Z3 un groupe -(C3-C10)cycloalkyle- lorsque Z3 est un groupe 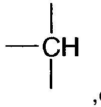 ou
   ○ est absent, Z3 étant alors un groupe 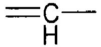 formant une double liaison avec le carbone du cyclohexyle qui lui est adjacent;
- **R5** représente un atome d'hydrogène ou un groupe alkyle éventuellement substitué par au moins un groupe hydroxyle, hétérocycloalkyle-(C1-C6)alkyle, amine ou alkyloxy,
sous forme d'acide, de base ou de sel d'addition à un acide ou à une base.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Dans les composés de formule (I), les substituants portés par le groupe cyclohexyle peuvent être en position cis ou trans. Les composés de formule (I) peuvent donc exister sous forme d'isomères de position tel que définis précédemment. Ces isomères de position ainsi que leur mélange font partie de l'invention.

Les composés de formule (I) peuvent exister à l'état de bases ou d'acides ou salifiés par des acides ou des bases, notamment des acides ou des bases pharmaceutiquement acceptables. De tels sels d'addition font partie de l'invention.

On entend par base pharmaceutiquement acceptable, par exemple, l'hydroxyde de sodium ou de potassium, la choline, la lysine ou l'arginine.

On entend par acide pharmaceutiquement acceptable, par exemple, l'acide chlorhydrique ou l'acide sulfurique.

Ces sels sont avantageusement préparés avec des bases pharmaceutiquement acceptables, mais les sels d'autres bases utiles, par exemple, pour la purification ou l'isolement des composés de formule (I), font également partie de l'invention.

Dans le cadre de la présente invention, et sauf mention différente dans le texte, on entend par :
- un atome d'halogène : un atome de fluor, de chlore, de brome ou d'iode ;
- un groupe alkyle: un groupe aliphatique saturé, linéaire ou ramifié, pouvant comporter 1, 2, 3, 4, 5 ou 6 atomes de carbone (abrégé par *-(C1-C6)alkyle).* A titre d'exemples, on peut citer comme (i) groupe *-C1alkyle,* le groupe méthyle, comme (ii) groupe *-C2alkyle,* le groupe éthyle, comme (iii) groupe *-C3alkyle,* le groupe propyle, isopropyle, comme (iv) groupe *-C4alkyle,* le groupe butyle, isobutyle, tertbutyle, comme (v) groupe *-C5alkyle,* le groupe pentyle, isopentyle, (vi) comme groupe *-C6alkyle,* le groupe hexyle;
- un groupe alkylène : un groupe alkyle tel que précédemment défini, divalent saturé, linéaire ou ramifié, pouvant comporter 1, 2, 3, 4, 5 ou 6 atomes de carbone (abrégé *-(C1-C6)alkylène-).* A titre d'exemple, on peut citer les radicaux méthylène (ou -CH₂-), éthylène (ou -CH₂-CH₂-), propylène (-CH₂-CH₂-CH₂-);
- un groupe cycloalkyle : un groupe alkyle cyclique pouvant comporter 3, 4, 5, 6, 7 , 8, 9 ou 10 atomes de carbone, abrégé également *-(C3-C10)cycloalkyle.* A titre d'exemples, on peut citer les groupes cyclopropyle, méthylcyclopropyle, cyclobutyle, cyclopentyle, cyclohexyle, adamantyle, pentalène ;
- groupe cycloalkyloxy: un radical de formule -O-cycloalkyle, où le groupe cycloalkyle est tel que défini précédemment ;
- un groupe alcoxy ou alkyloxy : un radical *-O-alkyle* où le groupe alkyle est tel que précédemment défini. A titre d'exemples, on peut citer les groupes -O-*(C1-C5)alkyle* ou *-(C1-C5)alcoxy,* et en particulier comme (i) groupe *-O-C1alkyle,* le groupe -Ométhyle, comme (ii) groupe *-O-C2alkyle,* le groupe -Oéthyle, comme (iii) groupe *-O-C3alkyle,* le groupe -Opropyle, -Oisopropyle, comme (iv) groupe -*O-C4alkyle,* le groupe -Obutyle, -Oisobutyle, -Otertbutyle, comme (v) groupe *-O-C5alkyle* le groupe -Opentyle, -Oisopentyle;
- groupe cycloalkyl-alcoxy ou cycloalkyl-alkyloxy : un radical de formule cycloalkyl-alkylène-O-, où le groupe cycloalkyle et alkylène sont tels que définis précédemment ;
- groupe heterocycloalkyl-alcoxy ou heterocycloalkyl-alkylon: un radical de formule heterocycloalkyl-alkylène-O-, où le groupe heterocycloalkyle et alkylène sont tels que définis ci-dessus et ci-dessous ;
- un hétérocycloalkyle-alkyle : un groupe hétérocycloalkyle-alkylène-, où les groupes heterocycloalkyle et alkylène sont tels que définis ci-dessus et ci-dessous ;
- un groupe alcoxy-alkyle : un radical de formule *-alkylène-O-alkyle,* où les groupes alkyle et alkylène, comprenant le même nombre de carbone ou ne comprenant pas le même nombre de carbone, sont tels que définis précédemment. A titre d'exemples, on peut citer les groupes *-(C1-C6)alkylène-O-(C1-C6)alkyle,* avec - (C1-C6)alkylène- et -(C1-C6)alkyle tels que définis ci-dessus ;
- un groupe halogénoalkyle : un groupe alkyle tel que défini ci-dessus substitué par 1, 2, 3, 4 ou 5 atomes d'halogène, tels que définis précédemment. On citera par exemple les groupes *-halogéno(C1-C5)alkyle,* avec (C1-C5)alkyle tel que défini ci-dessus, et en particulier le groupe trifluorométhyle (abrégé -CF₃) ;
- un groupe aryle : un groupe aromatique cyclique comprenant 6, 7, 8, 9 ou 10 atomes de carbone. A titre d'exemples de groupes aryles, on peut citer le groupe phényle(abrégé Ph) ou un groupe naphtyle ;
- un groupe arylalkyle : un groupe aryle, tel que défini ci-dessus, substitué par au moins un groupe alkyle, tel que défini ci-dessus. Avantageusement, il s'agit de radicaux -alkylène-aryle. On peut citer, par exemple, le benzyle c'est-à-dire le radical - CH₂-Ph ;
- un groupe aryloxy : un radical de formule -O-aryle, où le groupe aryle est tel que défini précédemment ;
- un groupe hétéroaryle : un groupe aromatique cyclique comprenant 2, 3, 4 ou 5 atomes de carbone et comprenant 1 à 3 hétéroatomes, qui peu(ven)t être choisi(s) parmi l'atome d'azote, l'atome d'oxygène et l'atome de soufre, indépendemment l'un de l'autre, de manière à être identiques ou différents, lorsqu'ils sont au nombre de 2 ou indépendemment les uns des autres, de manière à être identiques ou différents, lorsqu'ils sont au nombre de 3. On peut citer les groupes pyridyle, pyrrole, furanyle ;
- un hétérocycloalkyle : un groupe alkyle cyclique, éventuellement ponté, comprenant 5, 6 ou 7 atomes de carbone et comprenant 1, 2 ou 3 hétéroatomes qui peu(ven)t être choisi(s), indépendemment l'un de l'autre, de manière à être identiques ou différents, lorsqu'ils sont au nombre de 2 ou indépendemment les uns des autres, de manière à être identiques ou différents, lorsqu'ils sont au nombre de 3, parmi l'atome d'azote, l'atome d'oxygène et l'atome de soufre. On peut notamment citer les groupes pipéridinyle, pipérazinyle, pyrrolidinyle, hexaméthylèneimino, tétrahydrofuranyle, morpholinyle, 1,1-dioxydotetrahydrothiényle;
- les lettres α, β, γ et δ: les positions des différents atomes de carbone autour de la pyridine, lorsque W représente un atome d'azote dans les composés de formule (I). Ces lettres permettent d'identifier les positions des différents atomes de carbone.
- groupe protecteur, tel qu'un groupe R' ou R" dans ce qui suit, un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York). On peut citer comme groupes protecteurs les groupes (C1-C6)alkyle, par exemple, un groupe benzyle, méthyle, éthyle ou tert-butyle.
- groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Parmi les composés de formule (I) objets de l'invention, on peut citer un groupe dans lesquels :
- **U** représente un atome d'oxygène ou un atome d'azote, sachant que lorsque **U** représente un atome d'oxygène alors R5 est absent ;
   et/ou
- **n** est égale à 1, 2 ou 3 ;
   et/ou
- **p** est égal à 0, 1 ou 2 ;
   et/ou
- **D** représente un atome d'oxygène, un groupe -NH- ou une liaison ;
   et/ou
- **W** représente un atome de carbone ou d'azote ;
   et/ou
- **X** représente un hétéroatome choisi parmi un atome d'oxygène ou un atome de soufre ;
   et/ou
- **R1, R2, R3 et R4** représentent, indépendamment l'un de l'autre,
   o un atome d'hydrogène,
   o un groupe -(C1-C6)alkyle ou bien,
   o (i) R1 et R2 peuvent former avec l'atome de carbone auquel ils sont rattachés un groupe -(C3-C10)cycloalkyle- et/ou (ii) R3 et R4 peuvent former avec l'atome de carbone auquel ils sont rattachés un groupe - (C3-C10)cycloalkyle- ;
   et/ou
- **Y** représente un atome d'hydrogène, un groupe -(C1-C6)alkyle, -(C3-C10)cycloalkyle-, (C3-C10)cycloalkyloxy-, (C3-C10)cycloalkyl-(C1-C6)alkyloxy-, heterocycloalkyle-(C1-C6)alkyloxy-, un groupe -COOR1, aryle, arylalkyle, hétéroaryle, hétérocycloalkyle, aryloxy, -C(O)-hétérocycloalkyle, -C(O)aryle, -CH(OH)aryle, -NH-cycloalkyle lesdits groupes étant éventuellement substitués par un ou plusieurs substituants choisis parmi un atome d'halogène, un groupe hydroxyl, un groupe (C1-C6) alkyle, (C1-C6) alcoxy, hétérocycloalkyle ou un groupe aryloxy ;
   et/ou
- **R** représente un atome d'hydrogène ou d'halogène ;
   et/ou
- **Z1** est absent ou représente un atome de soufre, une fonction-NH-, -NHC(O)-, un groupe -S(O)-CH₂-, -SCH₂-, méthylène ou un groupe éthylène ;
   et/ou
- Z2 est absent ou représente un groupe méthylène, un groupe 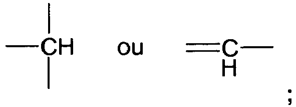 et/ou
- **Z3** est absent ou représente un atome d'oxygène ou un groupe méthylène, un groupe 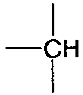 ou 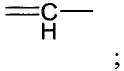 et/ou
- **Z2** et **Z3** sont présents et peuvent représenter chacun un groupe 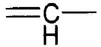 et forment ainsi une double liaison ;
   et/ou
- **Z2** et **Z3** sont présents et peuvent être compris dans un groupe cycloalkyle ;
   et/ou
- **Z4** est
   o un atome d'hydrogène,
   o un atome de carbone formant éventuellement avec Z3 un groupe -(C3-C10)cycloalkyle- lorsque Z3 est un groupe 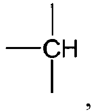
   ou o est absent, Z3 étant alors un groupe 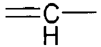 formant une double liaison avec le carbone du cyclohexyle qui lui est adjacent;
   et/ou
- **R5** représente un atome d'hydrogène ou un groupe alkyle éventuellement substitué par au moins un groupe hydroxyle, heterocycloalkyle-(C1-C6)alkyle, amine ou alkyloxy,
   et/ou
- ledit composé (I) se présente sous forme d'acide, de base ou de sel d'addition à un acide ou à une base.

Parmi les composés de formule (I) objets de l'invention, on peut citer un groupe de composés constitué par les composés répondant à la formule (I') suivante :

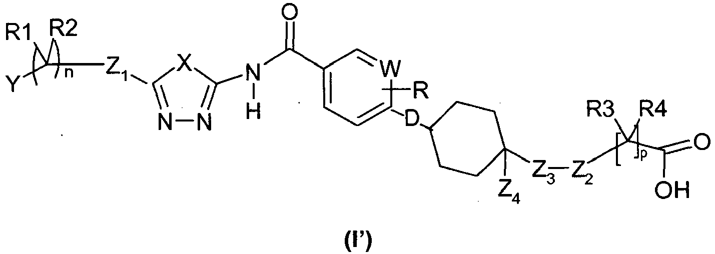

Dans laquelle **Y, R1, R2, n, Z1, X, W, R, D, Z4, Z3, Z2, R3, R4 et p** sont tels que définis ci-dessus.

Parmi les composés de formule (I) objets de l'invention, on peut citer un autre groupe de composés constitué par les composés répondant à la formule (I") suivante :

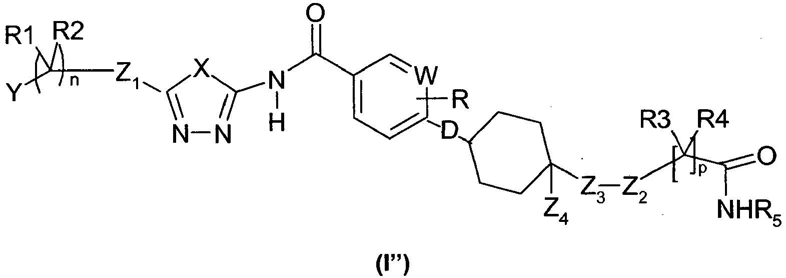

Dans laquelle **Y, R1, R2, n, Z1, X, W, R, D, Z4, Z3, Z2, R3, R4, p et R5** sont tels que définis ci-dessus.

Parmi les composés de formule (I) objets de l'invention, on peut citer un autre groupe de composés dans lesquels :
D représente une liaison ou un atome d'oxygène ;
   et/ou
p est égale à 0 ;
   et/ou
   - **Z3** et **Z2** représentent chacun un méthylène, ou
   - **Z3** représent un méthylène et **Z2** est absent, ou
   - **Z3** et **Z2** sont absents, ou
   - **Z3** et **Z2** sont compris dans un groupe cycloalkyle, avantageusement un cyclopropyle, ou
   - **Z3** et **Z2** forment ensemble une double liaison ;
et/ou
**W** représente un atome de carbone ou un atome d'azote ;
et/ou
**X** représente un atome de soufre.

Parmi les composés de formule (I) objets de l'invention, on peut citer un autre groupe de composés dans lesquels **D** représente un groupe NH.

Parmi les composés de formule (I) objets de l'invention, on peut citer un autre groupe de composés dans lesquels **X** représente un atome d'oxygène.

Parmi les composés de formule (I) objets de l'invention, on peut citer un autre groupe de composés dans lesquels **R1, R2, R3 et R4** représentent indépendamment l'un de l'autre un atome d'hydrogène.

Parmi les composés de formule (I) objets de l'invention, on peut citer un autre groupe de composés dans lesquels **R1** et **R2** et/ou **R3** et **R4** forment un groupe cycloalkyle. Avantageusement, **R1 et R2** et/ou **R3 et R4** forment un groupe cyclopropyle ou cyclobutyle.

Parmi les composés de formule (I) objets de l'invention, on peut citer un autre groupe de composés dans lesquels **Y** est un groupe aryle. Avantageusement, **Y** représente un groupe phényle.

Parmi les composés de formule (I) objets de l'invention, on peut citer un autre groupe de composés dans lesquels **Y** est un groupe hétéroaryle. Avantageusement, **Y** représente un groupe pyridinyle.

Parmi les composés de formule (I) objets de l'invention, on peut citer un autre groupe de composés dans lesquels **Y** est un groupe aryloxy. Avantageusement, **Y** représente un groupe phénoxy.

Parmi les composés de formule (I) objets de l'invention, on peut citer un autre groupe de composés dans lesquels **Y** est un groupe cycloalkyle. Avantageusement, **Y** représente un groupe cyclopentyle, adamantyle ou pentalène.

Parmi les composés de formule (I) objets de l'invention, on peut citer un autre groupe de composés dans lesquels **D** est une liaison et **p** est égal à 1.

Parmi les composés de formule (I) objets de l'invention, on peut citer un autre groupe de composés dans lesquels **D** est un atome d'oxygène et p est égal à 0.

Parmi les composés de formule (I) objets de l'invention, on peut citer un autre groupe de composés dans lesquels **D** est un groupe NH et **p** est égal à 0.

Parmi les composés de formule (I) objets de l'invention, on peut citer un autre groupe de composés dans lesquels **D** est une liaison et **p** est égal à 2.

Parmi les composés de formule (I) objets de l'invention, on peut citer un autre groupe de composés dans lesquels **Z1** est un atome de soufre.

Parmi les composés de formule (I) objets de l'invention, on peut citer un autre groupe de composés dans lesquels **Z3** est un atome d'oxygène.

Parmi les composés de formule (I) objets de l'invention, on peut notamment citer les composés suivants :
- acide {4-[4-(5-benzyl-[1,3,4]thiadiazol-2-ylcarbamoyl)-phényl]-cyclohexyl}-acétique
- acide (4-{4-[5-(4-méthyl-benzyl)-[1,3,4]thiadiazol-2-ylcarbamoyl]-phényl}-cyclohexyl)-acétique
- acide (4-{4-[5-(2-fluoro-benzyl)-[1,3,4]thiadiazol-2-ylcarbamoyl]-phényl}-cyclohexyl)-acétique
- acide (4-{4-[5-(3-fluoro-benzyl)-[1,3,4]thiadiazol-2-ylcarbamoyl]-phényl}-cyclohexyl)-acétique
- acide (4-{4-[5-(4-fluoro-benzyl)-[1,3,4]thiadiazol-2-ylcarbamoyl]-phényl}-cyclohexyl)-acétique
- acide (4-{4-[5-(2,4,5-trifluoro-benzyl)-[1,3,4]thiadiazol-2-ylcarbamoyl]-phényl}-cyclohexyl)-acétique
- acide [4-(4-{5-[1-(phényl)-cyclopropyl]-[1,3,4]thiadiazol-2-ylcarbamoyl}-phényl)-cyclohexyl]-acétique
- acide [4-(4-{5-[1-(4-fluoro-phényl)-cyclopropyl]-[1,3,4]thiadiazol-2-ylcarbamoyl}-phényl)-cyclohexyl]-acétique
- acide [4-(4-{5-[1-(3-fluoro-phényl)-cyclobutyl]-[1,3,4]thiadiazol-2-ylcarbamoyl}-phényl)-cyclohexyl]-acétique
- acide (4-{4-[5-(4-chloro-benzyl)-[1,3,4]thiadiazol-2-ylcarbamoyl]-phényl}-cyclohexyl)-acétique
- acide (4-{4-[5-(3-chloro-benzyl)-[1,3,4]thiadiazol-2-ylcarbamoyl]-phényl}-cyclohexyl)-acétique
- acide (4-{4-[5-(2-chloro-benzyl)-[1,3,4]thiadiazol-2-ylcarbamoyl]-phényl}-cyclohexyl)-acétique
- acide (4-{4-[5-(4-méthoxy-benzyl)-[1,3,4]thiadiazol-2-ylcarbamoyl]-phényl}-cyclohexyl)-acétique
- acide {4-[4-(5-cyclopentylméthyl-[1,3,4]thiadiazol-2-ylcarbamoyl)-phényl]-cyclohexyl}-acétique
- acide (4-{4-[5-(2-cyclopentyl-éthyl)-[1,3,4]thiadiazol-2-ylcarbamoyl]-phényl}-cyclohexyl)-acétique
- acide {4-[4-(5-phénéthyl-[1,3,4]thiadiazol-2-ylcarbamoyl)-phényl]-cyclohexyl}-acetique
- acide [4-(4-{5-[2-(4-fluoro-phényl)-éthyl]-[1,3,4]thiadiazol-2-ylcarbamoyl}-phényl)-cyclohexyl]-acétique
- acide {4-[4-(5-phénoxyméthyl-[1,3,4]thiadiazol-2-ylcarbamoyl)-phényl]-cyclohexyl}-acetique
- acide [4-(4-{5-[3-(4-fluoro-phényl)-propyl]-[1,3,4]thiadiazol-2-ylcarbamoyl}-phényl)-cyclohexyl]-acétique
- acide (4-{4-[5-(4-fluoro-phénoxyméthyl)-[1,3,4]thiadiazol-2-ylcarbamoyl]-phényl}-cyclohexyl)-acétique
- acide (4-{4-[5-(benzyl)-[1,3,4]oxadiazol-2-ylcarbamoyl]-phényl}-cyclohexyl)-acétique
- acide (4-{4-[5-(4-fluoro-benzyl)-[1,3,4]oxadiazol-2-ylcarbamoyl]-phényl}-cyclohexyl)-acétique
- acide cis-4-[4-(5-benzyl-[1,3,4]thiadiazol-2-ylcarbamoyl)-phénoxy]-cyclohexane-carboxylique
- acide trans-4-[4-(5-benzyl-[1,3,4]thiadiazol-2-ylcarbamoyl)-phénoxy]-cyclohexane-carboxylique
- acide cis-4-[4-(5-benzyl-[1,3,4]thiadiazol-2-ylcarbamoyl)-2-fluoro-phénoxy]-cyclohexanecarboxylique
- acide cis-4-[4-(5-benzyl-[1,3,4]thiadiazol-2-ylcarbamoyl)-2-chloro-phénoxy]-cyclohexanecarboxylique
- acide cis-4-{4-[5-(2-cyclopentyl-éthyl)-[1,3,4]thiadiazol-2-ylcarbamoyl]-phénoxy}-cyclohexanecarboxylique
- acide cis-4-[5-(5-benzyl-[1,3,4]thiadiazol-2-ylcarbamoyl)-pyridin-2-yloxy]-cyclohexanecarboxylique
- acide cis-4-{5-[5-(3-chloro-benzyl)-[1,3,4]thiadiazol-2-ylcarbamoyl]-pyridin-2-yloxy}-cyclohexanecarboxylique
- acide cis-4-[5-(5-phénéthyl-[1,3,4]thiadiazol-2-ylcarbamoyl)-pyridin-2-yloxy]-cyclohexanecarboxylique
- acide cis-4-[4-(5-benzyl-[1,3,4]thiadiazol-2-ylcarbamoyl)-phénylamino]-cyclohexane-carboxylique
- acide trans-4-[4-(5-benzyl-[1,3,4]thiadiazol-2-ylcarbamoyl)-phénylamino]-cyclohexane-carboxylique
- acide trans-{4-[4-(5-benzyl-[1,3,4]thiadiazol-2-ylcarbamoyl)-phényl]-cyclohexyl}-acétique
- acide trans-4-[4-(5-benzyl-[1,3,4]thiadiazol-2-ylcarbamoyl)-phényl]-cyclohexane-carboxylique
- acide trans-{4-[4-(5-cyclopentyloxyméthyl-[1,3,4]thiadiazol-2-ylcarbamoyl)-phényl]-cyclohexyl}-acétique
- acide trans-[4-(4-{5-[2-(tetrahydro-furan-2-yl)-éthyl]-[1,3,4]thiadiazol-2-ylcarbamoyl}-phényl)-cyclohexyl]-acétique
- acide trans-(4-{4-[5-(2-cyclohexyl-éthyl)-[1,3,4]thiadiazol-2-ylcarbamoyl]-phényl}-cyclohexyl)-acétique
- acide trans-{4-[4-(5-cyclopentylméthoxyméthyl-[1,3,4]thiadiazol-2-ylcarbamoyl)-phényl]-cyclohexyl}-acétique
- acide trans-{4-[4-(5-benzylsulfanyl-[1,3,4]thiadiazol-2-ylcarbamoyl)-phényl]-cyclohexyl}-acétique
- acide {4-[4-(5-benzyl-[1,3,4]thiadiazol-2-ylcarbamoyl)-phényl]-cyclohexylidène}-acétique
- acide 6-[4-(5-benzyl-[1,3,4]thiadiazol-2-ylcarbamoyl)-phényl]-spiro[2.5]octane-1-carboxylique
- acide (E)-3-{4-[4-(5-benzyl-[1,3,4]thiadiazol-2-ylcarbamoyl)-phényl]-cyclohexyl}-acrylique
- acide trans-(1 R,25/1S,2R)-2-{4-[4-(5-benzyl-[1,3,4]thiadiazol-2-ylcarbamoyl)-phényl]-cyclohexyl}-cyclopropanecarboxylique
- acide trans-3-{4-[4-(5-benzyl-[1,3,4]thiadiazol-2-ylcarbamoyl)-phényl]-cyclohexyl}-propionique
- acide (4-{4-[5-((1S,3S/1R,3R)-3-phénoxy-cyclohexyl)-[1,3,4]thiadiazol-2-ylcarbamoyl]-phényl}-cyclohexyl)-acétique
- acide trans-(4-{4-[(5-{[(3R,6S/3S,6R)-5-éthoxyoctahydropentalèn-2-yl]méthyl}-1,3,4-thiadiazol-2-yl)carbamoyl]phényl}cyclohexyl)acétique
- acide trans-{4-[4-(5-benzyl-[1,3,4]thiadiazol-2-ylcarbamoyl)-phényl]-cyclohexyloxy}-acétique
- acide trans-(4-{4-[5-(2-morpholin-4-yl-éthyl)-[1,3,4]thiadiazol-2-ylcarbamoyl]-phényl}-cyclohexyl)-acétique
- acide trans-(4-{4-[5-(2-oxo-2-pyrrolidin-1-yl-éthyl)-[1,3,4]thiadiazol-2-ylcarbamoyl]-phényl}-cyclohexyl)-acétique
- acide cis-4-[4-(5-cyclopentyloxyméthyl-[1,3,4]thiadiazol-2-ylcarbamoyl)-phénoxy]-cyclohexanecarboxylique.
- acide trans-[4-(4-{5-[2-(tetrahydro-furan-3-yl)-éthyl]-[1,3,4]thiadiazol-2-ylcarbamoyl}-phényl)-cyclohexyl]-acétique
- acide trans-(4-{4-[5-(3-phényl-cyclobutyl)-[1,3,4]thiadiazol-2-ylcarbamoyl]-phényl}-cyclohexyl)-acétique
- acide {4-[4-(5-Phenylacetylamino-[1,3,4]thiadiazol-2-ylcarbamoyl)-phenyl]-cyclohexyl}-acetique
- acide trans-{4-[4-(5-cyclopentyloxyméthyl-[1,3,4]thiadiazol-2-ylcarbamoyl)-phényl]-cyclohexyl}-acétique
- acide trans-(4-{4-[5-(3,5-difluoro-benzyl)-[1,3,4]thiadiazol-2-ylcarbamoyl]-phényl}-cyclohexyl)-acétique
- acide trans-(4-{4-[5-(4-hydroxy-cyclohexylméthyl)-[1,3,4]thiadiazol-2-ylcarbamoyl]-phényl}-cyclohexyl)-acétique
- acide trans-(4-{4-[5-(tetrahydro-furan-2-ylméthoxyméthyl)-[1,3,4]thiadiazol-2-ylcarbamoyl]-phényl}-cyclohexyl)-acétique
- acide trans-{4-[4-(5-benzyl-[1,3,4]thiadiazol-2-ylcarbamoyl)-phénoxy]-cyclohexyl}-acétique
- acide trans-4-{4-[5-(tetrahydro-furan-2-ylméthoxyméthyl)-[1,3,4]thiadiazol-2-ylcarbamoyl]-phénoxy}-cyclohexanecarboxylique
- acide trans-(4-{4-[5-(3-oxo-3-phényl-propyl)-[1,3,4]thiadiazol-2-ylcarbamoyl]-phényl)-cyclohexyl)-acétique
- acide trans-(4-{4-[5-(3-hydroxy-3-phényl-propyl)-[1,3,4]thiadiazol-2-ylcarbamoyl]-phényl}-cyclohexyl)-acétique
- trans-N-(5-benzyl-[1,3,4]thiadiazol-2-yl)-4-{4-[(2-hydroxy-2-méthyl-propylcarbamoyl)-méthyl]-cyclohexyl}-benzamide
- trans-N-(5-benzyl-[1,3,4]thiadiazol-2-yl)-4-(4-carbamoylméthyl-cyclohexyl)-benzamide
- trans-N-(5-benzyl-[1,3,4]thiadiazol-2-yl)-4-{4-[(2,3-dihydroxy-propylcarbamoyl)-méthyl]-cyclohexyl}-benzamide
- trans-N-(5-benzyl-[1,3,4]thiadiazol-2-yl)-4-{4-[(2-morpholin-4-yl-éthylcarbamoyl)-méthyl]-cyclohexyl}-benzamide
- trans-N-(5-benzyl-[1,3,4]thiadiazol-2-yl)-4-{4-[(2-diméthylamino-éthylcarbamoyl)-méthyl]-cyclohexyl}-benzamide
- trans-N-(5-benzyl-[1,3,4]thiadiazol-2-yl)-4-{4-[(2-méthoxy-éthylcarbamoyl)-méthyl]-cyclohexyl}-benzamide
- trans-N-(5-benzyl-[1,3,4]thiadiazol-2-yl)-4-(4-{[([1,4]dioxan-2-ylméthyl)-carbamoyl]-méthyl}-cyclohexyl)-benzamide
- acide trans-4-{4-[5-(3,5-difluoro-benzyl)-[1,3,4]thiadiazol-2-ylcarbamoyl]-phénoxy)-cyclohexanecarboxylique
- acide trans-{4-[4-(5-phénylméthanesulfinylméthyl-[1,3,4]thiadiazol-2-ylcarbamoyl)-phényl]-cyclohexyl}-acétique
- acide trans-{4-[4-(5-benzylsulfanylméthyl-[1,3,4]thiadiazol-2-ylcarbamoyl}-phényl]-cyclohexyl}-acétique
- acide trans- (4-{4-[5-(3-phényl-cyclobutyl)-[1,3,4]thiadiazol-2-ylcarbamoyl]-phényl}-cyclohexyl)-acétique
- acide cis-4-[5-(5-cyclopentyloxyméthyl-[1,3,4]thiadiazol-2-ylcarbamoyl)-pyridin-2-yloxy]-cyclohexanecarboxylique
- acide trans-(4-{4-[5-(2-cyclopentylamino-éthyl)-[1,3,4]thiadiazol-2-ylcarbamoyl]-phényl}-cyclohexyl)-acétique
- acide cis-4-(4-{5-[2-(3-morpholin-4-yl-cyclopentyl)-éthyl]-[1,3,4]thiadiazol-2-ylcarbamoyl}-phénoxy}-cyclohexanecarboxylique
- acide cis-4-{4-[5-(3-oxo-3-phényl-propyl)-[1,3,4]thiadiazol-2-ylcarbamoyl]-phénoxy}-cyclohexanecarboxylique
- acide cis-4-{4-[5-(3,5-difluoro-benzyl)-[1,3,4]thiadiazol-2-ylcarbamoyl]-phénoxy}-cyclohexanecarboxylique.

L'invention vise également un procédé de préparation d'un composé de formule (I) tel que défini précédemment, caractérisé en ce que l'on déprotège la fonction ester d'un composé choisi parmi (i) un composé de formule (V) 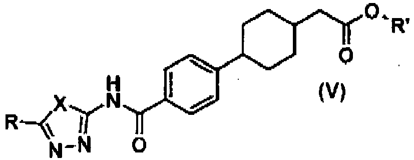
dans laquelle R représente un groupe -C(R1 R2)n-Y et X, Y, R1, R2 et n sont tels que définis précédemment et R' représente un groupe protecteur ;et
   (ii) un composé de formule (XXIII) : 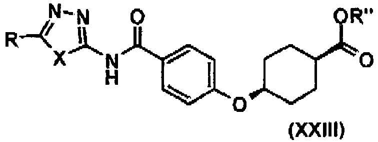
dans laquelle R représente un groupe -C(R1 R2)n-Y et X, Y, R1, R2 et n sont tels que définis précédemment et R" représente un groupe protecteur.

Selon un mode de réalisation, l'obtention du composé de formule (V), se fait par réaction (i) d'un composé de formule (II) : 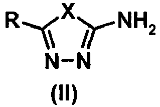
dans laquelle R représente un groupe -C(R1 R2)n-Y et X, Y, R1, R2 et n sont tels que définis précédemment, avec (ii) un composé de formule (III)

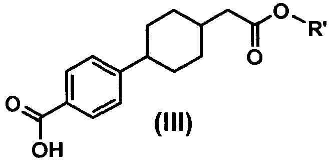

dans laquelle R' représente un groupe protecteur.

Selon un mode de réalisation, l'obtention du composé de formule (XXIII), se fait par réaction (i) d'un composé de formule (II) : 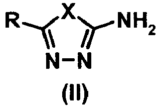
dans laquelle R représente un groupe -C(R1 R2)n-Y et X, Y, R1, R2 et n sont tels que définis précédemment, avec (ii) un composé de formule (XXI)

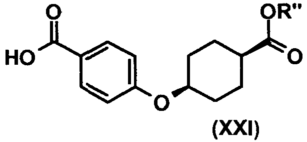

dans laquelle R" représente un groupe protecteur.

Dans les **schémas 1 à 21,** les composés de départ et les réactifs, quand leur mode de préparation, n'est pas décrit, sont disponibles dans le commerce ou décrits dans la littérature, ou bien, peuvent être préparés selon des méthodes qui y sont décrites ou qui sont connues de l'homme du métier.

Conformément à l'invention, on peut préparer les composés de formule générale (I) selon les procédés ci-après :
Le groupe R utilisé ci-après représente un groupe -Z1-C(R1R2)ₙ-Y avec R1, R2, Y, n et Z1 tels que définis précédemment.

Le schéma 1 décrit la synthèse des composés de formule (I) dans lesquels D est une liaison, n = 0, p = 1, ces composés seront appelés ci-après composés de formule (la).

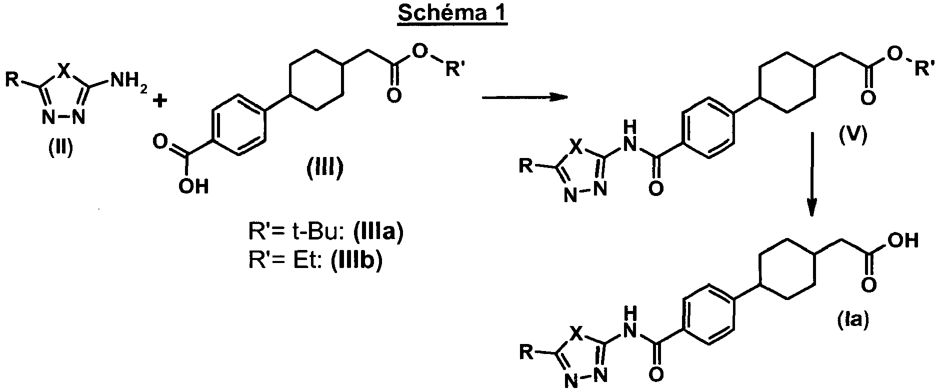

Dans le **schéma 1,** les intermédiaires de formule (V), pour lesquels R et X sont tels que définis précédemment et R' est un groupe protecteur tel qu'un groupe (C1-C6)alkyle par exemple un groupe éthyle ou tert-butyle, sont obtenus par couplage de l'acide de formule (III) avec les aminothiadiazoles ou aminooxadiazoles de formule (II) en présence d'un agent de couplage (par exemple, l'hexafluorophosphonate de bromo-tris-pyrrolidinophosphonium) dans un solvant polaire tel que le diméthylformamide ou l'acétonitrile entre 20 et 100°C. Les acides de formule (la) sont obtenus par la déprotection des esters de formule (V) par des méthodes choisies parmi celles connues de l'homme du métier, ces méthodes tenant compte de la stabilité du composé de formule (V) en milieu acide. Elles comprennent, entre autres, l'utilisation d'acide trifluoroacétique ou d'acide chlorhydrique dans un solvant polaire tel que le dichlorométhane ou le dioxane à température ambiante pour l'ester tertiobutylique ou l'hydroxyde de lithium dans un mélange de solvants polaires tels que l'eau, le méthanol ou le tetrahydrofurane pour l'ester éthylique.

Le schéma 2 détaille une synthèse des composés de formule (VIII) pour lesquels X représente un atome de soufre, ces composés seront appelés ci-après composés de formule (IIa).

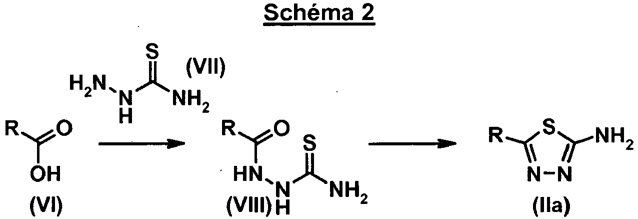

Dans le **schéma 2,** les composés de formules (VIII) peuvent être préparés par réaction d'un acide carboxylique et du thiosemicarbazide en présence d'un agent de couplage (par exemple la 1-éthyl-3-(3-diméthylaminopropyl) carbodiimide) dans un solvant polaire tel que le dichlorométhane ou le diméthylformamide à température ambiante. Les dérivés de formule (VIII) sont ensuite cyclisés dans un acide tel que l'acide sulfurique à température ambiante pour conduire aux aminothiadiazoles de formule (IIa).

Il est à noter que certains composés de formule générale (II) pour lesquels X représentent un atome de soufre ou d'oxygène sont commercialement disponibles. Certains composés de formule générale (VI) sont décrits dans la littérature (J. Org. Chem. 1968, 1959 ; J. Org. Chem. 1962, 1947).

Le schéma 3 détaille une synthèse des composés de formule (III) pour lesquels R' représente un groupe tert-butyle. Ces composés seront appelés ci-après composés de formule (IIIa).

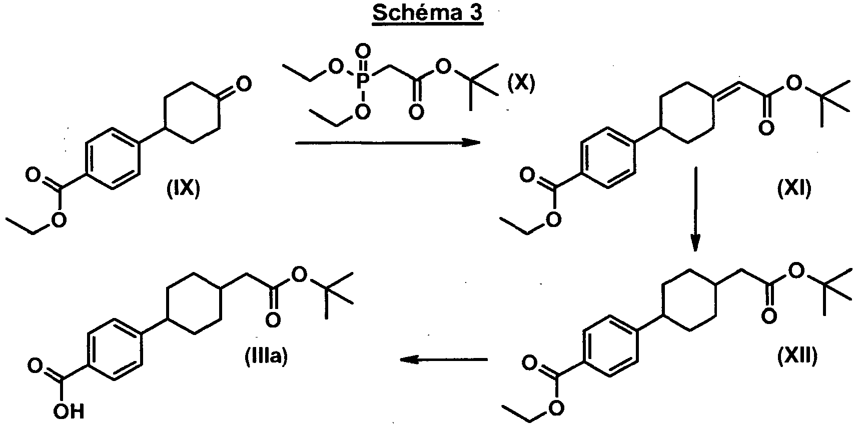

Dans le **schéma 3,** le composé de formule (XI) est préparé par une réaction de Horner-Wadsworth-Emmons à partir des dérivés de formules (IX) et (X) dans un solvant polaire tel que le diméthylformamide ou le tetrahydrofurane à température ambiante. Le composé (XI) est hydrogéné en présence d'un métal de transition tel que le palladium dans un solvant polaire tel que l'éthanol pour donner le composé de formule (XII). L'acide de formule (IIIa) est obtenu par hydrolyse de l'ester de formule (XII) en présence d'hydroxyde de lithium dans un mélange de solvants polaires tel que l'eau, le méthanol et le tetrahydrofurane.

Le composé de formule (IX) peut être préparé selon un schéma décrit dans la littérature (WO2003/099772).

Le **schéma 4** détaille une synthèse des composés pour lesquels R' représente un groupe éthyle. Ces composés seront appelés ci-après composés de formule (IIIb).

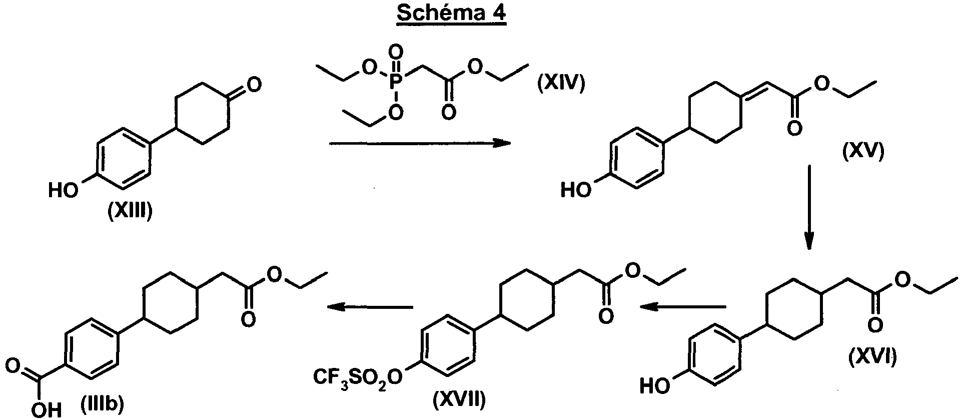

Dans le schéma 4, le composé de formule (XV) est préparé par une réaction de Horner-Wadsworth-Emmons à partir des dérivés de formules (XIII) et (XIV) dans un solvant polaire à température ambiante. Le composé de formule (XV) est hydrogéné en présence d'un métal de transition tel que le palladium dans un solvant polaire tel que l'éthanol ou l'acétate d'éthyle pour donner le composé de formule (XVI). Le composé de formule (XVI) est transformé en acide de formule (IIIb) dans un solvant polaire tel que le dichlorométhane entre 0 et 25°C du composé de formule (XVI) suivi d'une hydroxycarbonylation de l'intermédiaire de formule (XVII) dans un solvant polaire tel que le dioxane entre 100 et 120°C.

Le **schéma 5** décrit la synthèse des composés de formule (I) dans lesquels D est un atome d'oxygène et p = 0, ces composés seront appelés ci-après composés de formule (Ib).

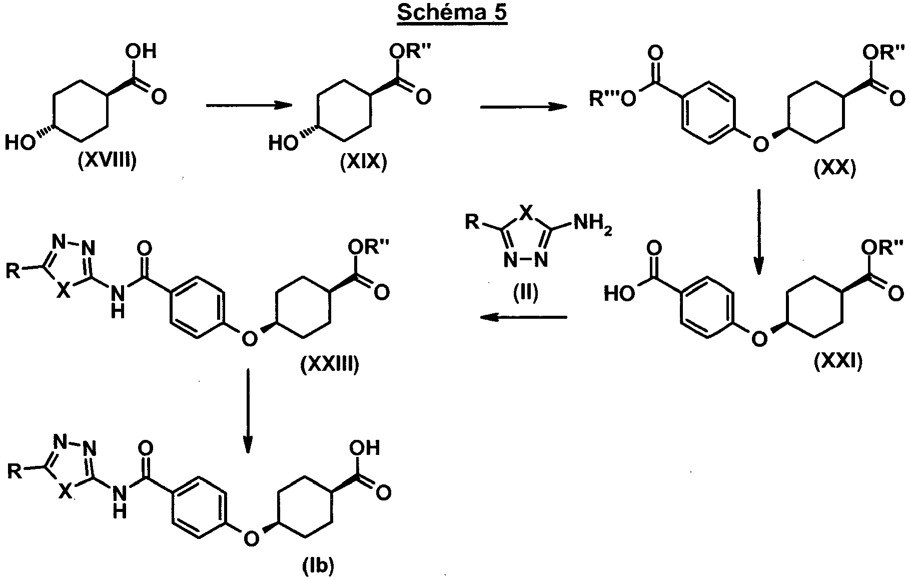

Dans le **schéma 5,** le composé de formule (XIX) pour lequel R" est un groupe protecteur tel qu'un groupe (C1-C6)alkyle, par exemple un groupe méthyle, est obtenu à partir de l'acide de formule (XVIII) par réaction sélective avec un composé connu de l'homme l'art tel que le triméthylsilyl diazométhane dans un mélange de solvant apolaire et polaire tel que respectivement le toluène et le méthanol à température ambiante. L'alcool de formule (XIX) où R" est un groupe (C1-C6)alkyle tel qu'un groupe tert-butyle, est engagé dans une réaction de Mitsunobu avec un alcool tel qu'un alcool du type 4-hydroxy-benzoate de (C1-C6)alkyle par exemple le 4-hydroxy-benzoate de *tert*-butyle dans un solvant polaire tel que le tetrahydrofurane à température ambiante pour donner l'éther de formule (XX). L'acide de formule (XXI) est obtenu par la déprotection de la fonction ester -COOR"' du composé de formule (XX) par des méthodes choisies parmi celles connues de l'homme du métier. Elles comprennent entre autres l'utilisation d'acide trifluoroacétique ou d'acide chlorhydrique dans des solvants polaires tels que le dichlorométhane ou le dioxane. Les intermédiaires de formule (XXIII) sont obtenus par couplage de l'acide de formule (XXI) avec les aminothiadiazoles ou aminooxadiazoles de formule (II) en présence d'un agent de couplage (par exemple, l'hexafluorophosphonate de bromo-tris-pyrrolidinophosphonium) dans un solvant polaire tel que le diméthylformamide ou l'acétonitrile à température ambiante. Les acides de formule (Ib) sont obtenus après déprotection de l'ester de formule (XXIII) par des méthodes choisies parmi celles connues de l'homme du métier. Elles comprennent entre autres l'utilisation d'hydroxyde de lithium dans un mélange de solvants polaires tel que l'eau et le tetrahydrofurane.

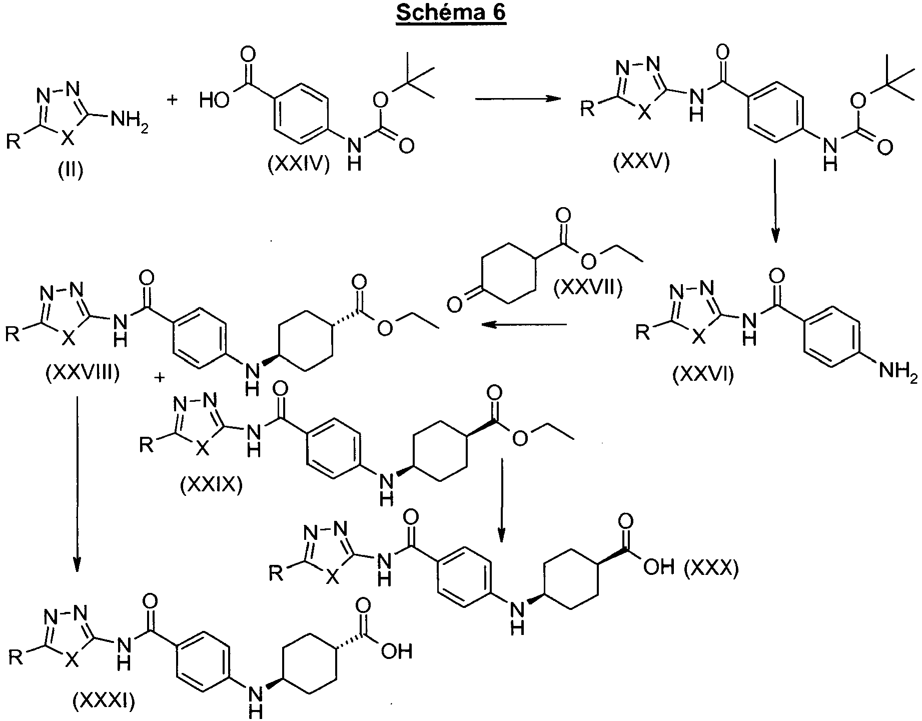

Dans le **schéma 6,** les intermédiaires de formule (XXV) sont obtenus par couplage de l'acide de formule (XXIV) avec les aminothiadiazoles ou aminooxadiazoles de formule (II) en présence d'un agent de couplage (par exemple, le 1-éthyl-3-(3-diméthylaminopropyl) carbodiimide) dans un solvant polaire tel que le diméthylformamide ou l'acétonitrile à température ambiante par des méthodes choisies parmi celles connues de l'homme du métier. La déprotection du carbamate de formule (XXV) en amine de formule (XXVI) est effectuée par des méthodes choisies parmi celles connues de l'homme du métier. Elles comprennent entre autres l'utilisation d'acide trifluoroacétique ou d'acide chlorhydrique dans des solvants polaires tels que le dichlorométhane ou le dioxane. L'amination réductrice de la cyclohexanone (XXVII) par l'amine de formule (XXVI) se fait en présence d'un donneur d'hydrure tel que le triacétoxyborohydrure de sodium dans un solvant polaire tel que le dichloroéthane par des méthodes choisies parmi celles connues de l'homme du métier. Les aminocyclohexyles (XXVIII) et (XXIX) obtenues sont séparées par chromatographie. Les acides de formule (XXX) et (XXXI) sont obtenus par la déprotection respective des esters de formule (XXVIII) et (XXIX) par l'utilisation d'hydroxyde de lithium dans un mélange de solvants polaires tels que l'eau, le méthanol ou le tetrahydrofurane.

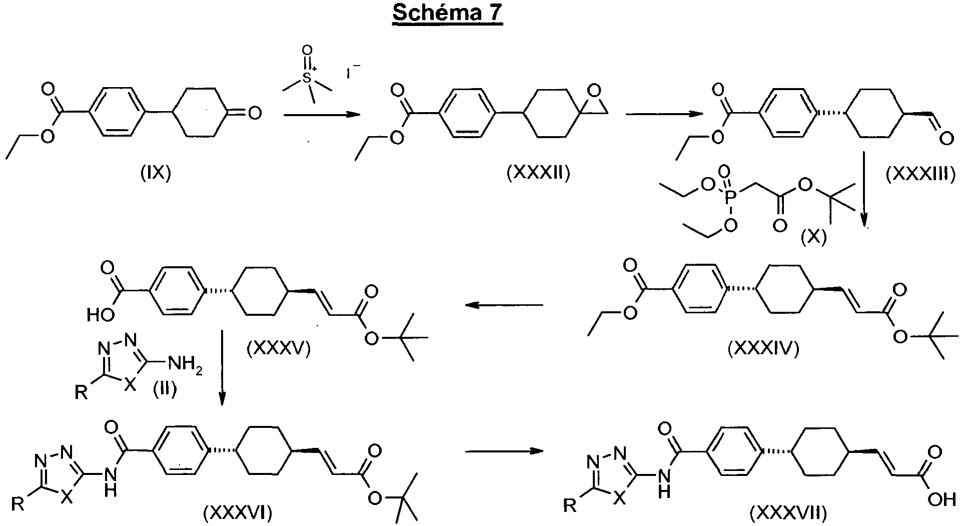

Dans le schéma 7, le composé (XXXII) est préparé à partir de la cétone (IX) par une réaction d'époxydation avec l'iodure de triméthylsulfoxonium dans un solvant polaire tel que le DMSO à des températures comprises entre l'ambiante et 50°C. Le composé (XXXII) est transformé en aldéhyde (XXXIII) par l'action d'un acide de lewis tel que l'éthérate de trifluorure de bore dans un solvant polaire tel que le dichlorométhane. Le composé de formule (XXXIV) est préparé par une réaction de Horner-Wadsworth-Emmons à partir du dérivé (XXXIII) et du phosphonate (X) dans un solvant polaire tel que le diméthylformamide ou le tétrahydrofurane à température ambiante en présence d'une base tel que l'hydrure de sodium. Les acides de formule (XXXV) sont obtenus par la déprotection des esters de formule (XXXIV) par une base alcaline tel que l'hydroxyde de lithium dans un mélange de solvants polaires tels que l'eau, le méthanol ou le tetrahydrofurane. Les intermédiaires de formule (XXXVI) sont obtenus par couplage de l'acide de formule (XXXV) avec les aminothiadiazoles ou aminooxadiazoles de formule (II) en présence d'un agent de couplage (par exemple, l'hexafluorophosphonate de bromo-tris-pyrrolidinophosphonium) dans un solvant polaire tel que le diméthylformamide ou l'acétonitrile à température ambiante. Les acides de formule (XXXVII) sont obtenus par hydrolyse des esters de formule (XXXVI) en présence d'acide trifluoroacétique ou d'acide chlorhydrique dans un solvant polaire tel que le dichlorométhane ou le dioxane à température ambiante.

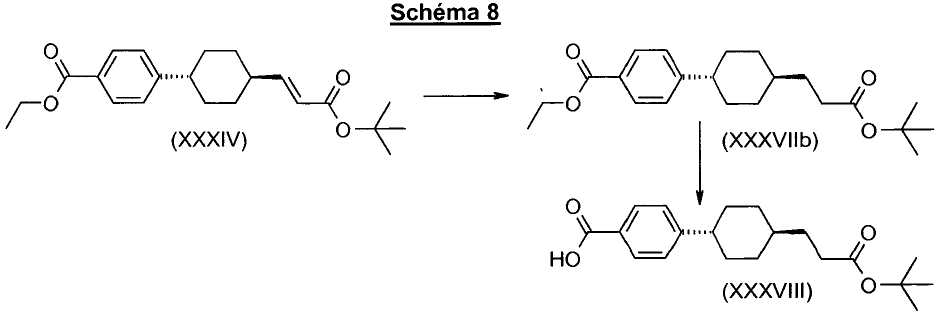

Dans le **schéma 8,** le composé (XXXIV) est hydrogéné en présence d'un métal de transition tel que le palladium dans un solvant polaire tel que l'éthanol pour donner le composé (XXXVIIb). L'acide (XXXVIII) est obtenu par la déprotection de l'ester (XXXVIIb) par une base alcaline tel que l'hydroxyde de lithium dans un mélange de solvants polaires tels que l'eau, le méthanol ou le tetrahydrofurane.

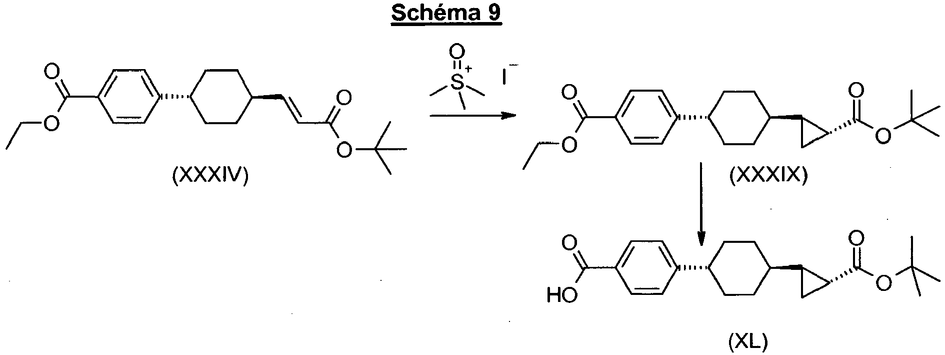

Dans le **schéma 9,** le composé (XXXIV) est engagé dans une réaction de cyclopropanation avec l'iodure de triméthylsulfoxonium dans un solvant polaire tel que le DMSO à température ambiante pour donner le composé (XXXIX). L'acide (XL) est obtenu par la déprotection de l'ester (XXXIX) par une base alcaline tel que l'hydroxyde de lithium dans un mélange de solvants polaires tels que l'eau, le méthanol ou le tetrahydrofurane.

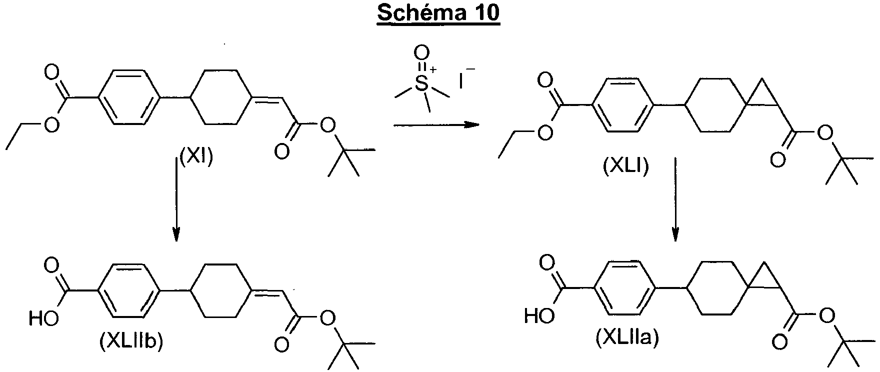

Dans le **schéma 10,** le composé (XI) est engagé dans une réaction de cyclopropanation avec l'iodure de triméthylsulfoxonium dans un solvant polaire tel que le DMSO à température ambiante pour donner le composé (XLI). Les acides (XLIIa) et (XLIIb) sont respectivement obtenus par la déprotection des esters (XLI) et (XI) par une base alcaline tel que l'hydroxyde de lithium dans un mélange de solvants polaires tels que l'eau, le méthanol ou le tetrahydrofurane.

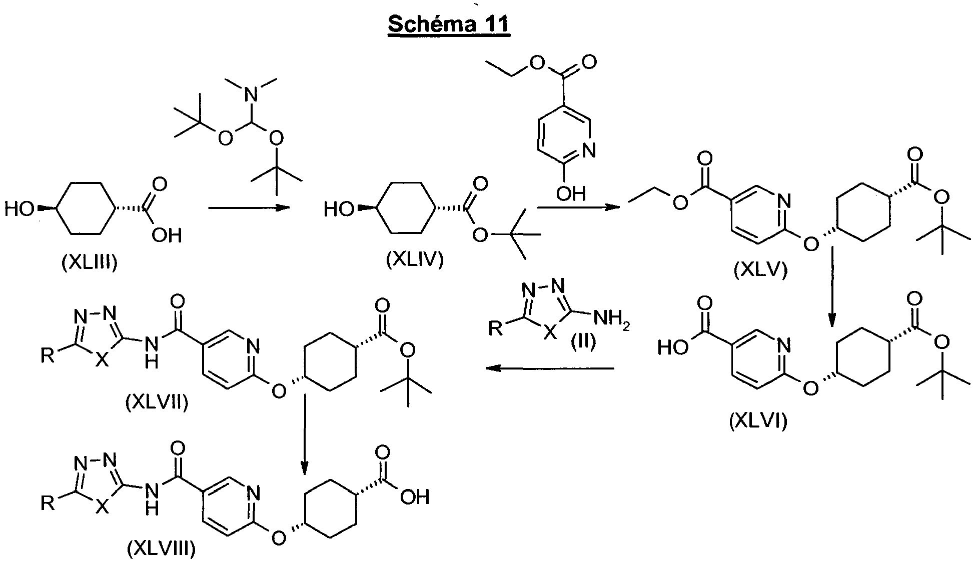

Dans le **schéma 11** le composé de formule (XLIV) est obtenu à partir de l'acide de formule (XLIII) par la réaction sélective avec un composé connu de l'homme de l'art tel que la bis(di-tert-butoxyméthyl)-N,N-diméthylméthylamine dans un solvant apolaire tel que le toluène à reflux. L'alcool de formule (XLIV) est engagé dans une réaction de Mitsunobu avec un alcool tel que le 6-hydroxy-nicotinate d'éthyle dans un solvant polaire tel que le tetrahydrofurane à température ambiante pour donner l'éther de formule (XLV). L'acide de formule (XLVI) est obtenu par la déprotection de l'ester (XLV) par une base alcaline tel que l'hydroxyde de lithium dans un mélange de solvants polaires tels que l'eau, le méthanol ou le tetrahydrofurane. Les intermédiaires de formule (XLVII) sont obtenus par couplage de l'acide de formule (XLVI) avec les aminothiadiazoles ou les aminooxadiazoles de formule (II) en présence d'un agent de couplage (par exemple, l'hexafluorophosphonate de bromo-tris-pyrrolidinophosphonium) dans un solvant polaire tel que le diméthylformamide ou l'acétonitrile à température ambiante. Les acides de formule (XLVIII) sont obtenus par hydrolyse des esters de formule (XLVII) en présence d'acide trifluoroacétique ou d'acide chlorhydrique dans un solvant polaire tel que le dichlorométhane ou le dioxane à température ambiante.

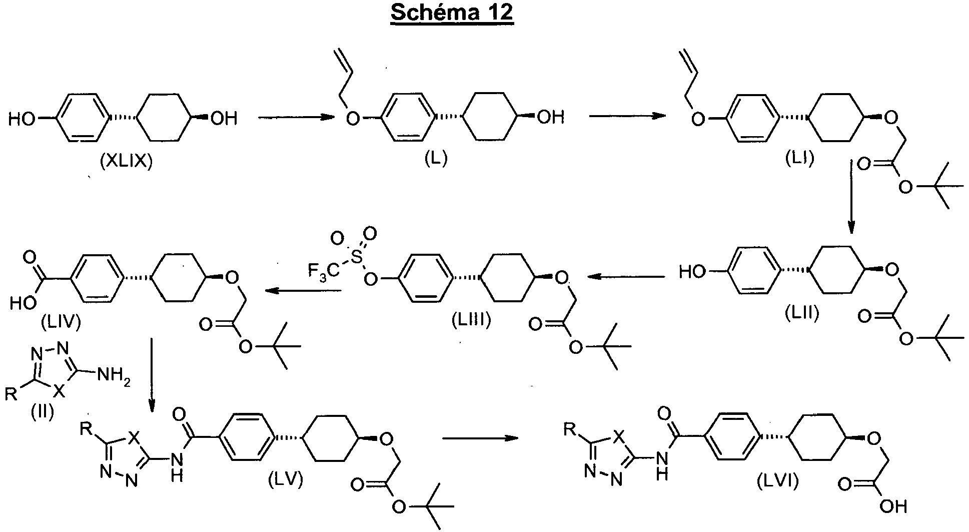

Dans le **schéma 12,** le composé (XLIX) est protégé par du bromure d'allyle dans un solvant polaire tel que l'acétone ou le DMF pour donner le composé (L). L'alkylation du composé (L) en intermédiaire (LI) est effectuée par un halogénoacétate d'alkyle tel que le bromoacétate de tert-butyle par des méthodes choisies parmi celles connues de l'homme du métier. Elles comprennent entre autres l'utilisation d'un catalyseur de transfert de phase tel que l'hydrogénosulfate de tétrabutylammonium et l'utilisation d'une base tel que l'hydroxyde de sodium en solution aqueuse dans un solvant apolaire tel que le toluène. La déprotection de l'intermédiaire (LI) en phénol (LII) se fait par des méthodes choisies parmi celles connues de l'homme du métier. Elles comprennent entre autres l'utilisation d'un catalyseur au palladium tel que le tétrakistriphénylphosphine de palladium dans un solvant polaire tel que le dichlorométhane. Le phénol (LII) est transformé en triflate (LIII) à l'aide d'un réactif tel que l'anhydride triflique dans un solvant polaire tel que le dichlorométhane entre 0 et 25°C en présence d'une base tel que la triéthylamine. L'acide (LIV) est obtenu par hydroxycarbonylation du triflate (LIII) dans un solvant polaire tel que le dioxane entre 100 et 120°C de l'intermédiaire de formule (LIII) par des méthodes choisies parmi celles connues de l'homme du métier. Elles comprennent entre autres l'utilisation d'un réactif de carbonylation tel que l'hexacarbonyle de molybdène et d'un catalyseur au palladium tel que le diacétate de palladium dans un solvant polaire tel que le dioxane. Les intermédiaires de formule (LV) sont obtenus par couplage de l'acide de formule (LIV) avec les aminothiadiazoles ou aminooxadiazoles de formule (II) en présence d'un agent de couplage (par exemple, le 1-éthyl-3-(3-diméthylaminopropyl) carbodiimide) dans un solvant polaire tel que le diméthylformamide ou l'acétonitrile à température ambiante. Les acides de formule (LVI) sont obtenus par hydrolyse des esters de formule (LV) en présence d'acide trifluoroacétique ou d'acide chlorhydrique dans un solvant polaire tel que le dichlorométhane ou le dioxane à température ambiante.

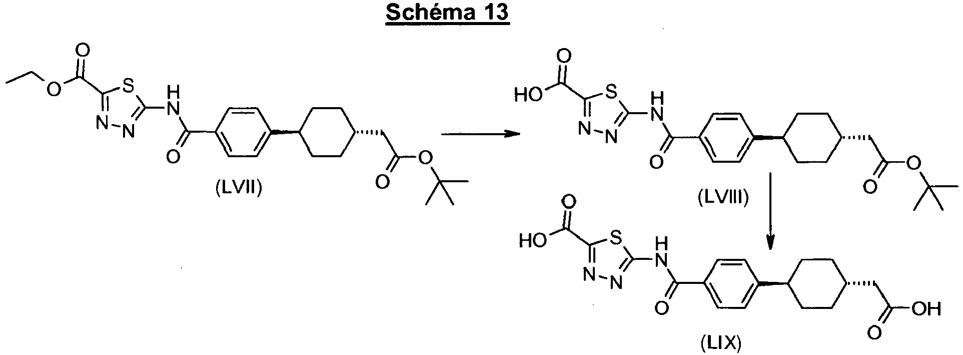

Dans le **schéma 13,** le composé (LVIII) est obtenu par l'hydrolyse chémosélective du diester (LVII) par une base alcaline tel que l'hydroxyde de lithium dans un mélange de solvants polaires tels que l'eau, le méthanol ou le tetrahydrofurane. Le composé (LIX) est obtenu par hydrolyse de l'ester de formule (LVIII) en présence d'acide trifluoroacétique ou d'acide chlorhydrique dans un solvant polaire tel que le dichlorométhane ou le dioxane à température ambiante.

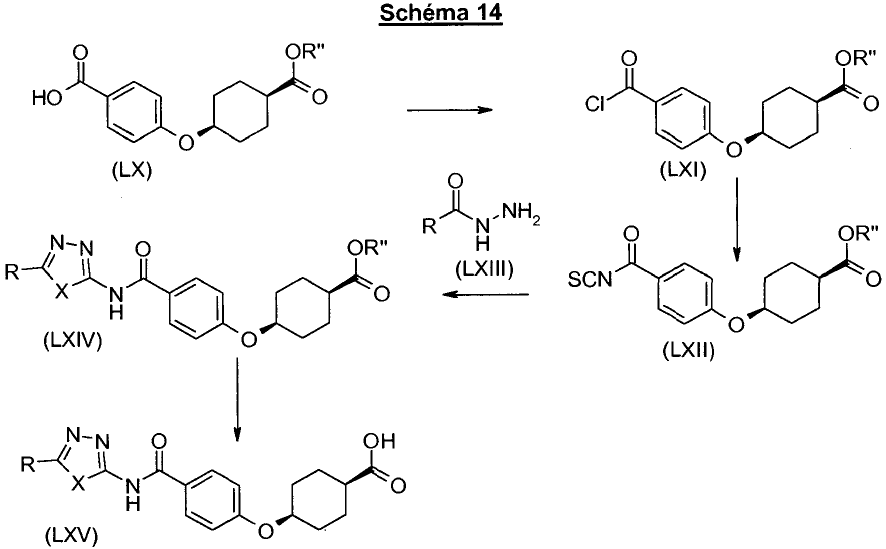

Dans le **schéma 14,** les chlorures d'acides de formule (LXI) sont obtenus à partir des acides carboxyliques de formule (LX) par action d'un agent de chloration tel que le chlorure d'oxalyle dans un solvant polaire tel que le dichlorométhane en présence d'un catalyseur tel que le DMF. Les chlorures d'acides de formule (LXI) ainsi obtenus réagissent avec le thioisocyanate de potassium dans un solvant polaire tel que l'acétonitrile ou l'acétone pour donner les acylthioisocyanates de formule (LXII). La réaction des hydrazides de formule (LXIII) sur les thioisocyanates de formule (LXII) dans un solvant polaire tel que l'acétonitrile entre 60 et 80°C conduit aux intermédiaires de formule (LXIV). Les acides de formule (LXV) sont obtenus par hydrolyse des esters de formule (LXIV) en présence d'acide trifluoroacétique ou d'acide chlorhydrique dans un solvant polaire tel que le dichlorométhane ou le dioxane à température ambiante.

Les hydrazides de formules (LXIII) sont obtenus à partir des esters ou des acides correspondants par des méthodes choisies parmi celles connues de l'homme du métier. Elles comprennent entre autres l'utilisation d'un réactif tel que l'hydrazine sur les esters dans un solvant polaire tel que l'éthanol à reflux ou d'un réactif tel que le carbazate de tertiobutyle sur les acides en présence d'un agent de couplage (par exemple, le 1-éthyl-3-(3-diméthylaminopropyl) carbodiimide) dans un solvant polaire tel que le dichlorométhane à température ambiante suivie d'une déprotection en présence d'acide trifluoroacétique ou d'acide chlorhydrique dans un solvant polaire tel que le dichlorométhane ou le dioxane à température ambiante.

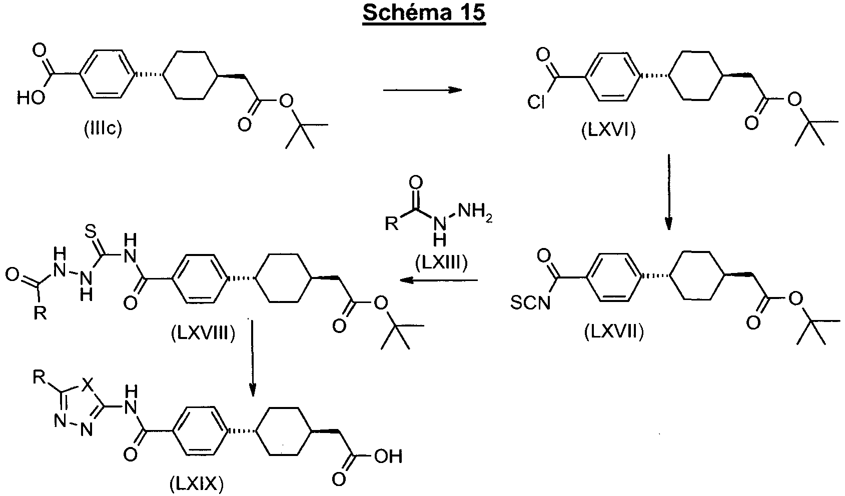

Dans le **schéma 15,** les chlorures d'acides de formule (LXVI) sont obtenus à partir des acides carboxyliques de formule (IIIc) par action d'un agent de chloration tel que le chlorure d'oxalyle dans un solvant polaire tel que le dichlorométhane en présence d'un catalyseur tel que le DMF. Les chlorures d'acides de formule (LXVI) ainsi obtenus réagissent avec le thioisocyanate de potassium dans un solvant polaire tel que l'acétonitrile ou l'acétone pour donner les acylthioisocyanates de formule (LXVII). La réaction des hydrazides de formule (LXIII) sur les thioisocyanates de formule (LXVII) dans un solvant polaire tel que l'acétonitrile entre 60 et 80°C conduit aux intermédiaires de formule (LXVIII). Les intermédiaires de formule (LXVIII) se cyclisent en composés de formule (LXIX) avec une hydrolyse des fonctions esters en présence d'acide trifluoroacétique ou d'acide chlorhydrique dans un solvant polaire tel que le dichlorométhane ou le dioxane à température ambiante.

Dans le **schéma 16,** la cétone (LXX) est réduite par un hydrure tel que le borohydrure de sodium dans un solvant polaire tel que le méthanol pour donner l'alcool (LXXI). L'intermédiaire de formule (LXXI) est engagé dans une réaction de Mitsunobu avec un alcool tel que le phénol dans un solvant polaire tel que le tetrahydrofurane à température ambiante pour donner l'éther de formule (LXXII). L'acide de formule (LXXIII) est obtenu par la déprotection de l'ester (LXXII) par une base alcaline tel que l'hydroxyde de lithium dans un mélange de solvants polaires tels que l'eau, le méthanol ou le tétrahydrofurane.

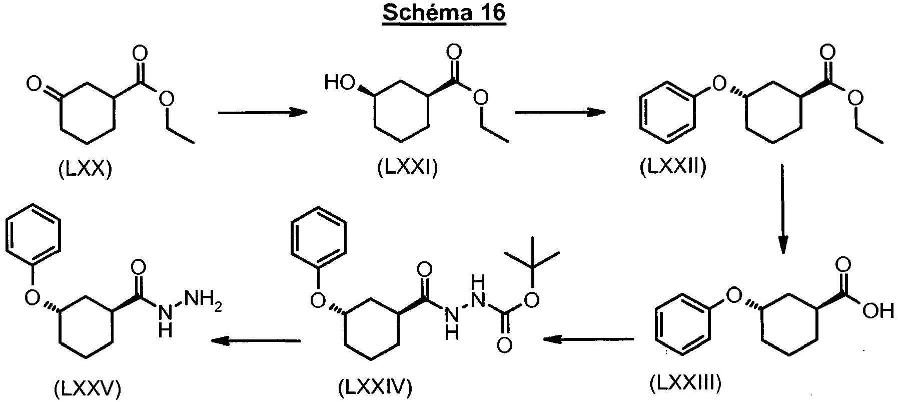

L'intermédiaire de formule (LXXIV) est obtenu par couplage de l'acide de formule (LXXIII) avec le carbazate de tert-butyle en présence d'un agent de couplage (par exemple, le 1-éthyl-3-(3-diméthylaminopropyl) carbodiimide) dans un solvant polaire tel que le dichlorométhane à température ambiante. Le composé (LXXV) est obtenu par hydrolyse de l'intermédiaire de formule (LXXIV) en présence d'acide trifluoroacétique ou d'acide chlorhydrique dans un solvant polaire tel que le dichlorométhane ou le dioxane à température ambiante.

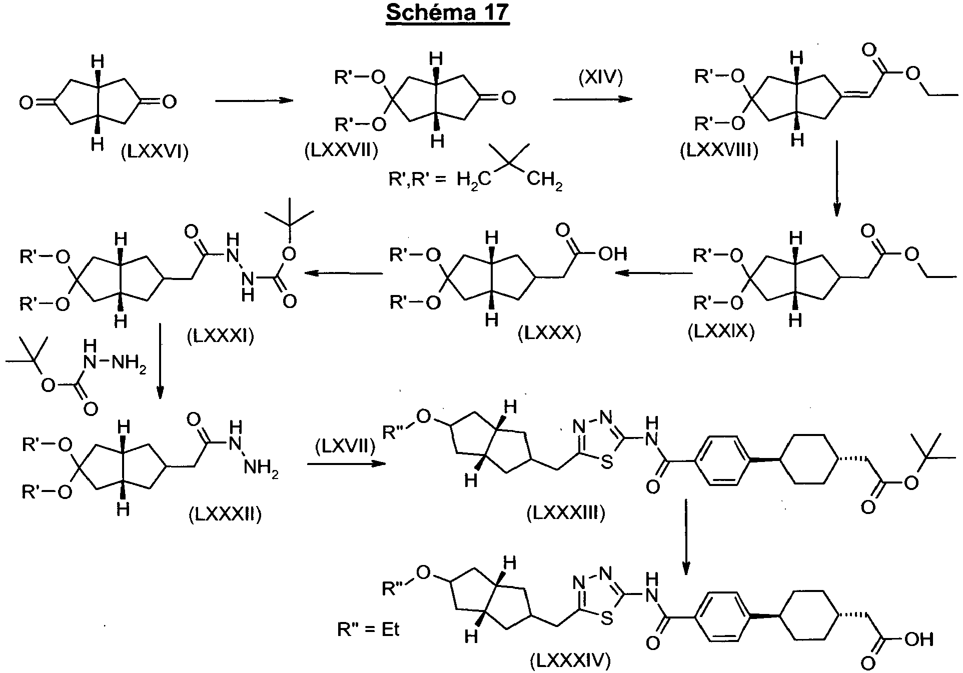

Dans le **schéma 17,** la monoprotection d'une fonction cétone du composé de formule (LXXVI) par un alcool de formule générale R'OH ou un diol de formule générale HOR'-R'OH donne les intermédiaires de formule (LXXVII) par des méthodes choisies parmi celles connues de l'homme du métier. Elles comprennent entre autres l'utilisation d'acide tel que l'acide para-toluènesulfonique dans un solvant tel que le toluène à reflux. Les composés de formule (LXXVIII) sont préparés par une réaction de Horner-Wadsworth-Emmons à partir des dérivés de formule (LXXVII) et du phosphonate de formule (XIV) dans un solvant polaire tel que le diméthylformamide ou le tetrahydrofurane à température ambiante en présence d'une base tel que l'hydrure de sodium. Les composés de formule (LXXVIII) sont hydrogénés en présence d'un métal de transition tel que le palladium dans un solvant polaire tel que l'éthanol pour donner le composé (LXXIX). Les acides de formule (LXXX) sont obtenus par la déprotection des esters de formule (LXXIX) par une base alcaline tel que l'hydroxyde de lithium dans un mélange de solvants polaires tels que l'eau, le méthanol ou le tetrahydrofurane. Les intermédiaires de formule (LXXXI) sont obtenus par couplage des acides de formule (LXXX) avec le carbazate de tert-butyle en présence d'un agent de couplage (par exemple, le 1-éthyl-3-(3-diméthylaminopropyl) carbodiimide) dans un solvant polaire tel que le dichlorométhane à température ambiante. Les composés de formule (LXXXII) sont obtenus par hydrolyse des intermédiaires de formule (LXXXI) en présence d'acide trifluoroacétique ou d'acide chlorhydrique dans un solvant polaire tel que le dichlorométhane ou le dioxane à température ambiante. La réaction des hydrazides de formule (LXXXII) sur les thioisocyanates de formule (LXVII) dans un solvant polaire tel que l'acétonitrile ou l'acétone entre 60 et 80°C conduit aux intermédiaires de formule (LXXXIII). Les acides de formule (LXXXIV) sont obtenus par hydrolyse des esters de formule (LXXXIII) en présence d'acide trifluoroacétique ou d'acide chlorhydrique dans un solvant polaire tel que le dichlorométhane ou le dioxane à température ambiante.

Dans le **schéma 18,** le composé de formule (IX) est transformé en triflate d'énol (LXXXV) par des méthodes choisies parmi celles connues de l'homme du métier. Elles comprennent entre autres l'utilisation d'un réactif tel que le N,N-bistrifluorométhanesulfonimide en présence d'une base tel que le n-butyllithium dans un solvant polaire tel que le tetrahydrofurane à des températures comprises entre - 70°C et 25°C.

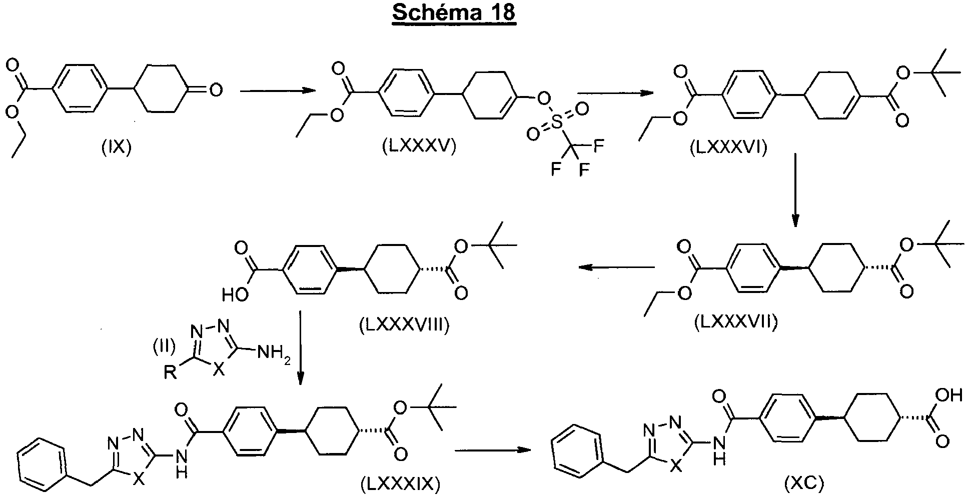

Le triflate d'énol (LXXXV) en transformé en intermédiaire (LXXXVI) dans une réaction d'alkoxycarbonylation par des méthodes choisies parmi celles connues de l'homme du métier. Elles comprennent entre autres l'utilisation d'un réactif de carbonylation tel que l'hexacarbonyle de molybdène et d'un catalyseur au palladium tel que le diacétate de palladium dans un solvant polaire tel que le tert-butanol entre 100 et 120°C. Le composé (LXXXVI) est hydrogéné en présence d'un métal de transition tel que le palladium dans un solvant polaire tel que l'éthanol à une température entre 25 et 40°C pour donner le composé de formule (LXXXVII). L'acide de formule (LXXXVIII) est obtenu par la déprotection de l'ester (LXXXVII) par une base alcaline tel que l'hydroxyde de lithium dans un mélange de solvants polaires tels que l'eau, le méthanol ou le tetrahydrofurane. Les intermédiaires de formule (LXXXIX) sont obtenus par couplage de l'acide de formule (LXXXVIII) avec les aminothiadiazoles ou aminooxadiazoles de formule (II) en présence d'un agent de couplage (par exemple, l'hexafluorophosphonate de bromo-tris-pyrrolidinophosphonium) dans un solvant polaire tel que le diméthylformamide ou l'acétonitrile à température ambiante. Les acides de formule (XC) sont obtenus par hydrolyse des esters de formule (LXXXIX) en présence d'acide trifluoroacétique ou d'acide chlorhydrique dans un solvant polaire tel que le dichlorométhane ou le dioxane à température ambiante.

Dans le **schéma 19,** le composé de formule (XCI) est transformé en alcool (XCII) par l'utilisation d'un réducteur tel que le borohydrure de sodium dans un solvant polaire tel que le DMF. L'intermédiaire (XCII) est traité par un acide tel que l'acide trifluoroacétique dans un solvant polaire tel que le dichlorométhane pour donner l'acide (XCIII).

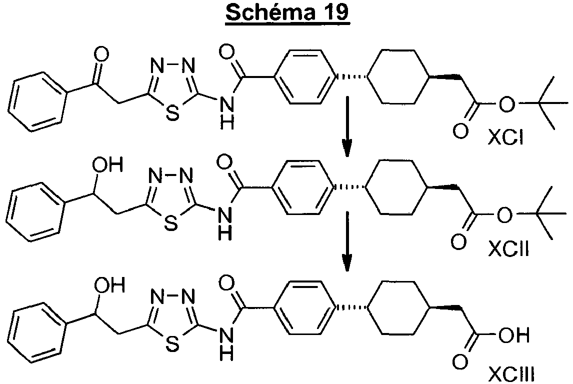

Dans le **schéma 20,** le thioether de formule (XCIV) est oxydé en sulfoxyde (XCV) par l'utilisation d'un peracide tel que l'acide méta-chloroperbenzoïque dans un solvant polaire tel que le dichlorométhane.

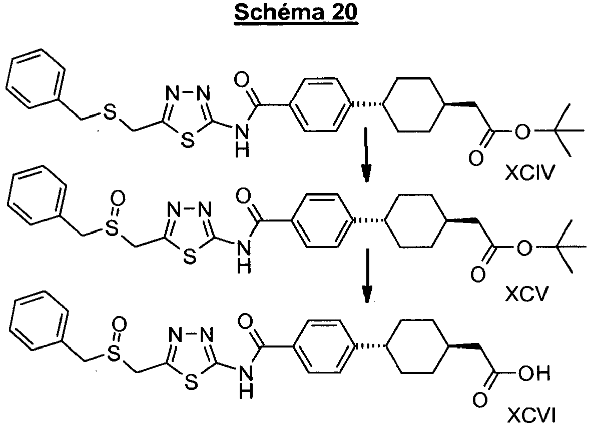

L'intermédiaire (XCV) est traité par un acide tel que l'acide trifluoroacétique dans un solvant polaire tel que le dichlorométhane pour donner l'acide (XCVI).

Dans le **schéma 21,** les acides (LXIX) sont transformés en amides (XCVII) par l'ammoniac ou une amine HNR5 en présence d'un agent de couplage (par exemple, l'hexafluorophosphonate de bromo-tris-pyrrolidinophosphonium) dans un solvant polaire tel que le DMF à température ambiante. HNR5 est une alkylamine substitutée par un groupe hydroxyle, dialkylamine, hétérocycloalkyle ou alkyloxy.

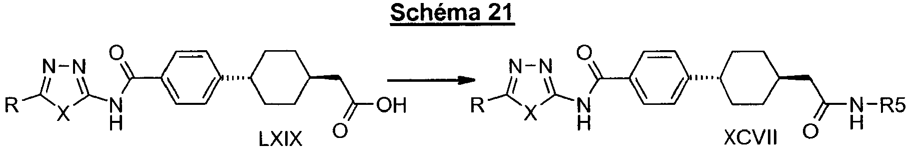

L'invention, selon un autre de ses aspects, a également pour objet les composés de formules (III), (IIIa), (IIIb), (IIIc), (V), (XXI) à l'exclusion du composé acide 4-(4-éthoxy-carbonyl-cyclohexyloxy)-benzoique, (XXIII), (XXVIII), (XXIX), (XXX), (XXXV), (XXXVI), (XXXVIII), (XL), (XLIIa), (XLIIb), (XLVI), (XLVII), (LIV), (LV), (LVIII), (LXIV), (LXVIII), (LXXV), (LXXXII), (LXXXIII), (LXXXIX), (XCI), (XCII), (XCIV) et (XCV). Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les abréviations et formules brutes suivantes sont utilisées :
- ACN: Acétonitrile
- BSA: Bovine serum albumine
- °C: Degré Celsius
- LC-MS: Chromatographie liquide-spectrométrie de masse
- CO₂: dioxyde de carbone
- cm²: Centimètre carré
- DIEA: Düsopropyléthylamine
- dec.: Décomposition
- DMAP: 4-Diméthylaminopyridine
- DMEM: Dulbecco's Minimum Essential Medium Modified
- DMF: Diméthylformamide
- DPPF: 1,1'-Bis(diphénylphosphino)ferrocène
- EDTA: acide éthylène-diamine-tétraacétique
- éq.: Equivalent
- ESI+: Electrospray lonization
- g: gramme
- RMN: résonnance magnétique nucléaire
- h: heure
- H₂O: Eau
- HPLC: chromatographie en phase liquide à haute performance
- Hz: Hertz
- M: masse
- MHz: mégahertz
- mg: milligramme
- mL: millilitre
- mm: millimètre
- mmoles: millimoles
- M.O.: micro-ondes
- N: Normal
- N-Boc-: N-tert-Butyloxycarbonyle
- nM: nanomolaire
- PBS: phosphate buffer saline
- ppm: parties par millions
- psi: pound per square inch
- SVF: Sérum de veau foetal
- SO₂: Dioxyde de soufre
- TFA: Acide trifluoroacétique
- THF: Tetrahydrofurane
- UPLC: chromatographie en phase liquide à très haute performance
- UV: ultra-violet
- VIH: Virus de l'immunodéficience humaine
- µCi: microcurie
- %: pourcentage

La mesure de radioactivité se fait par sur un appareil Flo One C625TR (Perkin Elmer).

Les spectres de résonnance magnétique du proton (RMN ¹H), tels que décrits ci-dessous, sont enregistrés à 400 MHz dans du DMSO-d6, en utilisant le pic du DMSO-d5 comme référence. Les déplacements chimiques δ sont exprimés en partie par million (ppm). Les signaux observés sont exprimés ainsi : s = singulet ; d = doublet ; t = triplet ; m = massif ou singulet large ;

Dans la colonne LC/MS et dans les exemples est indiqué le pic MH⁺ identifié par spectrométrie de masse. Les composés sont analysés par une chromatographie liquide (détecteur UV à 220nM) couplée à un spectromètre de masse avec un détecteur par ionisation electrospray. La méthode analytique est détaillée ci-dessous
UPLC/MS - gradient de 3 min -eau/ACN/TFA
T 0min 98% A - T1.6 à T2.1min : 100%B - T2.5 à T 3min : 98% A
Voie A : eau + 0,05% TFA, voie B : ACN + 0,035% TFA
Débit : 1,0mL/min - T° = 40°C - injection 2 µL
Colonne Acquity BEH C18 (50*2.1 mm ; 1.7 *µm)

### Exemple 1 : 5-(4-fluorobenzyl)-1,3,4-thiadiazol-2-amine

### 1.1 Synthèse de la 2-[(4-fluorophényl)acétyl]hydrazinecarbothioamide

On place 5 g d'acide 4-fluorophénylacétique (32,44 mmoles, 1 éq.) dans 50 mL de dichlorométhane sous agitation. On ajoute successivement toujours sous agitation à température ambiante, 3,25 g de thiosemicarbazide (35,68 mmoles, 1 éq.), 4,38 g d'hydroxybenzotriazole (32,44 mmoles, 1 éq.), 6,22 g de chlorhydrate de la 1-éthyl-3-(3-diméthylaminopropyl) carbodiimide (32,44 mmoles, 1,2 éq.). Après 18 h à température ambiante, le dichlorométhane est évaporé. Le résidu est repris par de l'acétate d'éthyle, lavé 3 fois à l'eau, 1 fois à la saumure et 3 fois à l'acide chlorhydrique 1 N. La phase organique est séchée sur sulfate de sodium et évaporée pour donner 5 g de 2-[(4-fluorophényl)acétyl]hydrazinecarbothioamide.
M-isobutène⁺ = 228.

### 1.2 Synthèse de la 5-(4-fluorobenzyl)-1,3,4-thiadiazol-2-amine

On place 5 mL d'acide sulfurique dans un ballon qui est mis à refroidir entre 0 et -10°C en plaçant dans un bain de glace et de chlorure de sodium.
0,38 g de 2-[(4-fluorophényl)acétyl]hydrazinecarbothioamide (1,67 mmoles) sont ajoutés par portions sous agitation. Après 3 h d'agitation entre 0 et -10°C, on ajoute goutte à goutte de l'eau puis on revient à pH basique avec de l'hydroxyde de sodium tout en maintenant une température entre 0 et -10°C. Le précipité est filtré, lavé à l'eau et séché. On obtient 0,264 g de 5-(4-fluorobenzyl)-1,3,4-thiadiazol-2-amine.
M+H⁺ = 210.

### Exemple 2 : acide 4-(4-tert-butoxycarbonylméthylcyclohexyl)benzoïque

### 2.1 Synthèse du 4-(4-tert-butoxycarbonylméthylènecyclohexyl)benzoate d'éthyle

On place 5,63 g de diéthylphosphoacétate de tert-butyl (20,3 mmoles, 1 éq.) dans 20 mL de diméthylformamide sous agitation. La solution est refroidie jusqu'à une température de 4°C en plaçant la solution dans un bain de glace puis 0,536 g d'hydrure de sodium (22,33 mmoles, 1,1 éq.) est ajouté par portions. Après 30 minutes, on ajoute 5 g de 4-(4-oxo-cyclohexyl)-benzoate d'éthyle (20,3 mmoles, 1 éq.) et on enlève le bain de glace. Après une heure d'agitation, on place le ballon dans un bain de glace pour refroidir le milieu réactionnel jusqu'à une température de 4°C et on ajoute 0,049 g d'hydrure de sodium (2,04 mmoles, 0,1 éq.). Le bain de glace est enlevé et après 30 minutes, on verse sur 200 mL d'hydrogénosulfate de potassium 1N et on extrait avec 300 mL d'éther diéthylique. La phase organique est lavée 4 fois avec de la saumure. La phase organique est séchée sur du sulfate de sodium et évaporée. Le résidu est chromatographié sur gel de silice en éluant avec un gradient de méthanol dans le dichlorométhane variant de 0% à 5%. On obtient 5,04 g de 4-(4-tert-butoxycarbonylméthylènecyclohexyl)benzoate d'éthyle.
M+H⁺ = 345.

### 2.2 Synthèse du 4-(4-tert-butoxycarbonylméthylcyclohexyl)benzoate d'éthyle

On place 5,04 g de 4-(4-tert-butoxycarbonylméthylènecyclohexyl)benzoate d'éthyle (14,63 mmoles, 1 éq.) et 15 mL d'éthanol dans une bouteille de Parr. 0,31 g de palladium 10 % sur charbon (0,29 mmoles, 0,02 éq.) est ajouté et le milieu réactionnel est mis sous 50 psi d'hydrogène pendant 3 h à une température de 25°C. Le milieu réactionnel est filtré et concentré pour donner 4,31 g de 4-(4-tert-butoxycarbonylméthylcyclohexyl) benzoate d'éthyle.
M-isobutène⁺ = 291.

### 2.3 Synthèse de l'acide 4-(4-tert-butoxycarbonylméthylcyclohexyl) benzoïque

3,3 g de 4-(4-tert-butoxycarbonylméthylcyclohexyl) benzoate d'éthyle (9,52 mmoles, 1 éq.) sont dissous dans 30 mL d'un mélange 2/1 de tetrahydrofurane/méthanol, puis 1,60g d'hydroxyde de lithium hydrate (38,10 mmoles, 4 éq dissous dans 10 mL d'eau) sont ajoutés. Le milieu réactionnel est agité pendant 4 h à température ambiante. Les solvants sont évaporés et une solution aqueuse de SO₂ est ajoutée. Le solide obtenu est filtré, lavé à l'eau et séché pour donner 2 g d'acide 4-(4-tert-butoxycarbonylméthylcyclohexyl) benzoïque.

### 2.4. Synthèse de l'acide trans-4-(4-tert-butoxycarbonylméthylcyclohexyl)benzoïque

On recristallise 0,5 g d'acide 4-(4-tert-butoxycarbonylméthylcyclohexyl) benzoïque dans de l'acétate d'éthyle au reflux de la solution. Après filtration, et séchage, on obtient 0,17 g de *trans*-4-(4-tert-butoxycarbonylméthylcyclohexyl) benzoïque.

RMN ¹H (400 MHz, DMSO-d6) δ ppm 12,77 (m, 1H), 7,87 (m, J = 9 Hz, 2H), 7,36 (m, J = 9 Hz, 2H), 2,54 (m, 1 H), 2,13 (d, J = 7,3 Hz, 2H), 1,87 à 1,68 (m, 5H), 1,49 (m, 2H), 1,43 (s, 9H), 1,14 (m, 2H).

### Exemple 3 : acide 4-(4-éthylcarbonylméthylcyclohexyl)benzoïque

### 3.1 Synthèse du [4-(4-hydroxy-phényl)-cyclohexylidène]-acétate d'éthyle

Dans un ballon de 250 mL sous azote, on place 10 g de 4-(4-hydroxy-phényl)-cyclohexanone (52,56 mmoles, 1 éq.) dans 150 mL de tetrahydrofurane. La solution est refroidie à 4°C au bain de glace et 2,523 g d'hydrure de sodium à 60% (63,08 mmoles, 1,2 éq.) sont ajoutés par portions. Dans un autre ballon de 250 mL sous azote, on place 14,141 g de diéthylphosphoacétate d'éthyle (63,08 mmoles, 1,2 éq.) dans 150 mL de tetrahydrofurane. Cette seconde solution est refroidie au bain de glace et 2,523 g d'hydrure de sodium à 60% (63,08 mmoles, 1,2 éq.) sont ajoutés par portions. Les bains de glace sont retirés et les milieux sont agités à température ambiante pendant 30 minutes. La solution de 4-(4-hydroxy-phényl)-cyclohexanone est additionnée à la solution de diéthylphosphoacétate d'éthyle. Le milieu réactionnel est agité pendant 1 heure 30 minutes. Une solution aqueuse saturée de chlorure d'ammonium est ajoutée et le milieu réactionnel est extrait 3 fois à l'acétate d'éthyle. La phase organique est séchée sur du sulfate de magnésium, filtrée et évaporée pour donner 13,5 g de [4-(4-hydroxy-phényl)-cyclohexylidène]-acétate d'éthyle.
M+H⁺ = 261.

### 3.2 Synthèse du [4-(4-hydroxy-phényl)-cyclohexyl]-acétate d'éthyle

Dans une bouteille de Parr sous azote, on place 6,646 g de [4-(4-hydroxy-phényl)-cyclohexylidène]-acétate d'éthyle (26,53 mmoles, 1 éq.) dans 150 mL d'acétate d'éthyle. 0,77 g de palladium 10% sur charbon (0,72 mmole, 0,03 éq.) est ajouté et le milieu réactionnel est mis sous 50 psi d'hydrogène pendant 3 h à 25°C. Le milieu réactionnel est filtré et concentré pour donner 6,27 g de [4-(4-hydroxy-phényl)-cyclohexyl]-acétate d'éthyle.
M+CH₃CN⁺ = 304.

### 3.3 Synthèse du [4-(4-trifluorométhanesulfonyloxy-phényl)-cyclohexyl]-acétate d'éthyle

On place 6,27 g de [4-(4-hydroxy-phényl)-cyclohexyl]-acétate d'éthyle (23,90 mmoles, 1 éq.) dans 100 mL de dichlorométhane. La solution est refroidie au bain de glace et on ajoute successivement 6,3 mL de diisopropyléthylamine (35,85 mmoles, 1,5 éq.) et 4,4 mL d'anhydride triflique (26,29 mmoles, 1,1 éq.). Le bain de glace est retiré et le milieu réactionnel est agité 16 heures. Le milieu est versé sur de l'eau glacée et extrait avec 250 mL de dichlorométhane. La phase organique est séchée sur du sulfate de magnésium, filtrée et évaporée. Le résidu est chromatographié sur gel de silice en éluant avec un gradient d'acétate d'éthyle dans l'heptane variant de 5% à 10%. On obtient 5,76 g de [4-(4- trifluorométhanesulfonyloxy-phényl)-cyclohexyl]-acétate d'éthyle
M+CH₃CN⁺ = 436.

### 3.4 Synthèse de l'acide 4-(4-ethoxycarbonylmethyl-cyclohexyl)-benzoïque

Dans 3 tubes micro-onde de 20 mL, on place respectivement un tiers des 3 g de [4-(4-trifluorométhanesulfonyloxy-phényl)-cyclohexyl]-acétate d'éthyle (3 g, 7,61 mmoles, 1 éq.) dans un tiers des 18 mL de dioxane. On ajoute successivement et respectivement un tiers de chaque quantité de réactifs dans chaque tube de molybdène hexacarbonyl (1 g, 3,80 mmoles, 0,5 éq.), 0,171 g d'acétate de palladium (II) (0,76 mmole, 0,1 éq.), 0,422 g de 1,1'bis(diphénylphosphino)ferrocène (0,76 mmole, 0,1 éq.), 1,859 g de 4-diméthylaminopyridine (15,21 mmoles, 2 éq.), 3,1 mL de diisopropylethylamine (17,49 mmoles, 2,3 éq.) et 2,74 mL d'eau. Les tubes sont chauffés à 120°C pendant 20 minutes dans un appareil à micro-onde Biotage. Les milieux réactionnels sont filtrés sur célite. Le filtrat est dilué avec 200 mL de dichlorométhane et lavé avec 2 fois 100 mL d'une solution saturée de carbonate de sodium. 10 mL d'éther diéthylique sont ajoutés. Après décantation, la phase aqueuse est acidifiée à pH=1 à l'aide d'une solution d'acide chlorhydrique 5N et extrait avec 2 fois 200 mL de dichlorométhane. La phase organique est concentrée à sec pour donner 0,560 g d'acide 4-(4-ethoxycarbonylmethyl-cyclohexyl)-benzoïque.
M+CH₃CN⁺ = 332.

### Exemple 4: acide (4-{4-[5-(4-fluoro-benzyl)-[1,3,4]oxadiazol-2-ylcarbamoyl]-phényl}-cyclohexyl)-acétique (composé n°33 du tableau I)

### 4.1 Synthèse du (4-{4-[5-(4-fluoro-benzyl)-[1,3,4]oxadiazol-2-ylcarbamoyl]-phényl}-cyclohexyl)-acétate d'éthyle

On place 0,183 g de 5-(4-fluoro-benzyl)-[1,3,4]oxadiazol-2-ylamine (0,95 mmoles, 1,1 éq.) dans 5 mL d'acétonitrile à température ambiante sous agitation. On ajoute successivement sous agitation 0,25 g d'acide 4-(4-ethoxycarbonylmethyl-cyclohexyl)-benzoïque (0,86 mmoles, 1 éq.), 0,128 g d'hydroxybenzotriazole (0,95 mmoles, 1,1 éq.), 0,182 g de chlorhydrate de la 1-éthyl-3-(3-diméthylaminopropyl) carbodiimide (0,95 mmoles, 1,1 éq.). Après 18 h, le dichlorométhane est évaporé. Le résidu est chromatographié sur gel de silice en éluant avec un gradient d'acétate d'éthyle dans l'heptane. On obtient 0,37 g d'ester activé qui est placé dans un tube à micro-onde de 20 mL avec la 5-(4-fluoro-benzyl)-[1,3,4]oxadiazol-2-ylamine (0,183 g, 0,95 mmoles, 1,1 éq.) et 5 mL de diméthylformamide. Le tube est chauffé à 100°C pendant 30 minutes et à 120°C pendant 30 minutes dans un appareil à micro-onde Biotage. Le milieu réactionnel est dilué par de l'acétate d'éthyle, lavé 3 fois à l'eau et finalement avec de la saumure. La phase organique est séchée sur du sulfate de magnésium, filtrée et évaporée. Le résidu est chromatographié sur gel de silice en éluant avec un gradient d'acétate d'éthyle dans l'heptane variant de 20% à 33%. On obtient 0,1 g du (4-{4-[5-(4-fluoro-benzyl)-[1,3,4]oxadiazol-2-ylcarbamoyl]-phényl}-cyclohexyl)-acétate d'éthyle.
M+H⁺ = 466.

### 4.2 Synthèse de l'acide (4-{4-[5-(4-fluoro-benzyl)-[1,3,4]oxadiazol-2-ylcarbamoyl]-phényl}-cyclohexyl)-acétique

0,1 g de (4-{4-[5-(4-fluoro-benzyl)-[1,3,4]oxadiazol-2-ylcarbamoyl]-phényl}-cyclohexyl)-acétate d'éthyle (0,21 mmoles, 1 éq.) et 0,036 g d'hydroxyde de lithium hydrate (0,86 mmoles, 4 éq.) sont dissous dans 4 mL d'un mélange 2/1/1 de tetrahydrofurane/méthanol/eau refroidi à 4°C à l'aide d'un bain de glace. Le milieu réactionnel est agité pendant 4h à température ambiante. Les solvants sont évaporés et une solution aqueuse de SO₂ est ajoutée. Le solide obtenu est filtré, lavé à l'eau et séché pour donner 0,05 g d'acide (4-{4-[5-(4-fluoro-benzyl)-[1,3,4]oxadiazol-2-ylcarbamoyl]-phényl}-cyclohexyl)-acétique.
M+H⁺ = 438.
RMN ¹H (400 MHz, DMSO-d6) δ ppm 11,9 (m, 2H), 7,92 (d, J = 8,4 Hz, 1 H), 7,41 (m, 4H), 7,21 (m, J = 8,8 Hz, 2H), 4,28 (s, 2H), 2,63-2,12 (m, 3H), 1,90-1,43 (m, 7H), 1,14 (m, 2H).

### Exemple 5: acide (4-{4-[5-(4-fluorobenzyl)-[1,3,4]thiadiazol-2-ylcarbamoyl]phényl} cyclohexyl)acétique (Composé N° 5 du tableau I)

### 5.1 Synthèse du (4-{4-[5-(4-fluorobenzyl)-[1,3,4]thiadiazol-2-ylcarbamoyl]phényl} cyclohexyl)acétate de tert-butyle

On place 0,3 g d'acide 4-(4-tert-butoxycarbonylméthylcyclohexyl)benzoïque (0,94 mmoles, 1 éq.) dans un mélange de 5 mL de dichlorométhane et de 2 mL de diméthylformamide à température ambiante. On ajoute successivement 0,394 g de 5-(4-fluorobenzyl)-1,3,4-thiadiazol-2-amine (1,88 mmoles, 2 éq.), 1,054 g d'hexafluorophosphonate de bromo-tris-pyrrolidinophosphonium (2,26 mmoles, 2,4 éq.) et 0,66 mL de diisopropyléthylamine (3,76 mmoles, 4 éq.). Le mélange réactionnel est agité pendant 6 jours à température ambiante, dilué dans l'acétate d'éthyle, lavé avec une solution aqueuse saturée de chlorure d'ammonium, lavé 2 fois à l'eau et 1 fois à la saumure. La phase organique est concentrée et le résidu est chromatographié sur gel de silice en éluant avec un gradient d'acétate d'éthyle dans l'heptane variant de 10% à 33%. On obtient 0,35 g de (4-{4-[5-(4-fluorobenzyl)-[1,3,4]thiadiazol-2-ylcarbamoyl]phényl}cyclohexyl)acétate de tert-butyle.
M+H⁺ = 510

### 5.2 Synthèse de l'acide (4-{4-[5-(4-fluorobenzyl)-[1,3,4]thiadiazol-2-ylcarbamoyl]phényl} cyclohexyl)acétique

On place 0,35 g de (4-{4-[5-(4-fluorobenzyl)-[1,3,4]thiadiazol-2-ylcarbamoyl]phényl} cyclohexyl)acétate de tert-butyle (0,69 mmol, 1 éq.) dans 5 mL de dichlorométhane. 1 mL d'acide trifluoroacétique (13,46 mmoles, 19,6 éq.) est ajouté et le milieu réactionnel est agité 18 heures à température ambiante. Le solvant est évaporé et le résidu est repris par de l'acétate d'éthyle, de l'éthanol et du méthanol pour donner 117 mg d'acide (4-{4-[5-(4-fluorobenzyl)-[1,3,4]thiadiazol-2-ylcarbamoyl]phényl}cyclohexyl)acétique.
M+H⁺ = 454
RMN ¹H (400 MHz, DMSO-d6) δ ppm 13,02 à 11,66 (m, 2H), 8,01 (m, 2H), 7,46 à 7,36 (m, 4H), 7,18 (m, 2H), 4,37 (s, 2H), 2,69 à 2,51 (m, 1 H), 2,41 à 2,10 (m, 2H), 1,89 à 1 (m, 9H).

### Exemple 6: acide trans 4-[4-(5-benzyl-[1,3,4]thiadiazol-2-ylcarbamoyl)-phenoxy]-cyclohexanecarboxylique (Composé N° 53 du tableau II)

### 6.1 Synthèse du cis-4-hydroxy-cyclohexanecarboxylate de méthyle

On place 2 g d'acide cis-4-hydroxy-cyclohexanecarboxylique (13,87 mmoles, 1 éq.) dans 50 mL d'un mélange comprenant 4/1 de toluène/méthanol à température ambiante. On coule le triméthylsilyl diazométhane (111 mL, 221,96 mmoles, 16 éq.) sur le milieu réactionnel sous agitation. Après 3 heures à température ambiante, le milieu réactionnel est évaporé pour donner 2,6 g de cis-4-hydroxy-cyclohexanecarboxylate de méthyle.

### 6.2 Synthèse du trans-4-(4-methoxycarbonyl-cyclohexyloxy)-benzoate de tert-butyle

On place 2,20 g de cis-4-hydroxy-cyclohexanecarboxylate de méthyle (13,88 mmoles, 1 éq.), 2,70 g de 4-hydroxy-benzoate de tert-butyle (13,88 mmoles, 1 éq.) et 5,46 g de triphénylphosphine (20,82 mmoles, 1,5 éq.) dans 30 mL de tetrahydrofurane à température ambiante. 4,04 mL de diisopropyl azodicarboxylate (20,82 mmoles, 1,5 éq.) est ajouté goutte à goutte sur le milieu réactionnel sous agitation. Après 3 heures à température ambiante, le milieu est concentré à sec et repris dans de l'éther diéthylique. La triphénylphosphine oxyde est filtrée. La phase organique est lavée successivement avec une solution d'hydroxyde de sodium puis avec de l'eau, séchée sur sulfate de sodium, filtrée et évaporée pour donner un résidu. Celui-ci est chromatographié sur gel de silice en éluant avec un gradient d'acétate d'éthyle dans l'heptane variant de 10% à 50 %. On obtient 1,6 g de trans-4-(4-methoxycarbonyl-cyclohexyloxy)-benzoate de tert-butyle.

### 6.3 Synthèse de l'acide trans-4-(4-methoxycarbonyl-cyclohexyloxy)-benzoïque

On place 1,6 g de trans-4-(4-methoxycarbonyl-cyclohexyloxy)-benzoate de tert-butyle (4,78 mmoles, 1 éq.) dans du dichlorométhane. Le milieu réactionnel est mis à refroidir jusqu'à une température 4°C sous agitation dans un bain de glace. 3 mL d'acide trifluoroacétique (40,39 mmoles, 8,44 éq.) sont ajoutés et le bain de glace est retiré. Après 17 h d'agitation à température ambiante, le milieu est concentré, repris par de l'éther diéthylique et filtré pour donner 1,0 g d'acide trans-4-(4-methoxycarbonyl-cyclohexyloxy)-benzoïque.
PF = 162°C.

### 6.4 Synthèse du trans-4-[4-(5-benzyl-[1,3,4]thiadiazol-2-ylcarbamoyl)-phénoxyl-cyclohexanecarboxylate de méthyle

On place 0,301 g d'acide trans-4-(4-méthoxycarbonyl-cyclohexyloxy)-benzoïque (1,08 mmoles, 1 éq.) dans 5 mL de dichlorométhane à température ambiante. On ajoute successivement 0,36 mL de diisopropyléthylamine (2,16 mmoles, 2 éq.) et 0,604 g d'hexafluorophosphonate de bromo-tris-pyrrolidinophosphonium (1,30 mmoles, 1,2 éq.), 0,207 g de 5-benzyl-1,3,4-thiadiazol-2-amine (1,08 mmoles, 1 éq.). On ajoute ensuite 2 mL de DMF. Le mélange réactionnel est agité pendant 18 heures à température ambiante. On ajoute 0,1 mL de diisopropyléthylamine (0.6 mmoles, 0.55 éq.), 0,2 g d'hexafluorophosphonate de bromo-tris-pyrrolidinophosphonium (0,43 mmoles, 0 ,4 éq.) et 0,05 g de 5-benzyl-1,3,4-thiadiazol-2-amine (0,26 mmoles, 0,24 éq.). Le milieu réactionnel est agité à 50°C pendant 6h, dilué dans le dichlorométhane, lavé à l'eau et avec une solution aqueuse saturée de bicarbonate de sodium. La phase organique est séchée sur sulfate de magnésium, concentrée et le résidu est chromatographié sur gel de silice en éluant avec un gradient d'acétate d'éthyle dans l'heptane variant de 10% à 70%. Les fractions d'intérêt sont évaporées. Le solide obtenu est trituré dans l'éthanol pour donner 0,128 g de trans-4-[4-(5-benzyl-[1,3,4]thiadiazol-2-ylcarbamoyl)-phénoxy]-cyclohexanecarboxylate de méthyle.
M + H⁺ = 452.

### 6.5 Synthèse de l'acide trans-4-[4-(5-benzyl-[1,3,4]thiadiazol-2-ylcarbamoyl)-phénoxy]-cyclohexanecarboxylique

On place 0,128 g de 4-[4-(5-benzyl-[1,3,4]thiadiazol-2-ylcarbamoyl)-phénoxy]-cyclohexanecarboxylate de méthyle (0,28 mmole, 1 éq.) dans 2 mL d'un mélange de tetrahydrofurane et d'eau à température ambiante. On ajoute 0,024 g d'hydroxyde de lithium monohydrate (0,57 mmole, 2 éq.) dans 1 mL d'eau. Après 2 h d'agitation à température ambiante, le tetrahydrofurane est évaporé et de l'eau est ajoutée. La phase aqueuse est lavée par du dichlorométhane. La phase aqueuse est refroidie jusqu'à une température de 4°C dans un bain de glace et acidifiée à l'aide d'une solution 1 N d'acide chlorhydrique. Le solide est filtré, lavé successivement à l'eau, à l'éthanol puis à l'éther diéthylique pour donner 0,069 g de l'acide 4-[4-(5-benzyl-[1,3,4]thiadiazol-2-ylcarbamoyl)-phenoxy]-cyclohexanecarboxylique.
M + H⁺ = 438.
RMN ¹H (400 MHz, DMSO-d6) δ ppm: 13,92-11,69 (m, 2H), 8,06 (m, J = 9 Hz, 2H), 7,37 (m, 4H), 7,30 (m, 1 H), 7,08 (m, J = 9 Hz, 2H), 4,48 (m, 1 H), 4,38 (s, 2H), 2,28 (m, 1 H), 2,09 (m; 2H), 1,96 (m, 2H), 1,56 (m, 2H), 1,43 (m, 2H).

### Exemple 7: acide cis-{4-[4-(5-benzyl-[1,3,4]thiadiazol-2-ylcarbamoyl)-phényl]-cyclohexyl}-acétique (Composé N° 34 du tableau I)

### 7.1 Synthèse du {4-[4-(5-benzyl-[1,3,4]thiadiazol-2-ylcarbamoyl)-phényl]-cyclohexyl}-acétate de tert-butyle

On place 0,45 g d'acide 4-(4-tert-butoxycarbonylméthylcyclohexyl)benzoïque (1,41 mmoles, 1 éq.) dans un mélange de 5 mL de diméthylformamide à température ambiante. On ajoute successivement 0,539 g de 5-benzyl-1,3,4-thiadiazol-2-amine (2,82 mmoles, 2 éq.), 0,791 g d'hexafluorophosphonate de bromo-tris-pyrrolidinophosphonium (1.70 mmoles, 1.2 éq.) et 0,49 mL de diisopropyléthylamine (2,83 mmoles, 2 éq.). Le mélange réactionnel est agité 16 h à température ambiante, dilué dans l'acétate d'éthyle et lavé 3 fois à l'eau. La phase organique est concentrée. Le résidu est repris et lavé par de l'éthanol, filtré et séché. On obtient 0,236 g de {4-[4-(5-benzyl-[1,3,4]thiadiazol-2-ylcarbamoyl)-phényl]-cyclohexyl}-acétate de tert-butyle.
M+H+ = 492.

### 7.2 Synthèse de l'acide cis-(4-{4-benzyl-[1,3,4]thiadiazol-2-ylcarbamoyl}phényl}cyclohexyl)acétique

On place 0,35 g de {4-[4-(5-benzyl-[1,3,4]thiadiazol-2-ylcarbamoyl)-phényl]-cyclohexyl}-acétate de tert-butyle (0,48 mmol, 1 éq.) dans 3 mL de dichlorométhane. 1 mL d'acide trifluoroacétique (13,46 mmoles, 28 éq.) est ajouté et le milieu réactionnel est agité 18 heures à température ambiante. Le solvant est évaporé et le résidu est repris par de l'acétate d'éthyle pour donner après filtration et séchage 194 mg d'acide (4-{4-[5-(4-fluorobenzyl)-[1,3,4]thiadiazol-2-ylcarbamoyl]phényl}cyclohexyl)acétique.

L'acide cis-{4-[4-(5-benzyl-[1,3,4]thiadiazol-2-ylcarbamoyl)-phényl]-cyclohexyl}-acétique est obtenu par séparation chromatographique en utilisant une phase stationnaire Chiralcel OJ-H 250*21 mm (5 µm) et une phase mobile CO₂ (MeOH + 0.5% d'isopropylamine). On obtient un premier isomère, 27 mg de l'acide trans-{4-[4-(5-benzyl-[1,3,4]thiadiazol-2-ylcarbamoyl)-phényl]-cyclohexyl}acétique
M+H+ = 436.

Un second isomère, 70mg de l'acide cis-{4-[4-(5-benzyl-[1,3,4]thiadiazol-2-ylcarbamoyl)-phényl]-cyclohexyl}-acétique.
M+H+ = 436.
RMN 1 H (400 MHz, DMSO-d6) d ppm 8.01 (m, 2H), 7.37 to 7.21 (m, 9H), 4.21 (s, 2H), 2.59 (m, 1 H), 2.34 (m, 2H), 2.18 (m, 1 H), 1.63 (m, 8H)

### Exemple 8: acide trans-{4-[4-(5-benzyl-[1,3,4]thiadiazol-2-ylcarbamoyl)-phényl]-cyclohexyl}-acétique (Composé N° 1 du tableau I)

### 8.1 Synthèse du trans-{4-[4-(5-benzyl-[1,3,4]thiadiazol-2-ylcarbamoyl)-phényl]-cyclohexyl}-acétate de tert-butyle

Dans un ballon de 250 mL sous atmosphère d'azote, on place 3 g d'acide trans-4-(4-tert-butoxycarbonylméthylcyclohexyl)benzoïque (9,42 mmol, 1 éq.) dans 35 mL d'un mélange de dichlorométhane et de diméthylformamide à température ambiante. On ajoute sous agitation successivement 1,528 g d'hydroxybenzotriazole (11,1 mmol, 2 éq.), 2,168 g de chlorhydrate de la 1-éthyl-3-(3-diméthylaminopropyl) carbodiimide (11,1 mmol, 2 éq.), 3,11 mL de diisopropyléthylamine (18,84 mmol, 2 éq.) et 2,162 g de 5-benzyl-1,3,4-thiadiazol-2-amine (11,1 mmol, 2 éq.). Le mélange réactionnel est agité pendant 4 jours à température ambiante. Le milieu réactionnel est évaporé à sec et dilué dans l'eau. Le précipité obtenu est filtré et lavé à l'eau. Le solide obtenu est solubilisé dans un minimum de dichlorométhane et chromatographié sur gel de silice en éluant avec un gradient d'acétate d'éthyle dans le dichlorométhane variant de 2,5% à 25%. On obtient 3,13 g de trans-{4-[4-(5-benzyl-[1,3,4]thiadiazol-2-ylcarbamoyl)-phényl]-cyclohexyl}-acétate de tert-butyle.
M+H+ = 492.

### 8.2 Synthèse de l'acide trans-(4-{4-benzyl-[1,3,4]thiadiazol-2-ylcarbamoyl}phényl}cyclohexyl)acétique

Dans un ballon de 100 mL, on place 2,11 g de {4-[4-(5-benzyl-[1,3,4]thiadiazol-2-ylcarbamoyl)-phényl]-cyclohexyl}-acétate de tert-butyle (4,29 mmol, 1 éq.) dans 15 mL de dichlorométhane. 2,55 mL d'acide trifluoroacétique (34,33 mmol, 8 éq.) sont ajoutés et le milieu réactionnel est agité 18 heures à température ambiante. 1 mL d'acide trifluoroacétique (13,46 mmol, 3,14 éq.) est ajouté et le milieu réactionnel est agité 3 heures. Le solvant est évaporé. Le résidu est repris et lavé successivement par de l'éther diéthylique et de l'éthanol pour donner 1,718 g d'acide trans-(4-{4-benzyl-[1,3,4]thiadiazol-2-ylcarbamoyl}phényl} cyclohexyl) acétique.
M+H+ = 436.

RMN ¹H (400 MHz, DMSO-d6) δ ppm 12.46 (m, 2H), 8.02 (d, J = 8 Hz, 2H), 7.41 (d, J = 8 Hz, 2H), 7.38 (m, 4H), 7.30 (m, 1 H), 4.39 (s, 2H), 2.57 (tt, J = 12 Hz and 3.4 Hz, 1 H), 2.16 (d, J = 6.8 Hz, 2H), 1.89 to 1.70 (m, 5H), 1.51 (m, 2H), 1.14 (m, 2H).

### Exemple 9 : acide cis et trans-4-[4-(5-benzyl-[1,3,4]thiadiazol-2-ylcarbamoyl)-phénylamino]-cyclohexanecarboxylique (Composés N° 68 et 69 du tableau III)

### 9.1 Synthèse du [4-(5-benzyl-[1,3,4]thiadiazol-2-ylcarbamoyl)-phényl]-carbamate de tert-butyle

On place 1 g d'acide 4-tert-butoxycarbonylaminobenzoïque (4,21 mmol, 1 éq.) dans 12 mL d'un mélange 5/1 de dichlorométhane et de diméthylformamide sous agitation. On ajoute successivement, 0,81 g de 5-benzyl-1,3,4-thiadiazol-2-amine (4,21 mmol, 1 éq.), 0,97 g du chlorhydrate de la 1-éthyl-3-(3-diméthylaminopropyl) carbodiimide (5,05 mmol, 1,2 éq.), 0,97 g d'hydroxybenzotriazole (6,32 mmol, 1,5 éq.) et 1,74 mL de diisopropyléthylamine (10,52 mmol, 2,5 éq.). Après 42 heures à température ambiante, le milieu est chauffé à 60°C pendant 6 heures. On ajoute 0,40 g de chlorhydrate de la 1-éthyl-3-(3-diméthylaminopropyl) carbodiimide (2,1 mmol, 0,5 éq.) et 0,322 g d'hydroxybenzotriazole (3,16 mmol, 0,5 éq.). Après 18 heures, le milieu est dilué au dichlorométhane et à l'eau. La phase organique est décantée, lavée à l'eau, séchée sur du sulfate de magnésium, filtrée et évaporée. Le résidu est chromatographié sur gel de silice avec un gradient d'acétate d'éthyle dans l'heptane variant de 0% à 50 %. La phase organique est évaporée pour donner 0,2 g de [4-(5-benzyl-[1,3,4]thiadiazol-2-ylcarbamoyl)-phényl]-carbamate de tert-butyle.
M+H⁺ = 411.

### 9.2 Synthèse du 4-amino-N-(5-benzyl-[1,3,4]thiadiazol-2-yl)-benzamide

On place 0,2 g de [4-(5-benzyl-[1,3,4]thiadiazol-2-ylcarbamoyl)-phényl]-carbamate de tert-butyle (0,49 mmol, 1 éq.) dans 5 mL de dichlorométhane. 1 mL d'acide trifluoroacétique (13,54 mmol, 27,6 éq.) est ajouté et le milieu réactionnel est agité 3 jours à température ambiante. Les solvants sont évaporés et le résidu est repris par de l'ether diéthylique et du pentane pour donner 0,14 g d'acide 4-amino-N-(5-benzyl-[1,3,4]thiadiazol-2-yl)-benzamide.
M+H⁺ = 311.

### 9.3 Synthèse du cis- et trans-4-[4-(5-benzyl-[1,3,4]thiadiazol-2-ylcarbamoyl)-phénylamino]-cyclohexanecarboxylate d'éthyle

On place 0,14 g d'acide 4-amino-N-(5-benzyl-[1,3,4]thiadiazol-2-yl)-benzamide (0,4 mmol, 1 éq.) et 1,5 mL de dichloroéthane dans un tube micro-onde. 0,134 g de 4-oxocyclohexanecarboxylate d'éthyle (0,79 mmol, 2 éq.), 0,15 g de triacétoxyborohydrure de sodium (0,99 mmol, 2,5 éq.) et 0,07 mL d'acide acétique sont ajoutés successivement sous agitation. Le tube est scellé et porté à 140°C pendant 20 minutes. Le milieu réactionnel est repris par de l'eau et de l'acétate d'éthyle. Après décantation, la phase organique est séchée sur sulfate de magnésium, filtrée et évaporée. Le résidu est chromatographié sur gel de silice avec un gradient d'acétate d'éthyle dans l'heptane variant de 10% à 50 %.
On obtient une première fraction de 50 mg de cis-4-[4-(5-benzyl-[1,3,4]thiadiazol-2-ylcarbamoyl)-phénylamino]-cyclohexanecarboxylate d'éthyle.
M+H⁺ = 465.

On obtient une seconde fraction de 20 mg de trans-4-[4-(5-benzyl-[1,3,4]thiadiazol-2-ylcarbamoyl)-phénylamino]-cyclohexanecarboxylate d'éthyle.
M+H⁺ = 465.

### 9.4 Synthèse de l'acide trans-4-[4-(5-benzyl-[1,3,4]thiadiazol-2-ylcarbamoyl)-phénylamino]-cyclohexanecarboxylique

On place 50 mg de trans-4-[4-(5-benzyl-[1 ,3,4]thiadiazol-2-ylcarbamoyl)-phénylamino]-cyclohexanecarboxylate d'éthyle (0,11 mmol, 1 éq.) dans 1 mL de tetrahydrofurane. 9 mg d'hydroxyde de lithium monohydrate (0,22 mmol, 2 éq.) dissous dans 1 mL sont ajoutés et l'agitation est poursuivie pendant 18 heures. Le milieu réactionnel est dilué par de l'eau, lavé à l'acétate d'éthyle et acidifié par une solution aqueuse à 6% d'anhydride sulfureux. Le solide obtenu est essoré, lavé successivement à l'eau, à l'éthanol et à l'éther diéthylique. On obtient 13 mg de l'acide trans-4-[4-(5-benzyl-[1,3,4]thiadiazol-2-ylcarbamoyl)-phénylamino]-cyclohexanecarboxylique.
M+H⁺ = 437.
RMN ¹H (400 MHz, DMSO-d6) δ ppm 12.20 (m, 2H), 7.87 (d, J = 9 Hz, 2H), 7.36 (m, 4H), 7.29 (m, 1 H), 6.62 (d, J = 9 Hz, 2H), 6.41 (d, J = 7.8 Hz, 1 H), 4.36 (s, 2H), 2.21 (tt, J = 12 Hz and 3.4 Hz, 1 H), 1.97 (m, 4H), 1.49 (m, 2H), 1.21 (m, 3H).

### 9.5 Synthèse de l'acide cis-4-[4-(5-benzyl-[1,3,4]thiadiazol-2-ylcarbamoyl)-phénylamino]-cyclohexanecarboxylique

Ce composé est obtenu selon la préparation 9.4 à partir du cis-4-[4-(5-benzyl-[1,3,4]thiadiazol-2-ylcarbamoyl)-phénylamino]-cyclohexanecarboxylate d'éthyle.
M+H⁺ = 437.
RMN ¹H (400 MHz, DMSO-d6) δ ppm 12.23 (m, 2H), 7.88 (d, J = 9 Hz, 2H), 7.37 (m, 4H), 7.30 (m, 1 H), 6.65 (d, J = 9 Hz, 2H), 6.45 (d, J = 7.6 Hz, 1 H), 4.36 (s, 2H), 3.49 (m, 1 H), 2.45 (m, 1 H), 1.91 (m, 1 H), 1.77 to 1.47 (m, 6H).

### Exemple 10 : acide 6-[4-(5-benzyl-[1,3,4]thiadiazol-2-ylcarbamoyl)-phényl]-spiro[2.5]octane-1-carboxylique (intermédiaire dans la synthèse du composé 43 du tableau V)

### 10.1 Synthèse du 6-(4-éthoxycarbonyl-phényl)-spiro[2.5]octane-1-carboxylate de tert-butyle

Dans un tricol de 250 mL sous atmosphère d'azote, 5,11 g d'iodure de triméthylsulfonium (23,23 mmol, 4 éq.) sont solubilisés sous agitation dans 20 mL de DMSO. 2,61 g de tert-butoxyde de potassium (23,23 mmol, 4 éq.) sont ajoutés et le milieu réactionnel est agité 3 heures. 2 g de 4-(4-tert-butoxycarbonylméthylène-cyclohexyl)-benzoate d'éthyle solubilisé dans 5 mL de DMSO sont ajoutés et l'agitation est poursuivie pendant 2 jours. Dans un tricol de 25 mL sous atmosphère d'azote, 2,56 g d'iodure de triméthylsulfonium (11,61 mmol, 2 éq.) sont solubilisés dans 20 mL de DMSO et 1.0 g de tert-butoxyde de potassium (11,61 mmol, 2 éq.) sont ajoutés sous agitation. Après 3 heures, ce milieu réactionnel est rajouté dans le tricol de 250 mL et l'agitation est poursuivie pendant 18 heures. On ajoute de l'acétate d'éthyle. La phase organique est lavée par une solution aqueuse saturée de chlorure d'ammonium, séchée sur sulfate de magnésium et filtrée. Le résidu est chromatographié sur gel de silice avec un gradient d'acétate d'éthyle dans l'heptane variant de 10% à 50%. On obtient 0,159 g de 6-(4-éthoxycarbonyl-phényl)-spiro[2.5]octane-1-carboxylate de tert-butyle.

### 10.2 Synthèse du 6-(4-carboxy-phényl)-spiro[2.5]octane-1-carboxylate de tert-butyle

Dans un ballon de 50 mL, on solubilise 0,159g de 6-(4-éthoxycarbonyl-phényl)-spiro[2.5]octane-1-carboxylate de tert-butyle (0,44 mmol, 1 éq.) dans 4 mL d'un mélange 3/1 de THF/méthanol. 0,075 g d'hydroxyde de lithium monohydrate (1,78 mmol ; 4 éq.) solubilisé dans 1 mL d'eau est ajouté sous agitation. Après 16 heures à température ambiante, le milieu réactionnel est évaporé et une solution aqueuse à 6% d'anhydride sulfureux est ajoutée. Le précipité est filtré pour obtenir 0,122 g de 6-(4-carboxy-phényl)-spiro[2.5]octane-1-carboxylate de tert-butyle.

### 10.3 Synthèse du 6-[4-(5-benzyl-[1,3,4]thiadiazol-2-ylcarbamoyl)-phényl]-spiro[2.5]octane-1-carboxylate de tert-butyle

Ce composé est obtenu selon la préparation 8.1. à partir du 6-(4-carboxy-phényl)-spiro[2.5]octane-1-carboxylate de tert-butyle.
M+H⁺ = 504.

### 10.4 Synthèse de l'acide 6-[4-(5-benzyl-[1,3,4]thiadiazol-2-ylcarbamoyl)-phényl]-spiro[2.5]octane-1-carboxylique

Ce composé est obtenu selon la préparation 8.2 à partir du 6-[4-(5-benzyl-[1,3,4]thiadiazol-2-ylcarbamoyl)-phényl]-spiro[2.5]octane-1-carboxylate de tert-butyle. M+H⁺ = 448.
RMN ¹H (400 MHz, DMSO-d6) δ ppm 12.82 (m, 2H), 8.04 (m, 2H), 7.48 to 7.25 (m, 7H), 4.39 (s, 1 H), 2.72 (m, 1 H), 2.05 to 1.46 (m, 7H), 1.29 (m, 1 H), 1.12 to 0.89 (m, 3H).

### Exemple 11 : acide (E)-3-{4-[4-(5-benzyl-[1,3,4]thiadiazol-2-ylcarbamoyl)-phényl]-cyclohexyl}-acrylique (Composé N° 71 tableau IV)

### 11.1 Synthèse du 4-(1-oxa-spiro[2.5]oct-6-yl)-benzoate d'éthyle

On place 0,246 g d'hydrure de sodium (9,74 mmol, 2 éq.) dans 25 mL d'un mélange THF/DMSO 1/1.5 sous agitation à 10°C. On ajoute 2,14 g d'iodure de triméthylsulfoxonium (9,74 mmol, 1,2 éq.). Après 5 minutes, on ajoute goutte à goutte 10 mL de DMSO. Après le dégagement gazeux, 2 g de 4-(4-oxo-cyclohexyl)-benzoate d'éthyle (8,12 mmol, 1 éq.) solubilisé dans un minimum de mélange DMSO/THF 2/1 sont ajoutés rapidement à température ambiante. Après 1 heure d'agitation à température ambiante et 1 heure à 50°C, le milieu réactionnel est dilué par 75 mL d'eau et extrait avec de l'acétate d'éthyle. Les phases organiques sont successivement lavées 2 fois à l'eau, lavées par de la saumure, séchées sur du sulfate de sodium et évaporées. Le résidu est chromatographié sur gel de silice avec un gradient de méthanol dans le dichlorométhane variant de 1 % à 2 %. On obtient 1,8 g de 4-(1-oxaspiro[2.5]oct-6-yl)-benzoate d'éthyle.

### 11.2 Synthèse du 4-(4-formyl-cyclohexyl)-benzoate d'éthyle

On place 1,4 g de 4-(1-oxa-spiro[2.5]oct-6-yl)-benzoate d'éthyle (5,38 mmol, 1,4 éq.) dans 40 mL de dichlorométhane à 4°C. 0,229 g d'éthérate de trifuoroborane (1,61 mmol, 0,3 éq.) est ajouté sous agitation. Après 2 heures, 0,153 g d'éthérate de trifluoroborane (1,07 mmol, 0,2 éq.) est ajouté. Après 1 heure, on additionne de l'eau et on extrait 3 fois le milieu réactionnel avec du dichlorométhane. Les phases organiques sont rassemblées et lavées successivement avec une solution aqueuse saturée d'hydrogénocarbonate de sodium et d'eau. La phase organique est séchée sur du sulfate de sodium et évaporée. Le résidu est chromatographié sur gel de silice avec un mélange heptane/acétate d'éthyle/méthanol 88/10/2. On obtient 0,64 g de trans-4-(4-formyl-cyclohexyl)-benzoate d'éthyle.

### 11.3 Synthèse du trans-4-[4-(E)-2-tert-butoxycarbonyl-vinyl)-cyclohexyl]-benzoate d'éthyle

On place 0,682 g de diéthylphosphonoacétate de tert-butyle (2,7 mmol, 1,1 éq.) dans 5 mL de DMF à 4°C sous agitation. 0,065 g d'hydrure de sodium (2,7 mmol, 1,1 éq.) est ajouté. Après 1 heure d'agitation, 0,64 g de trans-4-(4-formyl-cyclohexyl)-benzoate d'éthyle (2,46 mmol, 1 éq.) solubilisé dans 5 mL de DMF est ajouté goutte à goutte. Après 18 heures d'agitation, une solution à 10% d'hydrogénosulfate de potassium est ajoutée. Le milieu réactionnel est extrait 2 fois à l'aide d'acétate d'éthyle. Les phases organiques sont rassemblées, lavées 2 fois à l'eau, lavées à la saumure, séchées sur du sulfate de sodium et évaporées. Le résidu est chromatographié sur gel de silice avec un gradient d'acétate d'éthyle dans l'heptane variant de 10% à 20%. On obtient 0,71 g de trans-4-[4-((E)-2-tert-butoxycarbonyl-vinyl)-cyclohexyl]-benzoate d'éthyle.

### 11.4 Synthèse de l'acide trans-4-[4-(E)-2-tert-butoxycarbonyl-vinyl)-cyclohexyl]-benzoïque

On solubilise 0,150g de trans-4-[4-(E)-2-tert-butoxycarbonyl-vinyl)-cyclohexyl]-benzoate d'éthyle (0,42 mmol, 1 éq.) dans 3 mL d'un mélange 2/1 de THF/méthanol. A 0°C, 0,07 g d'hydroxyde de lithium monohydrate (1,67 mmol ; 4 éq.) est ajouté sous agitation. Après 3 heures à température ambiante, le milieu réactionnel est évaporé et une solution aqueuse à 6% d'anhydride sulfureux est ajoutée. Le précipité est filtré et lavé à l'eau pour obtenir 0,085 g d'acide trans-4-[4-(E)-2-tert-butoxycarbonyl-vinyl)-cyclohexyl]-benzoïque.

### 11.5 Synthèse du trans-(E)-3-{4-[4-(5-benzyl-[1,3,4]thiadiazol-2-ylcarbamoyl)-phényl]-cyclohexyl}-acrylate de tert-butyle

On place 0,85 g d'acide trans-4-[4-(E)-2-tert-butoxycarbonyl-vinyl)-cyclohexyl]-benzoïque (0,26 mmol, 1 éq.) dans 2 mL de DMF à température ambiante. On ajoute successivement 0,059 g de 5-benzyl-1,3,4-thiadiazol-2-amine (0,31 mmol, 1,2 éq.), 0,144 g d'hexafluoro-phosphonate de bromo-tris-pyrrolidinophosphonium (0,31 mmol, 1,2 éq.) et 0,09 mL de diisopropyléthylamine (0,51 mmol, 2 éq.). Le mélange réactionnel est agité pendant 1 jour à température ambiante, dilué dans l'acétate d'éthyle, lavé 2 fois à l'eau et 2 fois à la saumure. La phase organique est concentrée séchée sur du sulfate de sodium et le résidu est chromatographié sur gel de silice en éluant avec un gradient d'acétate d'éthyle dans l'heptane variant de 20% à 33%. On obtient 0,035 g de trans-(E)-3-{4-[4-(5-benzyl-[1,3,4]thiadiazol-2-ylcarbamoyl)-phenyl]-cyclohexyl}-acrylate de tert-butyle.
M+H⁺ = 504.

### 11.6 Synthèse de l'acide trans-(E)-3-{4-[4-(5-benzyl-[1,3,4]thiadiazol-2-ylcarbamoyl)-phényl]-cyclohexyl}-acrylique

On place 35 mg de trans-(E)-3-{4-[4-(5-benzyl-[1,3,4]thiadiazol-2-ylcarbamoyl)-phényl]-cyclohexyl}-acrylate de tert-butyle (0,07 mmol, 1 éq.) dans 1 mL de dichlorométhane. 0,5 mL d'acide trifluoroacétique (6,73 mmol, 97 éq.) sont ajoutés et le milieu réactionnel est agité 3 heures à température ambiante. Le solvant est évaporé. Le résidu est trituré dans l'acétate d'éthyle pour donner 8 mg d'acide trans-(E)-3-{4-[4-(5-benzyl-[1,3,4]thiadiazol-2-ylcarbamoyl)-phényl]-cyclohexyl}-acrylique.
M+H⁺ = 448.
RMN ¹H (400 MHz, DMSO-d6) δ ppm 12.55 (m, 2H), 8.03 (d, J = 8.4 Hz, 2H), 7.42 (d, J = 8.4 Hz, 2H), 7.38 (m, 4H), 7.30 (m, 1 H), 6.84 (m, 1 H), 5.77 (dd, J = 15.6 Hz and 3.4 Hz, 1 H), 4.39 (s, 2H), 2.61 (m, 1 H), 2.26 (m, 1 H), 1.88 (m, 4H), 1.57 (m, 2H), 1.32 (m, 2H).

### Exemple 12: acide trans-(1R,2S/1S,2R)-2-{4-[4-(5-benzyl-[1,3,4]thiadiazol-2-ylcarbamoyl)-phenyl]-cyclohexyl}-cyclopropanecarboxylique (intermédiaire dans la synthèse du composé 72 du tableau IV)

### 12.1 Synthèse du trans-4-[4-((1S,2R/1R,2S)-2-tert-butoxycarbonyl-cyclopropyl)-cyclohexyl]-benzoate d'éthyle

On place 0,442 g d'iodure de triméthylsulfoxonium (2,01 mmol, 1,8 éq.) dans 3 mL de DMSO. Sous agitation, 0,225 g de tert-butoxyde de potassium (2,01 mmol, 1,8 éq.) est ajouté. Après 3 heures à température ambiante, 0,4 g de trans-4-[4-(©-2-tert-butoxycarbonyl-vinyl)-cyclohexyl]-benzoate d'éthyle (1,12 mmol, 1 éq.) solubilisé dans 2 mL de DMSO est ajouté et l'agitation est maintenue pendant 18 heures. On ajoute successivement de la saumure et une solution saturée de chlorure d'ammonium. Le milieu réactionnel est extrait 3 fois à l'aide d'acétate d'éthyle. Les phases organiques sont rassemblées, lavées successivement 3 fois à l'eau, à la saumure, séchées sur du sulfate de sodium et évaporées pour donner 310 mg de trans-4-[4-((1S,2R/1R,2S)-2-tert-butoxycarbonyl-cyclopropyl)-cyclohexyl]-benzoate d'éthyle.

### 12.2 Synthèse de l'acide trans-4-[4-((1S,2R/1R,2S)-2-tert-butoxycarbonyl-cyclopropyl)-cyclohexyl]-benzoïque

Ce composé est obtenu selon la préparation 11.4 à partir du trans-4-[4-((1S,2R/1R,2S)-2-tert-butoxycarbonyl-cyclopropyl)-cyclohexyl]-benzoate d'éthyle. RMN ¹H (400 MHz, DMSO-d6) δ ppm 12.76 (m, 1 H), 7.86 (d, J = 8.8 Hz, 2H), 7.33 (d, J = 8.8 Hz, 2H), 2.57 (m, 1 H), 1.83 (m, 4H), 1.50 to 1.33 (m, 12H), 1.26 (m, 2H), 1.08 (m, 1 H), 0.96 to 0.74 (m, 3H).

### 12.3 Synthèse du trans-(1R,2S/1S,2R)-2-{4-[4-(5-benzyl-[1,3,4]thiadiazol-2-ylcarbamoyl)-phenyl]-cyclohexyl}-cyclopropanecarboxylate de tert-butyle

Ce composé est obtenu selon la préparation 11.5 à partir de l'acide trans-(1R,2S/1S,2R)-4-[4-(2-tert-butoxycarbonyl-cyclopropyl)-cyclohexyl]-benzoïque. M+H⁺ = 518.

### 12.4 Synthèse de l'acide trans-(1R,2S/1S,2R)-2-{4-[4-(5-benzyl-[1,3,4]thiadiazol-2-ylcarbamoyl)-phenyl]-cyclohexyl}-cyclopropanecarboxylique

Ce composé est obtenu selon la préparation 11.6 à partir du trans-(1R,2S/1S,2R)-2-{4-[4-(5-benzyl-[1,3,4]thiadiazol-2-ylcarbamoyl)-phényl]-cyclohexyl}-cyclopropanecarboxylate de tert-butyle.
M+H+ = 462.
RMN ¹H (400 MHz, DMSO-d6) δ ppm 12.81 (m, 1 H), 11.97 (m, 1 H), 8.01 (d, J = 8 Hz, 2H), 7.38 (m, 6H), 7.30 (m, 1 H), 4.39 (s, 2H), 2.59 (tt, J = 12 Hz and 3 Hz, 1 H), 1.85 (m, 4H), 1.50 to 1.20 (m, 5H), 1.10 (m, 1 H), 0.96 (m, 1H), 0.89 to 0.75 (m, 2H).

### Exemple 13 : acide trans-3-{4-[4-(5-benzyl-[1,3,4]thiadiazol-2-ylcarbamoyl)-phényl]-cyclohexyl}-propionique (intermédiaire dans la synthèse du composé 73 du tableau IV)

### 13.1 Synthèse du trans-4-[4-(2-tert-butoxycarbonyl-2thyl)-cyclohexyl]-benzoate d'éthyle

On solubilise 0,150 g de trans-4-[4-((E)-2-tert-butoxycarbonyl-vinyl)-cyclohexyl]-benzoate d'éthyle (0,42 mmol, 1 éq.) à l'aide de 15 mL d'éthanol dans une bouteille de Parr. On ajoute 0,044g de palladium 10% sur charbon (0,04 mmol, 0,1 éq.) et on soumet le milieu à 50 psi d'hydrogène pendant 5 heures. Le milieu est filtré sur papier Whatman, lavé à l'éthanol et évaporé pour donner 0,19 g de trans 4-[4-(2-tert-butoxycarbonyl-éthyl)-cyclohexyl]-benzoate d'éthyle.

### 13.2 Synthèse de l'acide trans-4-[4-(2-tert-butoxycarbonyl-éthyl)-cyclohexyl]-benzoïque

Ce composé est obtenu selon la préparation 11.4 à partir de trans-4-[4-(2-tert-butoxycarbonyl-éthyl)-cyclohexyl]-benzoate d'éthyle.

### 13.3 Synthèse du trans-3-{4-[4-(5-benzyl-[1,3,4]thiadiazol-2-ylcarbamoyl)-phényl]-cyclohexyl}-propionate tert-butyle

Ce composé est obtenu suivant la préparation 11.5 avec un temps de réaction de 2 jours, 1.7 éq. de d'hexafluorophosphonate de bromo-tris-pyrrolidinophosphonium et 3 éq. de diisopropyléthylamine à partir de l'acide trans-4-[4-(2-tert-butoxycarbonylcyclopropyl)-cyclohexyl]-benzoïque.
M+H⁺ = 506.

### 13.4 Synthèse de l'acide 3-{4-[4-(5-benzyl-[1,3,4]thiadiazol-2-ylcarbamoyl)-phényl]-cyclohexyl}-propionique

Ce composé est obtenu selon la préparation 11.6 à partir du trans-3-{4-[4-(5-benzyl-[1,3,4]thiadiazol-2-ylcarbamoyl)-phényl]-cyclohexyl}-propionate tert-butyle.
M+H⁺ = 450.
RMN ¹H (400 MHz, DMSO-d6) δ ppm 12.83 (m, 1 H), 12 (m, 1 H), 8.02 (d, J = 8.4 Hz, 2H), 7.40 (d, J = 8.4 Hz, 2H), 7.38 (m, 4H), 7.30 (m, 1 H), 4.39 (s, 2H), 2.57 (m, 1 H), 2.26 (m, 1 H), 1.83 (m, 4H), 1.48 (m, 4H), 1.33 (m, 1 H), 1.07 (m, 2H).

### Exemple 14 : acide cis-4-{4-[5-(3-chloro-phényl)-[1,3,4]thiadiazol-2-ylcarbamoyl]-phénoxy}-cyclohexanecarboxylique (Composé N° 59 du tableau II)

### 14.1 Synthèse du trans-4-hydroxy-cyclohexanecarboxylate de méthyle

On place 1 g d'acide 4-hydroxy-cyclohexanecarboxylique (6,94 mmol, 1 éq.) dans 50 mL d'un mélange 4/1 de toluène et de méthanol. On coule goutte à goutte sous agitation 5,55 mL de triméthylsilyldiazométhane (11,10 mmol, 1,6 éq.) sur le milieu réactionnel. Après 16 heures, les solvants sont évaporés pour donner 1,3 g de trans-4-hydroxy-cyclohexanecarboxylate de méthyle.

### 14.2 Synthèse du cis-4-(4-méthoxycarbonyl-cyclohexyloxy)-benzoate de tert-butyle

On place 1,1 g de trans-4-hydroxy-cyclohexanecarboxylate de méthyle (6,95 mmol, 1,35 éq.), 1 g de 4-hydroxy-benzoate de tert-butyle (5,15 mmol, 1 éq.) et 2,03 g de triphénylphosphine (7,72 mmol, 1,5 éq.) dans 10 mL de tetrahydrofurane à température ambiante. 1,5 mL de diisopropyl azodicarboxylate (7,72 mmol, 1,5 éq.) sont ajoutés goutte à goutte sur le milieu réactionnel sous agitation. Après 18 heures à température ambiante, le milieu est concentré à sec et repris dans de l'éther diéthylique. La triphénylphosphine oxyde est filtrée. La phase organique est lavée avec une solution d'hydroxyde de sodium, séchée sur sulfate de sodium, filtrée et évaporée pour donner un résidu. Celui-ci est chromatographié sur gel de silice en éluant avec un gradient d'acétate d'éthyle dans l'heptane variant de 0% à 50 %. On obtient 1,23 g de cis-4-(4-méthoxycarbonyl-cyclohexyloxy)-benzoate de tert-butyle.

### 14.3 Synthèse de l'acide cis-4-(4-méthoxycarbonyl-cyclohexyloxy)-benzoïque

On place 0,6 g de cis-4-(4-méthoxycarbonyl-cyclohexyloxy)-benzoate de tert-butyle (1,79 mmol, 1 éq.) dans 2,5 mL de dichlorométhane. Le milieu réactionnel est mis à refroidir jusqu'à une température 4°C sous agitation dans un bain de glace. 1 mL d'acide trifluoroacétique (13,46 mmol, 7.5 éq.) est ajouté et le bain de glace est retiré. Après 5 heures d'agitation à température ambiante, le milieu est concentré, repris par de l'éther diéthylique, essoré et filtré pour donner 0,30 g d'acide cis-4-(4-méthoxycarbonyl-cyclohexyloxy)-benzoïque.
M+H⁺ = 279.

### 14.4 Synthèse du cis-4-{4-[5-(3-chloro-phényl)-[1,3,4]thiadiazol-2-ylcarbamoyl]-phénoxy}-cyclohexanecarboxylate de méthyle

On place 0,351 g d'acide cis-4-(4-méthoxycarbonyl-cyclohexyloxy)-benzoïque (1,26 mmol, 1 éq.) dans 5 mL de dichlorométhane à température ambiante. On ajoute successivement 0,42 mL de diisopropyléthylamine (2,52 mmol, 2 éq.), 0,705 g d'hexafluorophosphonate de bromo-tris-pyrrolidinophosphonium (1,51 mmol, 1.2 éq.) et 0,267 g de 3-chloro-phényl-1,3,4-thiadiazol-2-amine (1,26 mmol, 1 éq.). On ajoute 2 mL de DMF. Le mélange réactionnel est agité pendant 1 jour à température ambiante, dilué dans le dichlorométhane, et additionné d'eau. La filtration de ce milieu donne 0,06 g de cis-4-{4-[5-(3-chloro-phényl)-[1,3,4]thiadiazol-2-ylcarbamoyl]-phénoxy}-cyclohexanecarboxylate de méthyle. La phase organique est décantée, lavée à l'eau, séchée sur du sulfate de sodium, filtrée et évaporée. Le résidu est repris par de l'éthanol, essoré et lavé à l'ether diéthylique. On obtient 0,090 g de cis-4-{4-[5-(3-chloro-phényl)-[1,3,4]thiadiazol-2-ylcarbamoyl]-phénoxy}-cyclohexanecarboxylate de méthyle.

### 14.5 Synthèse de l'acide cis-4-{4-[5-(3-chloro-phényl)-[1,3,4]thiadiazol-2-ylcarbamoyl]-phénoxy}-cyclohexanecarboxylique

On place 150 mg de cis-4-{4-[5-(3-chloro-phényl)-[1,3,4]thiadiazol-2-ylcarbamoyl]-phénoxy}-cyclohexanecarboxylate de méthyle (0,32 mmol, 1 éq.) dans 1 mL de tetrahydrofurane. 27 mg d'hydroxyde de lithium monohydrate (0,64 mmol, 2 éq.) dissous dans 1 mL sont ajoutés et l'agitation est poursuivie pendant 18 heures. Le milieu réactionnel est dilué par de l'eau, lavé à l'acétate d'éthyle et acidifié par une solution aqueuse à 6% d'anhydride sulfureux. Le solide obtenu est essoré, lavé successivement à l'eau, à l'éthanol et à l'éther diéthylique. On obtient 96 mg de l'acide cis-4-{4-[5-(3-chloro-phényl)-[1,3,4]thiadiazol-2-ylcarbamoyl]-phénoxy}-cyclohexane-carboxylique.
M+H+ = 458.
RMN ¹H (400 MHz, DMSO-d6) δ ppm 13.04 (m, 1 H), 12.16 (m, 1 H), 8.15 (d, J = 9 Hz, 2H), 8.04 (m, 1 H), 7.94 (m, 1 H), 7.61 (m, 2H), 7.13 (d, J = 9 Hz, 2H), 4.72 (m, 1 H), 2.42 (m, 1 H), 1.92 to 1.64 (m, 8H).

### Exemple 15 : acide cis-4-{5-[5-(3-chloro-benzyl)-[1,3,4]thiadiazol-2-ylcarbamoyl]-pyridin-2-yloxy}-cyclohexanecarboxylique (Composé N° 63 du tableau II)

### 15.1 Synthèse du trans-4-hydroxy-cyclohexanecarboxylate de tert-butyle

On place 1,5 g de l'acide trans-4-hydroxy-cyclohexanecarboxylique (10,40 mmol, 1 éq.) dans 15 mL de toluène. Le milieu réactionnel est porté à reflux et 7,5 mL de la di-tert-butoxyméthyl-diméthyl-amine (31,32 mmol, 3 éq.) sont ajoutés goutte à goutte. Après 16 heures de reflux, le milieu réactionnel est dilué par de l'acétate d'éthyle, lavé successivement avec une solution aqueuse saturée d'hydrogénocarbonate de sodium et de la saumure. La phase organique est séchée sur du sulfate de magnésium, filtrée et évaporée pour donner 1.63 g du trans 4-hydroxy-cyclohexanecarboxylate de tert-butyle.

### 15.2 Synthèse du cis-6-(4-tert-butoxycarbonyl-cyclohexyloxy)-nicotinate d'éthyle

On place 1,63 g de trans-4-hydroxy-cyclohexanecarboxylate de tert-butyle (8,14 mmol, 1 éq.) et 1,62 g de 6-hydroxy-nicotinate d'éthyle (10,58 mmol, 1,3 éq.) dans 24 mL de tétrahydrofurane à température ambiante. 3,20 g de triphénylphosphine (12,21 mmol, 1,5 éq.) sont ajoutés suivi d'un goutte à goutte de 2,37 mL de diisopropyl azodicarboxylate (12,21 mmol, 1,5 éq.) sur le milieu réactionnel sous agitation. Après 1 heure à température ambiante, le milieu est concentré à sec et repris dans de l'éther diéthylique. La triphénylphosphine oxyde est filtrée. La phase organique est lavée successivement avec une solution d'hydroxyde de sodium puis avec de l'eau, séchée sur sulfate de magnésium, filtrée et évaporée pour donner un résidu. Celui-ci est chromatographié sur gel de silice en éluant avec un gradient d'acétate d'éthyle dans l'heptane variant de 3% à 30 %. On obtient 1,3 g de cis-6-(4-tert-butoxycarbonylcyclohexyloxy)-nicotinate d'éthyle.
M+H⁺ = 336.

### 15.3 Synthèse de l'acide cis-6-(4-tert-butoxycarbonyl-cyclohexyloxy)-nicotinique

On solubilise 1,33 g de cis-6-(4-tert-butoxycarbonyl-cyclohexyloxy)-nicotinate d'éthyle (3,97 mmol, 1 éq.) dans 60 mL d'un mélange 1/1 de THF/méthanol. A 0°C, 0,333 g d'hydroxyde de lithium monohydrate (7,93 mmol ; 2 éq.) est ajouté sous agitation. Après 16 heures d'agitation à température ambiante, le milieu réactionnel est évaporé et une solution aqueuse à 6% d'anhydride sulfureux est ajoutée. Le précipité formé est filtré et lavé à l'eau pour obtenir 1,25 g d'acide cis-6-(4-tert-butoxycarbonylcyclohexyloxy)-nicotinique.
M+H⁺ = 322.

### 15.4 Synthèse du cis-4-{5-[5-(3-chloro-benzyl)-[1,3,4]thiadiazol-2-ylcarbamoyl]-pyridin-2-yloxy}-cyclohexanecarboxylate de tert-butyle

On place 0,321 g d'acide cis-6-(4-tert-butoxycarbonyl-cyclohexyloxy)-nicotinique (1 mmol, 1 éq.) dans 50 mL d'acétonitrile à température ambiante. On ajoute successivement 0,33 mL de diisopropyléthylamine (2 mmol, 2 éq.), 0,466 g d'hexafluorophosphonate de bromo-tris-pyrrolidinophosphonium (1 mmol, 1 éq.) et 0,270 g de 5-(3-chloro-benzyl)-[1,3,4]thiadiazol-2-ylamine (1,2 mmol, 1,2 éq.) sous agitation. Après 4 jours à température ambiante, le milieu réactionnel est évaporé et 15 mL de DMF sont ajoutés. Le milieu est remis sous agitation pendant 18 heures, dilué dans l'acétate d'éthyle, lavé 3 fois à la saumure, séché sur du sulfate de magnésium, filtré et évaporé. Le résidu est chromatographié sur gel de silice en éluant avec un gradient d'acétate d'éthyle dans l'heptane variant de 5 à 50%. Après évaporation des fractions d'intérêt, le résidu est chromatographié sur gel de silice avec un gradient d'acétate d'éthyle dans le dichlorométhane variant de 2% à 20%. On obtient 0,262 g du cis-4-{5-[5-(3-chloro-benzyl)-[1,3,4]thiadiazol-2-ylcarbamoyl]-pyridin-2-yloxy}-cyclohexane carboxylate de tert-butyle.

### 15.5 Synthèse de l'acide cis-4-{5-[5-(3-chloro-benzyl)-[1,3,4]thiadiazol-2-ylcarbamoyl]-pyridin-2-yloxy}-cyclohexanecarboxylique

On place 0,264 g du cis-4-{5-[5-(3-chloro-benzyl)-[1,3,4]thiadiazol-2-ylcarbamoyl]-pyridin-2-yloxy}-cyclohexane carboxylate de tert-butyle (0,5 mmol, 1 éq.) dans 5 mL de dichlorométhane sous agitation. 0,37 mL d'acide trifluoroacétique (4,99 mmol, 10 éq.) est ajouté lentement. Après 18 heures d'agitation à température ambiante, le milieu est concentré, repris et évaporé successivement par de l'éthanol et du dichlorométhane. Le résidu est trituré par de l'éther diéthylique, essoré et filtré pour donner 0,213 g d'acide cis-4-{5-[5-(3-chloro-benzyl)-[1,3,4]thiadiazol-2-ylcarbamoyl]-pyridin-2-yloxy}-cyclohexane carboxylique.
M+H⁺ = 473.
RMN ¹H (400 MHz, DMSO-d6) δ ppm 12.57 (m, 2H), 8.88 (d, J = 2.4 Hz, 1 H), 8.32 (dd, J = 8.8 Hz and 2.4 Hz, 1 H), 7.47 (m, 1 H), 7.37 (m, 3H), 6.94 (d, J = 9 Hz, 1 H), 5.27 (m, 1 H), 4.42 (s, 2H), 2.41 (m, 1 H), 1.92 to 1.65 (m, 8H).

### Exemple 16 : acide trans-{4-[4-(5-benzyl-[1,3,4]thiadiazol-2-ylcarbamoyl)-phényl]-cyclohexyloxy}-acétique (Composé N° 46 du tableau V)

### 16.1 Synthèse du trans-4-(4-allyloxy-phényl)-cyclohexanol

10,6 g de trans-4-(4-hydroxy-cyclohexyl)-phénol (55,13 mmol, 14 éq.) sont dissous dans 150 mL d'un mélange 2/1 de DMF et d'acétone auquel on ajoute sous agitation 4.8 mL de bromure d'allyle (55,13 mmol, 14 éq.) suivi de 11,43 g (82,7, 1,5 éq.) de carbonate de potassium. Après 3 jours, le milieu réactionnel est versé sur 300 mL d'eau, filtré, lavé à l'eau et séché pour donner 12,6 g de trans-4-(4-allyloxy-phényl)-cyclohexanol.

### 16.2 Synthèse du trans-[4-(4-allyloxy-phényl)-cyclohexyloxy]-acétate de tert-butyle

2 g de trans-4-(4-allyloxy-phényl)-cyclohexanol (8,61 mmol, 1 éq.) sont dissous dans 50 mL d'un mélange 1/1 toluène/solution aqueuse à 32% d'hydroxyde de sodium auquel on ajoute sous agitation 3,81 mL (25,83 mmol, 3 éq.) de bromoacétate de tert-butyle suivi de 0,292 g de bromure de tétrabutylammonium (0,86 mmol, 0,1 éq.). Après 18 heures, le milieu réactionnel est dilué par de l'acétate d'éthyle et lavé à l'eau. La phase aqueuse est extraite par de l'acétate d'éthyle. Les phases organiques sont rassemblées, lavées par de la saumure séchées sur du sulfate de magnésium, filtrées et évaporées. Le résidu est chromatographié sur gel de silice en éluant avec un gradient d'acétate d'éthyle dans l'heptane variant de 3% à 30 %. On obtient 2,9 g de trans-4-(4-allyloxy-phényl)-cyclohexyloxy]-acétate de tert-butyle.

### 16.3 Synthèse du trans-[4-(4-hydroxy-phényl)-cyclohexyloxy]-acétate de tert-butyle

3,6 g de trans-[4-(4-allyloxy-phényl)-cyclohexyloxy]-acétate de tert-butyle sont dissous dans 70 mL de dichlorométhane auquel on ajoute sous agitation 4,87 g d'acide barbiturique (31,17 mmol, 3 éq.) puis 1,20 g de tétrakistriphénylphosphine de palladium (1,04 mmol, 0.1 éq.). Le milieu réactionnel est porté au reflux pendant 3 heures. Après 16 heures à température ambiante, le milieu réactionnel est dilué par du dichlorométhane et lavé par de la saumure. La phase aqueuse est extraite par du dichlorométhane. Les phases organiques sont rassemblées, lavées par de la saumure, séchées sur du sulfate de magnésium, filtrées et évaporées. Le résidu est chromatographié sur gel de silice en éluant avec un gradient d'acétate d'éthyle dans l'heptane variant de 2% à 20 %. On obtient 3,2 g de trans-[4-(4-hydroxy-phényl)-cyclohexyloxy]-acétate de tert-butyle.

### 16.4 Synthèse du trans-4-(4-trifluorométhanesulfonyloxy-phényl)-cyclohexyloxy]-acétate de tert-butyle

3,2 g de trans-[4-(4-hydroxy-phényl)-cyclohexyloxy]-acétate de tert-butyle (10,44 mmol. 1 éq.) sont dissous dans 50 mL de dichlorométhane et refroidi à l'aide d'un bain de glace. On ajoute 4,4 mL de triéthylamine (31,33 mmol. 3 éq.) suivi d'un goutte à goutte de 2,6 mL d'anhydride triflique (15,67 mmol, 1,5 éq.). Après 30 minutes d'agitation, le bain de glace est enlevé et l'agitation est poursuivie pendant 16 heures. Le milieu réactionnel est dilué par du dichlorométhane et lavé par une solution aqueuse saturée de chlorure d'ammonium. La phase aqueuse est extraite par du dichlorométhane. Les phases organiques sont rassemblées, lavées par une solution aqueuse saturée d'hydrogénocarbonate de sodium, séchées sur du sulfate de magnésium, filtrées et évaporées. Le résidu est chromatographié sur gel de silice en éluant avec un gradient d'acétate d'éthyle dans l'heptane variant de 4% à 40 %. On obtient 3,3 g de trans-[4-(4-trifluorométhanesulfonyloxy-phényl)-cyclohexyloxy]-acétate de tert-butyle.

### 16.5 Synthèse de l'acide trans-4-(4-tert-butoxycarbonylméthoxy-cyclohexyl)-benzoïque

On place 250 mg de trans-[4-(4-trifluorométhanesulfonyloxy-phényl)-cyclohexyloxy]-acétate de tert-butyle dans 1,5 mL de dioxane dans un tube micro-onde. On ajoute successivement 83 mg d'hexacarbonylmolybdène (0,32 mmol, 0,5 éq.), 14 mg de diacétate de palladium (0,06 mmol, 0,1 éq.), 35 mg de DPPF (0,06 mmol, 0,1 éq.), 154 mg de DMAP (1,26 mmol, 2 éq.), 0,25 mL de DIEA (1,45 mmol, 2,3 éq.) et 0,23 mL d'eau. Le tube est scellé et chauffé au M.O. à 120°C pendant 30 minutes. Le milieu réactionnel est dilué au dichlorométhane et lavé 3 fois à l'aide d'une solution aqueuse saturée de carbonate de sodium. Le milieu réactionnel est dilué à l'éther diéthylique et lavé avec une solution aqueuse d'HCl 5N. Les phases organiques sont rassemblées et évaporées pour donner 270 mg d'acide trans-4-(4-tert-butoxycarbonylméthoxycyclohexyl)-benzoïque qui est engagé tel quel dans l'étape suivante.
M+H⁺ = 293.

### 16.6 Synthèse du trans-{4-[4-(5-benzyl-[1,3,4]thiadiazol-2-ylcarbamoyl)-phényl]-cyclohexyloxy}-acétate de tert-butyle

On place 0,6 g d'acide trans-4-(4-tert-butoxycarbonylméthoxy-cyclohexyl)-benzoïque (1,79 mmol, 1 éq.) dans 8 mL de dichlorométhane sous agitation. On ajoute successivement, 0,7 mL de diisopropyléthylamine (4,49 mmol, 2,5 éq.), 0,275 g d'hydroxybenzotriazole (1,79 mmol, 1 éq.), 0,413 g du chlorhydrate de la 1-éthyl-3-(3-diméthylaminopropyl) carbodiimide (2,15 mmol, 1,2 éq.) et après 10 minutes, 0,377 g de 5-benzyl-1,3,4-thiadiazol-2-amine (1,97 mmol, 1.1 éq.). Après 16 heures, on ajoute 0,137 g d'hydroxybenzotriazole (0,895 mmol, 0,5 éq.) et 0,172 g de chlorhydrate de la 1-éthyl-3-(3-diméthylaminopropyl) carbodiimide (0,17 mmol, 0,5 éq.). Après 18 heures, le milieu est évaporé, dilué à l'acétate d'éthyle et lavé 3 fois avec de la saumure. La phase organique est séchée sur du sulfate de sodium, filtrée et évaporée. Le résidu est chromatographié sur gel de silice avec un gradient de méthanol dans le dichlorométhane variant de 0% à 2 %. On obtient 0,09 g de trans-{4-[4-(5-benzyl-[1,3,4]thiadiazol-2-ylcarbamoyl)-phényl]-cyclohexyloxy}-acétate de tert-butyle.
M+H+ = 508.

### 16.7 Synthèse de l'acide trans-{4-[4-(5-benzyl-[1,3,4]thiadiazol-2-ylcarbamoyl)-phényl]-cyclohexyloxy}-acétique

On place 90 mg de trans-{4-[4-(5-benzyl-[1,3,4]thiadiazol-2-ylcarbamoyl)-phényl]-cyclohexyloxy}-acétate de tert-butyle (0,18 mmol, 1 éq.) dans 1 mL de dichlorométhane. 0,13 mL d'acide trifluoroacétique (1,77 mmol, 10 éq.) sont ajoutés et le milieu réactionnel est agité 18 heures à température ambiante. Le solvant est évaporé. Le résidu est trituré dans l'acétate d'éthyle et le méthanol pour donner 15 mg d'acide trans-{4-[4-(5-benzyl-[1,3,4]thiadiazol-2-ylcarbamoyl)-phényl]-cyclohexyloxy}-acétique.
M+H⁺ = 452.
RMN ¹H (400 MHz, DMSO-d6) δ ppm 12.68 (m, 2H), 8.01 (d, J = 8.3 Hz, 2H), 7.41 (d, J = 8.3 Hz, 2H), 7.37 (m, 4H), 7.29 (m, 1 H), 4.39 (s, 2H), 4.06 (s, 2H), 3.40 (m, 1 H), 2.61 (tt, J = 12 Hz and 3.4 Hz, 1 H), 2.13 (m, 2H), 1.84 (m, 2H), 1.51 (m, 2H), 1.32 (m, 2H).

### Exemple 17 : acide trans-5-[4-(4-carboxymethyl-cyclohexyl)-benzoylamino]-[1,3,4]thiadiazole-2-carboxylique (Composé N° 50 du tableau 1)

### 17.1 Synthèse du trans-5-[4-(4-tert-butoxycarbonylméthyl-cyclohexyl)-benzoylamino]-[1,3,4]thiadiazole-2-carboxylate d'éthyle

On place 0,8 g d'acide trans-4-(4-tert-butoxycarbonylméthylcyclohexyl) benzoïque (2,51 mmol, 1 éq.) dans 12 mL de diméthylformamide à température ambiante dans un tube à micro-onde. On ajoute sous agitation successivement 0,769 g d'hydroxybenzotriazole (5,02 mmol, 2 éq.), 0,962 g de chlorhydrate de la 1-éthyl-3-(3-diméthylaminopropyl) carbodiimide (5,02 mmol, 2 éq.) et 0,522 g de 5-amino-[1,3,4]thiadiazole-2-carboxylate d'éthyle (3,01 mmol, 1,2 éq.). Le tube est scellé et le mélange réactionnel est chauffé 30 minutes à 100°C. Le milieu réactionnel est dilué par de l'acétate d'éthyle, lavé 3 fois à la saumure, séché sur du sulfate de sodium, filtré et évaporé. Le résidu est chromatographié sur gel de silice en éluant avec un gradient de méthanol dans le dichlorométhane variant de 0% à 2%. Les fractions d'intérêt sont évaporées et triturées dans l'acétate d'éthyle. Le solide obtenu est séché et on obtient 0,28 g du trans-5-[4-(4-tert-butoxycarbonylméthyl-cyclohexyl)-benzoylamino]-[1,3,4]thiadiazole-2-carboxylate d'éthyle.
M+H+ = 475.

### 17.2 Synthèse de l'acide trans-5-[4-(4-tert-butoxycarbonylméthyl-cyclohexyl)-benzoylamino]-[1,3,4]thiadiazole-2-carboxylique

Ce composé est obtenu selon la préparation 11.4 à partir du trans-5-[4-(4-tert-butoxycarbonyl méthyl-cyctohexyl)-benzoylamino]-[1,3,4]thiadiazole-2-carboxylate d'éthyle.

### 17.3 Synthèse de l'acide trans 5-[4-(4-carboxyméthyl-cyclohexyl)-benzoylamino]-[1,3,4]thiadiazole-2-carboxylique

Ce composé est obtenu selon la préparation 11.6 à partir de l'acide trans-5-[4-(4-tert-butoxy carbonylméthyl-cyclohexyl)-benzoylamino]-[1,3,4]thiadiazole-2-carboxylique. RMN ¹H (400 MHz, DMSO-d6) δppm 12.83 (m, 1 H), 12.11 (m, 1 H), 9.23 (s, 1 H), 9.07 (m, 2H), 7.46 (m, 2H), 2.76 to 2.45 (m, 1 H), 2.45 to 2.11 (m, 2H), 1.94 to 1.45 (m, 7H), 1.17 (m, 1H).

### Exemple 18: acide cis 4-[4-(5-cyclopentyloxyméthyl-[1,3,4]thiadiazol-2-ylcarbamoyl)-phénoxy]-cyclohexanecarboxylique (Composé N°67 du tableau II)

### 18.1 Synthèse de l'acide cyclopentyloxy-acétique hydrazide

On place 2,60 g d'acide cyclopentyloxy-acétique (18,03 mmol, 1,0 éq.) dans 50 mL de dichlorométhane à température ambiante sous agitation. On ajoute successivement sous agitation 2,86 g de l'hydrazinecarboxylate de tert-butyle (21,64 mmol, 1,2 éq.), 2,437 g d'hydroxybenzotriazole (18,03 mmol, 1,0 éq.), 4,148 g de chlorhydrate de la 1-éthyl-3-(3-diméthylaminopropyl) carbodiimide (21,64 mmol, 1,2 éq.) et 4,08 mL de diisopropyléthylamine (23,44 mmol, 1,3 éq.). Après 18 h, le milieu réactionnel est dilué avec du dichlorométhane et lavé 2 fois à l'eau. La phase organique est séchée sur du sulfate de sodium, filtrée et évaporée. Le résidu est mis en solution dans 60 mL de dichlorométhane. On ajoute 15 mL d'acide trifluoroacétique (201,93 mmoles, 11,20 éq.). Le milieu réactionnel est agité pendant 2 h et concentré sous vide. Le résidu est chromatographié sur gel de silice en éluant avec un gradient dichlorométhane / méthanol / ammoniaque variant de 99/1/0,1 à 95/5/0,5. On obtient 2,7 g d'acide cyclopentyloxy-acétique hydrazide.

### 18.2 Synthèse du cis 4-[4-(5-cyclopentyloxyméthyl-[1,3,4]thiadiazol-2-ylcarbamoyl)-phénoxy]-cyclohexanecarboxylate de méthyle

On place 0,4 g d'acide cis 4-(4-méthoxycarbonyl-cyclohexyloxy)-benzoïque dans 10 mL de dichlorométhane sous agitation, à température ambiante et sous atmosphère d'azote. On ajoute successivement 0,18 mL de chlorure d'oxalyle (2,16 mmol, 1,5 éq.) et 2 gouttes de diméthylformamide. Le milieu réactionnel est agité pendant 2 h et concentré sous vide. Le cis 4-(4-chlorocarbonyl-phénoxy)-cyclohexanecarboxylate de méthyle formé est dissous dans 15 mL d'acétonitrile et placé sous azote. On refroidit dans un bain de glace et on ajoute 0,210 g de thiocyanate de potassium (2,16 mmol, 1,5 éq.). Le mélange réactionnel est agité à température ambiante pendant 1 h30. On ajoute alors au cis 4-(4-isothiocyanatocarbonyl-phenoxy)-cyclohexanecarboxylate de méthyle obtenu 0,455 g d'acide cyclopentyloxy-acetique hydrazide (2,87 mmol, 2 éq.) en solution dans 5 mL d'acétonitrile et le mélange est chauffé au reflux pendant 2h30 puis laissé au repos et à température ambiante pendant 36 h. Le précipité formé est filtré et lavé avec de l'acétonitrile. On obtient 0,450 g de cis 4-[4-(5-cyclopentyloxyméthyl-[1,3,4]thiadiazol-2-ylcarbamoyl)-phénoxy]-cyclohexane-carboxylate de méthyle.
M+H⁺ = 460.

### 18.3 Synthèse de l'acide cis 4-[4-(5-cyclopentyloxyméthyl-[1,3,4]thiadiazol-2-ylcarbamoyl)-phénoxy]-cyclohexanecarboxylique

On place 0,446 g de cis 4-[4-(5-cyclopentyloxyméthyl-[1,3,4]thiadiazol-2-ylcarbamoylphénoxy]-cyclohexanecarboxylate de méthyle (0,97 mmol, 1 éq.) dans 10 mL de THF sous agitation, à température ambiante. On ajoute 0,111 g d'hydroxyde de lithium monohydrate (2,64 mmol, 2,7éq.) en solution dans 10 mL d'eau et le mélange est agité pendant 36 h et laissé au repos pendant 48h. On ajoute alors une solution aqueuse d'acide chlorhydique 1 N jusqu'à pH acide. Le précipité formé est filtré, lavé à l'eau et avec de l'éthanol. On obtient 0,320 g d'acide cis 4-[4-(5-cyclopentyloxyméthyl-[1,3,4]thiadiazol-2-ylcarbamoyl)-phénoxy]-cyclohexanecarboxylique.
M + H⁺ = 445.
RMN ¹H (400 MHz, DMSO-d6) δ ppm 12.48 (m, 2H), 8.09 (d, J = 9 Hz, 2H), 7.09 (d, J = 9 Hz, 2H), 4.79 (s, 2H), 4.69 (m, 1 H), 4.08 (m, 1 H), 2.40 (m, 1 H), 1.89 to 1.46 (m, 16H).

### Exemple 19: acide trans {4-[4-(5-cyclopentyloxyméthyl-[1,3,4]thiadiazol-2-ylcarbamoyl)-phényl]-cyclohexyl}-acétique (Composé N° 37 du tableau I)

On place 0,330 g d'acide trans 4-(4-tert-butoxycarbonylméthyl-cyclohexyl)-benzoïque dans 10 mL de dichlorométhane sous agitation, à température ambiante et sous atmosphère d'azote. On ajoute successivement 0,12 mL de chlorure d'oxalyle (1,42 mmol, 1,4 éq.) et 2 gouttes de diméthylformamide. Le milieu réactionnel est agité pendant 2 h et concentré sous vide. Le trans 4-(4-chlorocarbonylphényl)-cyclohexaneacétate de tertiobutyle formé est dissous dans 8 mL d'acétonitrile et placé sous azote. On refroidit dans un bain de glace et on ajoute 0,17 g de thiocyanate de potassium (1,75 mmol, 1,7 éq.). Le mélange réactionnel est agité à température ambiante pendant 1h30. On ajoute alors 0,380 g d'acide cyclopentyloxy-acetique hydrazide (2,40 mmol, 2,32 éq.) en solution dans 5 mL d'acétonitrile et le mélange est chauffé au reflux pendant 2h30 puis laissé au repos et à température ambiante pendant 36 h. Le précipité formé est filtré et lavé avec de l'acétonitrile. Le filtrat est dilué par du dichlorométhane et lavé avec de l'eau. La phase organique est séchée sur sulfate de sodium et concentré sous vide. Le résidu est dissous dans 5 mL de dichlorométhane ; on ajoute 3 mL d'acide trifluoroacétique et le milieu réactionnel est agité pendant 1 h30 et concentré sous vide. Après trituration dans le méthanol, l'éthanol et l'acétate d'éthyle, le résidu est chromatographié sur gel de silice en éluant avec un gradient de méthanol dans le dichlorométhane variant de 3 à 5%. Après trituration dans l'éthanol, on obtient 0,070 g d'acide trans {4-[4-(5-cyclopentyloxyméthyl-[1,3,4]thiadiazol-2-ylcarbamoyl)-phényl]-cyclohexyl}-acétique.
Cyclohexanecarboxylique.
M + H⁺ = 444.
1H NMR (400 MHz, DMSO-d6) d ppm 12.90 to 12.00 (m, 1H), 8.06 (m, 2H), 7.43 (m, 2H), 4.80 (s, 2H), 4.09 (m, 1 H), 2.59 (m, 1 H), 2.17 (d, J = 6.1 Hz, 2H), 1.92 to 1.45 (m, 15H), 1.15 (m, 2H).

### Exemple 20: hydrazide de l'acide (1S,3S/1R,3R)-3-phénoxycyclohexanecarboxylique (intermédiaire pour la synthèse du composé 44 du tableau I)

### 20.1 Synthèse du (1S,3S/1R,3R)-3-hydroxy-cyclohexanecarboxylate d'éthyle

On place 3 g du 3-oxo-cyclohexanecarboxylate d'éthyle (17,63 mmol, 1 éq.) dans 30 mL de méthanol. Le milieu réactionnel est refroidit à l'aide d'un bain de glace sous agitation et 0,733 g de borohydrure de sodium (19,39 mmol, 1.1 éq.) est ajouté par portions. Après 4 h, le milieu réactionnel est versé sur de l'eau et extrait 3 fois au dichlorométhane. Les phases organiques sont rassemblées, séchées sur du sulfate de magnésium, filtrées et évaporées pour donner 2,61 g de (1S,3S/1R,3R)-3-hydroxycyclohexanecarboxylate d'éthyle.

### 20.2 Synthèse du (1S,3S/1R,3R)-3-phénoxy-cyclohexanecarboxylate d'éthyle

On place 1,88 g de (1 S,3S/1R,3R)-3-hydroxy-cyclohexanecarboxylate d'éthyle dans 33 mL de THF. On ajoute successivement sous agitation 1,54 g de phénol (16,35 mmol, 1,5 éq.) et 4,29 g de triphénylphosphine (16,35 mmol, 1,5 éq.). Le milieu réactionnel est refroidit au bain de glace et 3,31 g d'azodicarboxylate de diéthyle (16,35 mmol, 1,5 éq.) sont ajoutés goutte à goutte. Après 18 h d'agitation, le milieu réactionnel est versé sur de l'eau et extrait 2 fois avec de l'acétate d'éthyle. Les phases organiques sont rassemblées, séchées sur du sulfate de magnésium, filtrées et évaporées. Le résidu est chromatographié sur gel de silice en éluant avec un gradient de dichlorométhane dans l'heptane variant de 40 à 100%. On obtient 0,369 g de (1S,3S/1R,3R)-3-phénoxycyclohexanecarboxylate d'éthyle.

### 20.3 Synthèse de l'acide (1S,3S/1R,3R)-3-phénoxy-cyclohexanecarboxylique

On place 0,369 g de (1S,3S/1R,3R)-3-phénoxy-cyclohexanecarboxylate d'éthyle (1,49 mmol, 1 éq.) dans 15 mL d'un mélange 1/1/1 de THF/méthanol/eau sous agitation. On ajoute 0,249 g d'hydroxyde de lithium monohydrate (5,94 mmol, 4éq.). Après 16 h d'agitation, le milieu réactionnel est évaporé, repris par de l'eau et solution aqueuse à 6% d'anhydride sulfureux est ajoutée. La phase aqueuse est extraite 2 fois par du dichlorométhane. Les phases organiques sont rassemblées, séchées sur sulfate de magnésium, filtrées et évaporées. On obtient 0,274 g d'acide (1S,3S/1R,3R)-3-phénoxy-cyclohexanecarboxylique

### 20.4 Synthèse du N'-((1S,3S/1R,3R)-3-phénoxy-cyclohexanecarbonyl)-hydrazinecarboxylate de tert-butyle

On place 0,274 g d'acide (1S,3S/1R,3R)-3-phénoxy-cyclohexanecarboxylique (1,24 mmol, 1,0 éq.) dans 3,5 mL de dichlorométhane à température ambiante sous agitation. On ajoute successivement sous agitation 0,197 g de l'hydrazinecarboxylate de tert-butyle (1,49 mmol, 1,2 éq.), 0,168 g d'hydroxybenzotriazole (1,24 mmol, 1,0 éq.), 0,286 g de chlorhydrate de la 1-éthyl-3-(3-diméthylaminopropyl) carbodiimide (1,49 mmol, 1,2 éq.) et 0,27 mL de diisopropyléthylamine (1,62 mmol, 1,3 éq.). Après 18 h, le milieu réactionnel est dilué avec du dichlorométhane et lavé successivement à l'eau, avec une solution aqueuse saturée d'hydrogénocarbonate de sodium et à l'eau. La phase organique est séchée sur du sulfate de magnésium, filtrée et évaporée. On obtient 0,45 g de N'-((1S,3S/1R,3R)-3-phénoxy-cyclohexanecarbonyl)-hydrazinecarboxylate de tert-butyle.

### 20.5 Synthèse de l'hydrazide de l'acide (1S,3S/1R,3R)-3-phénoxycyclohexanecarboxylique

On place 0,45 g de N'-((1S,3S/1R,3R)-3-phénoxy-cyclohexanecarbonyl)-hydrazine carboxylate de tert-butyle dans 2 mL de dichlorométhane et on ajoute 1,5 mL d'acide trifluoroacétique. Après 2h30 d'agitation, le milieu réactionnel est évaporé à sec pour donner 0,35 g de l'hydrazide de l'acide (1S,3S/1R,3R)-3-phénoxycyclohexanecarboxylique.
M + H⁺ = 235.

### Exemple 21 : acide (trans-4-{4-[(5-{[(3R,6S/3S,6R)-5-éthoxyoctahydropentalèn-2-yl]méthyl}-1,3,4-thiadiazol-2-yl)carbamoyl]phényl}cyclohexyl)acétique (Composé N° 45 du tableau I)

### 21.1 Synthèse de la (3R,6S/3S,6R)-5,5-diméthylhydro-1'H-spiro[1,3-dioxane-2,2'-pentalèn]-5'(3'H)-one

On porte à reflux 5 g de hydro-pentalène-2,5-dione (36,19 mmol, 1 éq.), 3,77 g de 2,2-diméthyl-1,3-propanediol (36,19 mmol, 1 éq.) et 0,069 g d'acide para-toluènesulfonique (0,36 mmol, 0,01 éq.) dans 50 mL de toluène pendant 18 h. Le milieu est dilué dans de l'acétate d'éthyle et lavé successivement par une solution aqueuse 1 N d'hydroxyde de sodium, de l'eau et de la saumure. La phase organique est séchée sur sulfate de sodium, filtrée et évaporée. Le résidu est chromatographié sur gel de silice en éluant avec un gradient d'acétate d'éthyle dans l'heptane variant de 30% à 33%. On obtient 3,7 g de (3R,6S/3S,6R)-5,5-diméthylhydro-1'H-spiro[1,3-dioxane-2,2'-pentalèn]-5'(3'H)-one.

### 21.2 Synthèse du (2E)-[(3R,6S/3S,6R)-5,5-diméthylhydro-1'H-spiro[1,3-dioxane-2,2'-pentalèn]-5'(3'H)-ylidène]éthanoate d'éthyle

On place 2,4 g de triéthylphosphonoacétate (10,7 mmol, 1,2 éq.) dans 30 mL de THF. Cette solution sous agitation est refroidit à l'aide d'un bain de glace et 0,428 g d'hydrure de sodium (10,7 mmol, 1,2 éq.) à 60% en dispersion dans de l'huile minérale est ajouté. Après 30 minutes, 2 g de (3R,6S/3S,6R)-5,5-diméthylhydro-1'H-spiro[1,3-dioxane-2,2'-pentalèn]-5'(3'H)-one (8,92 mmol, 1 éq.) en solution dans 20 mL de THF sont ajoutés. Le bain de glace est enlevé et l'agitation est poursuivie pendant 16 h. Le milieu réactionnel est neutralisé par une solution aqueuse de chlorure d'ammonium et extrait 3 fois avec du dichlorométhane. Les phases organiques sont rassemblées et lavées 2 fois à l'eau. La phase organique est séchée sur sulfate de sodium, filtrée et évaporée. Le résidu est chromatographié sur gel de silice en éluant avec un mélange d'acétate d'éthyle et d'heptane dans un rapport 1/2. On obtient 1,7 g de (2^{E})-[(3R,6S/3S,6R)-5,5-diméthylhydro-1'H-spiro[1,3-dioxane-2,2'-pentalèn]-5'(3'H)-ylidène]éthanoate d'éthyle.

### 21.3 Synthèse du [(3R,6S/3S,6R)-5,5-diméthylhexahydro-1'H-spiro[1,3-dioxane-2,2'-pentalèn]-5'-yl]acétate d'éthyle

On place dans une bouteille de Parr, on place 2,1 g de (2^{E})-[(3R,6S/3S,6R)-5,5-diméthylhydro-1'H-spiro[1,3-dioxane-2,2'-pentalèn]-5'(3'H)-ylidène]éthanoate d'éthyle (7,13 mmol, 1 éq.) dans 40 mL d'éthanol. On ajoute 0,152 g de palladium 10% sur charbon (0,14 mmol, 0,02 éq.) et on soumet la bouteille de Parr à 50 psi d'hydrogène pendant 5 h. Après filtration sur papier Whatman et évaporation de l'éthanol, on obtient 2 g du [(3R,6S/3S,6R)-5,5-diméthylhexahydro-1'H-spiro[1,3-dioxane-2,2'-pentalèn]-5'-yl]acétate d'éthyle.

### 21.4 Synthèse de l'acide [(3R,6S/3S,6R)-5,5-diméthylhexahydro-1'H-spiro[1,3-dioxane-2,2'-pentalèn]-5'-yl]acétique

Ce composé est obtenu suivant la préparation 20.3. On obtient 0,637 g d'acide [(3R,6S et 3S,6R)-5,5-diméthylhexahydro-1'H-spiro[1,3-dioxane-2,2'-pentalèn]-5'-yl]acétique à partir du [(3R,6S/3S,6R)-5,5-diméthylhexahydro-1'H-spiro[1,3-dioxane-2,2'-pentalèn]-5'-yl]acétate d'éthyle.

### 21.5 Synthèse du 2-{[(3R,6S/3S,6R)-5,5-diméthylhexahydro-1'H-spiro[1,3-dioxane-2,2'-pentalen]-5'-yl]acétyl}hydrazinecarboxylate de tert-butyle

On place 0,637 g d'acide [(3R,6S)-5,5-diméthylhexahydro-1'H-spiro[1,3-dioxane-2,2'-pentalèn]-5'-yl]acétique (2,37 mmol, 1,0 éq.) dans 15 mL de dichlorométhane à température ambiante sous agitation. On ajoute successivement sous agitation 0,377 g de l'hydrazinecarboxylate de tert-butyle (2,85 mmol, 1,2 éq.), 0,321 g d'hydroxybenzotriazole (2,37 mmol, 1,0 éq.), 0,546 g de chlorhydrate de la 1-éthyl-3-(3-diméthylaminopropyl) carbodiimide (2,85 mmol, 1,2 éq.) et 0,51 mL de diisopropyléthylamine (3,09 mmol, 1,3 éq.). Après 18 h, le milieu réactionnel est dilué avec du dichlorométhane et lavé 2 fois à l'eau. La phase organique est séchée sur du sulfate de sodium, filtrée et évaporée. On obtient 0,910 g du 2-{[(3R,6S/3S,6R)-5,5-diméthylhexahydro-1'H-spiro[1,3-dioxane-2,2'-pentalèn]-5'-yl]acétyl} hydrazinecarboxylate de tert-butyle.

### 21.6 Synthèse de la 2-[(3R,6S/3S,6R)-5,5-diméthylhexahydro-1'H-spiro[1,3-dioxane-2,2'-pentalèn]-5'-yl]acétohydrazide

Ce composé est obtenu suivant la préparation 20.5. On obtient 0,77 g de la 2-[(3R,6S/3S,6R)-5,5-diméthylhexahydro-1'H-spiro[1,3-dioxane-2,2'-pentalèn]-5'-yl]acétohydrazide à partir du 2-{[(3R,6S/3S,6R)-5,5-diméthylhexahydro-1'H-spiro[1,3-dioxane-2,2'-pentalèn]-5'-yl]acétyl} hydrazinecarboxylate de tert-butyle.

### 21.7 Synthèse du (trans-4-{4-[(5-{[(3R,6S/3S,6R)-5-éthoxyoctahydropentalèn-2-yl]méthyl}-1,3,4-thiadiazol-2-yl)carbamoyl]phényl}clohexyl)acétate de tert-butyle

On place 0,400 g d'acide trans 4-(4-tert-butoxycarbonylméthyl-cyclohexyl)-benzoïque (1,26 mmol, 1 éq.) dans 8 mL de dichlorométhane sous agitation, à température ambiante et sous atmosphère d'azote. On ajoute successivement 0,21 mL de chlorure d'oxalyle (2,51 mmol, 2 éq.) et 2 gouttes de diméthylformamide. Le milieu réactionnel est agité pendant 2 h et concentré sous vide. Le trans 4-(4-chlorocarbonylphényl)-cyclohexaneacétate de tertiobutyle formé est dissous dans 15 mL d'acétone et placé sous azote. On refroidit dans un bain de glace et on ajoute 0,147 g de thiocyanate de potassium (1,51 mmol, 1,2 éq.). Le mélange réactionnel est agité à température ambiante pendant 2 h. On ajoute alors 0,669 g de 2-[(3R,6S/3S,6R)-5,5-diméthylhexahydro-1'H-spiro[1,3-dioxane-2,2'-pentalèn]-5'-yl]acéto-hydrazide (2,37 mmol, 1,89 éq.) en solution dans 10 mL d'acétone et le mélange est chauffé au reflux pendant 16 h. Le milieu réactionnel est dilué par du dichlorométhane et lavé 3 fois avec de l'eau. La phase organique est séchée sur sulfate de sodium et concentrée sous vide. Le résidu est chromatographié sur gel de silice en éluant avec un mélange d'acétate d'éthyle et d'heptane dans un rapport 1/2. Après trituration dans l'éthanol, on obtient 0,286 g de (trans-4-{4-[(5-{[(3R,6S/3S,6R)-5-éthoxyoctahydropentalèn-2-yl]méthyl}-1,3,4-thiadiazol-2-yl)carbamoyl]phényl}cyclohexyl)acétate de tert-butyle.

### 21.8 Synthèse de l'acide (trans-4-{4-[(5-{[(3R,6S/3S,6R)-5-éthoxyoctahydropentalèn-2-yl]méthyl}-1,3,4-thiadiazol-2-yl)carbamoyl]phényl}cyclohexyl)acétique

On place 0,286 g de (trans-4-{4-[(5-{[(3R,6S/3S,6R)-5-éthoxyoctahydropentalèn-2-yl]méthyl}-1,3,4-thiadiazol-2-yl)carbamoyl]phényl}cyclohexyl)acétate de tert-butyle (0,46 mmol, 1 éq.) dans 4 mL de dichlorométhane et on ajoute 1 mL d'acide trifluoroacétique (5 mmol, 10.9 éq.). Après 16 h d'agitation, le milieu réactionnel est évaporé à sec et une solution aqueuse saturée d'hydrogénocarbonate de sodium est ajoutée. La phase aqueuse est extraite 3 fois avec du dichlorométhane. Le résidu est chromatographié sur gel de silice en éluant avec un gradient dichlorométhane / méthanol / acide acétique variant de 97/3/0,3 à 95/5/0,5. On obtient après trituration dans l'eau, l'éthanol et le méthanol 0,024 g d'acide (trans-4-{4-[(5-{[(3R,6S/3S,6R)-5-éthoxyoctahydropentalèn-2-yl]méthyl}-1,3,4-thiadiazol-2-yl)carbamoyl]phényl} cyclohexyl)acétique.
M+H⁺ = 511.
RMN ¹H (400 MHz, DMSO-d6) δ ppm 12.9 to 11.7 (m, 2H), 8.03 (m, 2H), 7.42 (m, 2H), 3.83 (m, 1 H), 3.35 (m, 2H), 3.02 (m, 2H), 2.64 to 1.94 (m, 8H), 1.93 to 1.00 (m, 18H).

### Exemple 22 : acide trans 4-[4-(5-benzyl-[1,3,4]thiadiazol-2-ylcarbamoyl)-phényl]-cyclohexanecarboxylique (Composé N° 70 du tableau III)

### 22.1 Synthèse du 4-(4-trifluorométhanesulfonyloxy-cyclohex-3-ényl)-benzoate d'éthyle

A -70°C, on place 3,3 mL de diisopropyléthylamine (23,55 mmol, 1,16 éq.) dans 50 mL de THF et on ajoute sous agitation 9,3 mL de n-BuLi (1,14 éq.) en solution 2,5 M dans le THF. Après 15 minutes, 5 g de 4-(4-oxo-cyclohexyl)-benzoate d'éthyle (20,30 mmol, 1 éq.) sont ajoutés goutte à goutte. Après 1 h30 d'agitation, 8,27 g de N-phényltrifluorométhanesulfonimide en solution dans 50 mL de THF sont ajoutés goutte à goutte. Le bain froid est retiré et l'agitation est maintenue pendant 4 h. Le milieu réactionnel est dilué par de l'eau et extrait à l'éther diéthylique. La phase organique est lavée 2 fois à l'eau et 1 fois à la saumure. Les phases organiques sont rassemblées et évaporées. Le résidu est chromatographié sur gel de silice en éluant avec un gradient d'acétate d'éthyle dans l'heptane variant de 15% à 20 %. On obtient 2,8 g de 4-(4-trifluorométhanesulfonyloxy-cyclohex-3-ényl)-benzoate d'éthyle.

### 22.2 Synthèse du 4-(4-tert-butoxycarbonyl-cyclohex-3-ényl)-benzoate d'éthyle

On place 1 g de 4-(4-trifluorométhanesulfonyloxy-cyclohex-3-ényl)-benzoate d'éthyle dans 10 mL de dioxane que l'on dispose dans 2 tubes micro-onde. On ajoute successivement 349 mg d'hexacarbonylmolybdène (1,32 mmol, 0,5 éq.), 59 mg de diacétate de palladium (0,26 mmol, 0,1 éq.), 147 mg de DPPF (0,26 mmol, 0,1 éq.), 646 mg de DMAP (5,29 mmol, 2 éq.), 1,06 mL de DIEA (6,08 mmol, 2,3 éq.) et 2,53 mL de tert-butanol (26,43 mmol, 10 éq.) que l'on dispose dans 2 tubes. Les tubes sont scellés et chauffés au M.O. à 120°C pendant 10 minutes. Le milieu réactionnel est dilué par du dichlorométhane et lavé avec une solution aqueuse d'HCl 1N. Les phases organiques sont séchées sur sulfate de sodium, filtrées et évaporées. Le résidu est chromatographié sur gel de silice en éluant avec un gradient d'acétate d'éthyle dans l'heptane variant de 15% à 20 %. On obtient 0,66 g de 4-(4-tert-butoxycarbonylcyclohex-3-ényl)-benzoate d'éthyle.

### 22.3 Synthèse du 4-(4-tert-butoxycarbonyl-cyclohexyl)-benzoate d'éthyle

Ce composé est obtenu suivant la préparation 13.1 avec une température de chauffage de 40°C. 0,4 g de 4-(4-tert-butoxycarbonyl-cyclohexyl)-benzoate d'éthyle est isolé à partir de 4-(4-tert-butoxycarbonyl-cyclohex-3-ényl)-benzoate d'éthyle.

### 22.4 Synthèse de l'acide 4-(4-tert-butoxycarbonyl-cyclohexyl)-benzoïque

On place 400 mg de 4-(4-tert-butoxycarbonyl-cyclohexyl)-benzoate d'éthyle (1.2 mmol, 1 éq.) dans 6 mL d'un mélange 2/1 de tetrahydrofurane et de méthanol. Le milieu réactionnel est refroidit à l'aide d'un bain de glace et 202 mg d'hydroxyde de lithium monohydrate (4,81 mmol, 4 éq.) dissous dans 2 mL sont ajoutés et l'agitation est poursuivie pendant 18 heures. Le milieu réactionnel est évaporé et acidifié par une solution aqueuse à 6% d'anhydride sulfureux. Après 1 h d'agitation, le solide obtenu est essoré, lavé successivement à l'eau, à l'éthanol et à l'acétate d'éthyle. Le résidu est chromatographié sur gel de silice en éluant avec un gradient de méthanol dans le dichlorométhane variant de 3% à 5%. On obtient 125 mg d'acide 4-(4-tert-butoxycarbonyl-cyclohexyl)-benzoïque.

### 22.5 Synthèse du 4-[4-(5-benzyl-[1,3,4]thiadiazol-2-ylcarbamoyl)-phényl]-cyclohexanecarboxylate de tert-butyle

Ce composé est obtenu suivant la préparation 11.5. 85 mg de 4-[4-(5-benzyl-[1,3,4]thiadiazol-2-ylcarbamoyl)-phényl]-cyclohexanecarboxylate de tert-butyle sont obtenus à partir d'acide 4-(4-tert-butoxycarbonyl-cyclohexyl)-benzoïque et de 5-benzyl-1,3,4-thiadiazol-2-amine.
M + H⁺ = 478.

### 22.6 Synthèse de l'acide 4-[4-(5-benzyl-[1,3,4]thiadiazol-2-ylcarbamoyl)-phényl]-cyclohexanecarboxylique

On place 85 mg de 4-[4-(5-benzyl-[1,3,4]thiadiazol-2-ylcarbamoyl)-phényl]-cyclohexanecarboxylate de tert-butyle (0,18 mmol, 1 éq.) dans 1 mL de dichlorométhane. 1 mL d'acide trifluoroacétique (13,46 mmol, 76 éq.) est ajouté et le milieu réactionnel est agité 3 h à température ambiante. Les solvants sont évaporés et le résidu est repris par de l'acétate d'éthyle et de l'éthanol pour donner après filtration 30 mg d'acide 4-[4-(5-benzyl-[1,3,4]thiadiazol-2-ylcarbamoyl)-phényl]-cyclohexane-carboxylique.
M + H⁺ = 422.
RMN ¹H (400 MHz, DMSO-d6) δ ppm 13.01 to 11.86 (m, 2H), 8.02 (m, 2H), 7.46 to 7.25 (m, 7H), 4.39 (s, 2H), 2.72 to 2.56 (m, 2H), 2.39 to 1.95 (m, 3H), 1.91 to 1.40 (m, 5H).

### Exemple 23 : acide (4-{4-[5-(3-hydroxy-3-phényl-propyl)-[1,3,4]thiadiazol-2-ylcarbamoyl]-phényl}-cyclohexyl)-acétique (Composé N° 84 du tableau V)

### 23.1 Synthèse du trans-(4-{4-[5-(3-hydroxy-3-phényl-propyl)-[1,3,4]thiadiazol-2-ylcarbamoyl]-phényl}-cyclohexyl)-acétate de tert-butyle

On place 0,159 g de trans-(4-{4-[5-(3-oxo-3-phényl-propyl)-[1,3,4]thiadiazol-2-ylcarbamoyl]-phényl}-cyclohexyl)-acétate de tert-butyle (0,3mmol, 1 éq.) dans 5 mL de méthanol sous agitation. Le milieu réactionnel est refroidi au bain de glace et 0,012 g de borohydrure de sodium (0,33 mmol, 1,1 éq.) est ajouté. 3 mL de DMF sont ajoutés. Après 16 h d'agitation, le milieu réactionnel est refroidi au bain de glace et 0,023 g de borohydrure de sodium (0.6 mmol, 2 éq.) et 3 mL de DMF sont ajoutés. Après 2 h d'agitation, le milieu réactionnel est versé sur de l'eau et extrait 2 fois à l'aide d'acétate d'éthyle. La phase organique est lavée 3 fois à l'eau, séchée sur sulfate de magnésium et évaporée pour donner 0,146 g de trans-(4-{4-[5-(3-hydroxy-3-phényl-propyl)-[1,3,4]thiadiazol-2-ylcarbamoyl]-phényl}-cyclohexyl)-acétate de tert-butyle.
M+H⁺ = 536.

### 23.2 Synthèse de l'acide trans-(4-{4-[5-(3-hydroxy-3-phényl)-propyl)-[1,3,4]thiadiazol-2-ylcarbamoyl]-phényl}-cyclohexyl)-acétique

On place 0,146 g de trans-(4-{4-[5-(3-hydroxy-3-phényl-propyl)-[1,3,4]thiadiazol-2-ylcarbamoyl]-phényl}-cyclohexyl)-acétate de tert-butyle (0,27 mmol, 1 éq.) dans 2 mL de dichlorométhane sous agitation. 0,4 mL de TFA (5,45 mmol, 20 éq.) est ajouté. Après 4 h d'agitation, le milieu réactionnel est évaporé et le résidu est chromatographié sur gel de silice en éluant avec un gradient de méthanol dans le dichlorométhane variant de 1% à 10 %. Les fractions d'intérêt sont concentrées, triturées à l'acétate d'éthyle et au méthanol. On obtient 0,020 g de l'acide trans-(4-{4-[5-(3-hydroxy-3-phényl-propyl)-[1,3,4]thiadiazol-2-ylcarbamoyl]-phényl}-cyclohexyl)-acétique.
M+H⁺ = 480.
RMN ¹H (400 MHz, DMSO-d6) δ ppm 12.97 to 11.74 (m, 2H), 8.04 (m, 2H), 7.45 to 7.32 (m, 6H), 7.25 (m, 1 H), 5.39 (d, J = 4.5 Hz, 1 H), 4.66 (m, 1 H), 3.06 (m, 2H), 2.58 (tt, J = 12 Hz and 3 Hz, 1 H), 2.17 (d, J = 7 Hz, 2H), 2.06 (m, 2H), 1.90 to 1.70 (m, 5H), 1.52 (m, 2H), 1.15 (m, 2H).

### Exemple 24 : acide trans-{4-[4-(5-phénylméthanesulfinylméthyl-[1,3,4]thiadiazol-2-ylcarbamoyl)-phényl]-cyclohexyl}-acétique (Composé N° 95 du tableau V)

### 24.1 Synthèse du trans-{4-[4-(5-phénylméthanesulfinylméthyl-[1,3,4]thiadiazol-2-ylcarbamoyl)-phényl]-cyclohexyl}-acétate de tert-butyle

On place 0,1 g de trans-{4-[4-(5-benzylsulfanylméthyl-[1,3,4]thiadiazol-2-ylcarbamoyl)-phényl]-cyclohexyl}-acétate de tert-butyle (0,11 mmol, 1 éq.) dans 10 mL de dichlorométhane sous agitation. On ajoute 0,027 g d'acide méta-chloroperbenzoïque à 70% (0,11 mmol, 1 éq.). Après 2h, on ajoute 0,01 g d'acide méta-chloroperbenzoique à 70% (0,41 mmol, 0,37 éq.). Après 16h, le milieu réactionnel est dilué par du dichlorométhane et lavé avec une solution aqueuse saturée d'hydrogénocarbonate de sodium. La phase organique est séchée et évaporée. Le résidu est trituré dans l'acétate d'éthyle pour donner 0,072 g de trans-{4-[4-(5-phénylméthanesulfinylméthyl-[1,3,4]thiadiazol-2-ylcarbamoyl)-phényl]-cyclohexyl}-acétate de tert-butyle.
M+H⁺ = 554.

### 24.2 Synthèse de l'acide trans-{4-[4-(5-phénylméthanesulfinylméthyl-[1,3,4]thiadiazol-2-ylcarbamoyl)-phényl]-cyclohexyl}-acétique

On place 0,072 g de trans-{4-[4-(5-phénylméthanesulfinylméthyl-[1,3,4]thiadiazol-2-ylcarbamoyl)-phényl]-cyclohexyl}-acétate de tert-butyle (0,13 mmol, 1 éq.) dans 2 mL de dichlorométhane sous agitation. On ajoute 1 mL de TFA (13,46 mmol, 104 éq.). Après 1 h, le milieu réactionnel est évaporé et le résidu est trituré dans l'éthanol pour donner 0,055 g d'acide trans-{4-[4-(5-phénylméthanesulfinylméthyl-[1,3,4]thiadiazol-2-ylcarbamoyl)-phényl]-cyclohexyl}-acétique.
M+H⁺ = 498.
RMN ¹H (400 MHz, DMSO-d6) δ ppm 12.99 (m, 1 H), 12.02 (m, 1H), 8.07 (m, 2H), 7.49 to 7.33 (m, 7H), 4.70 (m, 1 H), 4.45 (m, 1 H), 4.29 (m, 1 H), 4.02 (m, 1 H), 2.59 (m, 1 H), 2.17 (d, J = 6.8 Hz, 2H), 1.90 to 1.71 (m, 5H), 1.53 (m, 2H), 1.15 (m, 2H).

### Exemple 25 : acide cis 4-[4-(5-cyclopentylamino-[1,3,4]thiadiazol-2-ylcarbamoyl)-phénoxy]-cyclohexanecarboxylique (Composé N° 101 du tableau V)

### 25.1 Synthèse du cis-4-[4-(5-cyclopentylamino-[1,3,4]thiadiazol-2-ylcarbamoyl)-phénoxy]-cyclohexanecarboxylate de méthyle

On place 0,350 g d'acide cis 4-(4-méthoxycarbonyl-cyclohexyloxy)-benzoïque (1,26 mmol, 1 éq.) dans 13 mL de dichlorométhane sous agitation, à température ambiante et sous atmosphère d'azote. On ajoute successivement 0,16 mL de chlorure d'oxalyle (1,89 mmol, 1,5 éq.) et 2 gouttes de diméthylformamide. Le milieu réactionnel est agité pendant 1,5 h et concentré sous vide. Le cis 4-(4-chlorocarbonyl-phénoxy)-cyclohexanecarboxylate de méthyle formé est dissous dans 13 mL d'acétonitrile et placé sous azote. On additionne successivement 0,255 g N-cyclopentyl-[1,3,4]thiadiazole-2,5-diamine (1,39 mmol, 1.1 éq.) et 0.12 mL de pyridine (1,51 mmol, 1,2 éq.). Après 3 jours, le milieu est filtré et rincé à l'acétonitrile pour donner 0,33 g du cis-4-[4-(5-cyclopentylamino-[1,3,4]thiadiazol-2-ylcarbamoyl)-phénoxy]-cyclohexanecarboxylate de méthyle.
M+H⁺ = 445.

### 25.2 Synthèse de l'acide cis-4-[4-(5-cyclopentylamino-[1,3,4]thiadiazol-2-ylcarbamoyl)-phénoxy]-cyclohexanecarboxylique

On place 0,310 g du cis-4-[4-(5-cyclopentylamino-[1,3,4]thiadiazol-2-ylcarbamoyl)-phénoxy]-cyclohexanecarboxylate de méthyle (0,70 mmol, 1 éq.) dans 8 mL d'un mélange tetrahydrofurane/méthanol. 112 mg d'hydroxyde de sodium (2,79 mmol, 4 éq.) dissous dans 6 mL d'eau sont ajoutés et l'agitation est poursuivie pendant 18 heures. Le milieu réactionnel est évaporé. Le résidu est repris par de l'eau et lavé 2 fois à l'éther diéthylique. La phase aqueuse est partiellement concentrée et acidifiée par une solution aqueuse à 6% d'anhydride sulfureux. La phase aqueuse est partiellement concentrée, essorée, lavée successivement à l'eau et à l'éther diéthylique. On obtient 0,19 mg d'acide cis-4-[4-(5-cyclopentylamino-[1,3,4]thiadiazol-2-ylcarbamoyl)-phénoxy]-cyclohexanecarboxylique.
M+H⁺ = 431.
RMN ¹H (400 MHz, DMSO-d6) δ ppm 12.15 (m, 2H), 8.04 (m, 2H), 7.31 (m, 1 H), 7.06 (m, 2H), 4.68 (m, 1 H), 3.98 (m, 1 H), 2.40 (m, 1 H), 2.00 to 1.45 (m, 16H).

### Exemple 26 : N-(5-benzyl-[1,3,4]thiadiazol-2-yl)-4-{4-[(2,3-dihydroxypropylcarbamoyl)-méthyl]-cyclohexyl}-benzamide (Composé N° 88 du tableau V)

On place 0,25 g d'acide trans-(4-{4-benzyl-[1,3,4]thiadiazol-2-ylcarbamoyl}phényl}-cyclohexyl)acétique (0,57 mmoles, 1 éq.) dans 4 mL de DMF à température ambiante sous agitation. On ajoute 0,294 g d'hexafluorophosphonate de bromo-tris-pyrrolidinophosphonium (0,63 mmoles, 1,1 éq.) et 0,20 mL de diisopropyléthylamine (1,15 mmoles, 2 éq.) et 0,09 mL de 3-amino-1,2-propanediol (1,15 mmol, 2 éq.). Le mélange réactionnel est agité pendant 18 h à température ambiante, dilué dans l'acétate d'éthyle et lavé à l'eau. La phase aqueuse est extraite à l'aide d'acétate d'éthyle. Les phases organiques sont concentrées et le résidu est chromatographié sur gel de silice en éluant avec un gradient de méthanol dans le dichlorométhane variant de 1% à 10%. Les fractions d'intérêt sont évaporées et le résidu est trituré à l'éthanol pour obtenir 0,171 g de N-(5-benzyl-[1,3,4]thiadiazol-2-yl)-4-{4-[(2,3-dihydroxypropylcarbamoyl)-méthyl]-cyclohexyl}-benzamide.
M+H⁺ = 509.
RMN ¹H (400 MHz, DMSO-d6) δ ppm 12.82 (m, 2H), 8.02 (m, 2H), 7.79 (t, J = 5.8 Hz, 1H), 7.41 (m, 2H), 7.37 (m, 4H), 7.30 (m, 1H), 4.70 (d, J = 4.8 Hz, 1H), 4.50 (t, J = 5.8 Hz, 1 H), 4.38 (s, 2H), 3.49 (m, 1 H), 3.30 (m, 1 H), 3.21 (m, 1 H), 3.01 (m, 1 H), 2.56 (m, 1 H), 2.05 (d, J = 6.6 Hz, 2H), 1.86 to 1.70 m, 5H), 1.49 (m, 2H), 1.11 (m, 2H).

### Exemple 27 : acide cis-4-{4-[5-(3,5-difluoro-benzyl)-[1,3,4]thiadiazol-2-ylcarbamoyl]-phénoxy}-cyclohexanecarboxylique (Composé N° 94 du tableau V)

### 27.1 Synthèse du [(3,5-difluorophényl)acétyl]hydrazinecarbothioamide

On place 2,5 g d'acide 3,5-difluorophénylacétique (14,52 mmoles, 1 éq.) dans 70 mL de dichlorométhane sous agitation. On ajoute successivement toujours sous agitation à température ambiante, 1,46 g de thiosemicarbazide (15,98 mmoles, 1,1 éq.), 2,22 g d'hydroxybenzotriazole (14,52 mmoles, 1 éq.) et 2,78 g de chlorhydrate de la 1-éthyl-3-(3-diméthylaminopropyl) carbodiimide (14,52 mmoles, 1 éq.). Après 18 h à température ambiante, le dichlorométhane est évaporé. Le résidu est repris par de l'acétate d'éthyle et de l'acide chlorhydrique 1 N. Le précipité est filtré et séché pour donner 1,67 g de [(3,5-difluorophényl)acétyl]hydrazinecarbothioamide.
M+H⁺ = 246.

### 27.2 Synthèse de la 5-(3,5-difluoro-benzyl)-[1,3,4]thiadiazol-2-ylamine

On place 20 mL d'acide sulfurique dans un ballon qui est mis à refroidir à 0°C. 1,67 g de la 5-(3,5-difluoro-benzyl)-[1,3,4]thiadiazol-2-ylamine (1,67 mmol) sont ajoutés par portions sous agitation. Après 3 h d'agitation, on ajoute de la glace puis on revient à pH basique avec de l'hydroxyde de sodium. Le précipité est filtré, lavé à l'eau et séché. On obtient 1,6 g de la 5-(3,5-difluoro-benzyl)-[1,3,4]thiadiazol-2-ylamine.
M+H⁺ = 228.

### 27.3 Synthèse du cis-4-{4-[5-(3,5-difluoro-benzyl)-[1,3,4]thiadiazol-2-ylcarbamoyl]-phénoxy}-cyclohexanecarboxylate de méthyle

On place 2,2 g d'acide cis-4-(4-méthoxycarbonyl-cyclohexyloxy)-benzoïque (7,91 mmol, 1 éq.) dans 30 mL de diméthylformamide à température ambiante. On ajoute successivement 1,98 g de la 5-(3,5-difluoro-benzyl)-[1,3,4]thiadiazol-2-ylamine (8,70 mmol, 1,1 éq.), 4,42 g d'hexafluorophosphonate de bromo-tris-pyrrolidinophosphonium (9,49 mmol, 1,2 éq.) et 2,76 mL de diisopropyléthylamine (15,81 mmol, 2 éq.). Le mélange réactionnel est agité pendant 6 jours à température ambiante, versé sur de l'eau et extrait à l'aide d'acétate d'éthyle. La phase organique est lavée 3 fois à l'eau et 1 fois à la saumure. La phase organique est concentrée et le résidu est chromatographié sur gel de silice en éluant avec un gradient d'acétate d'éthyle dans l'heptane variant de 7% à 30%. On obtient 1,5 g du cis 4-{4-[5-(3,5-difluoro-benzyl)-[1,3,4]thiadiazol-2-ylcarbamoyl]-phénoxy}-cyclohexane-carboxylate de méthyle.
M+H⁺ = 488.

### 27.4 Synthèse de l'acide cis-4-{4-[5-(3,5-difluoro-benzyl)-[1,3,4]thiadiazol-2-ylcarbamoyl]-phénoxy}-cyclohexanecarboxylique

On solubilise 1,5 g de 4-{4-[5-(3,5-difluoro-benzyl)-[1,3,4]thiadiazol-2-ylcarbamoyl]-phénoxy}-cyclohexane-carboxylate de méthyle (3,08 mmol, 1 éq.) dans 20 mL d'un mélange 1/1 de THF/méthanol. 0,258 g d'hydroxyde de lithium monohydrate (6,15 mmol ; 2 éq.) est ajouté sous agitation. Après 16 heures à température ambiante, le milieu réactionnel est évaporé, dilué par de l'eau et une solution aqueuse à 6% d'anhydride sulfureux est ajoutée. Le précipité est filtré et lavé à l'eau. Le résidu est ensuite successivement trituré et filtré dans l'acétate d'éthyle et l'éthanol. On obtient 1,14 g d'acide cis-4-{4-[5-(3,5-difluoro-benzyl)-[1,3,4]thiadiazol-2-ylcarbamoyl]-phénoxy}-cyclohexanecarboxylique.
M+H⁺ = 472.
RMN ¹H (400 MHz, DMSO-d₆) δ ppm 12.44 (m, 2H), 8.12 (m, 2H), 7.24 to 7.07 (m, 5H), 4.72 (m, 1 H), 4.45 (s, 2H), 2.45 (m, 1 H), 1.93 to 1.67 (m, 8H).

Les **tableaux I à V** qui suivent illustrent les structures chimiques et les propriétés physiques de quelques composés selon l'invention, répondant à la formule (I).
Les composés No. 14, 15, 16, 19, 25, 26, 27, 28, 29, 30, 31, 35, 36, 47, 49, 50, 54, 58, 59, 60, 61, 65, 66, 85, 92 et 101 sont exemples de comparaison et indiquée avec <*>.
Le **tableau I** illustre des composés de formule (I) selon l'invention pour lesquels **W est** un atome de carbone, **D** est une liaison, **p** = 0, **R** est un atome d'hydrogène, **U** est un atome d'oxygène donc R5 est absent, **Z4** est un atome d'hydrogène et **Z2** est absent; ces composés sont appelés ci-après composés de formule (A).

Le **tableau II** illustre des composés de formule (I) selon l'invention pour lesquels, **D** est un atome d'oxygène, **Z1**, **Z2 et Z3** sont absents, **Z4** représente un atome d'hydrogène, **X** représente un atome de soufre et **p** = 0, ces composés sont appelés ci-après composés de formule (B).
Le **tableau III** illustre des composés de formule (I) selon l'invention pour lesquels, **Y** représente Ph, **Z4, R, R1 et R2** représentent des atomes d'hydrogène, **n=1, Z1, Z2 et Z3** sont absents, **X** représente un atome de soufre, **p**=0, W représente un atome de carbone, ces composés sont appelés ci-après composés de formule (C).

Le **tableau IV** illustre des composés de formule (I) selon l'invention pour lesquels **R1, R2 et Z4** représente chacun un atome d'hydrogène, **n=1** et **Z1** est absent, **W** représente un atome de carbone, **D** représente une liaison, et **p =** 0 ; ces composés sont appelés ci-après composés de formule (D).

Le **tableau V** illustre des composés de formule (I).

Dans ces tableaux :
- les composés du **tableau I** sont sous forme trans majoritaire ou sous forme trans uniquement, sauf indication contraire ;
- dans les **tableaux II, III, IV et** V, la stéréochimie cis/trans des composés est indiquée ;
- « - » dans la colonne « Z1, Z3 » ou la colonne « R1 » ou la colonne « R2 » indique que le groupe correspondant est absent ;
- « * » indique l'atome de liaison,
- Me, Et, n-Pr, i-Pr, t-Bu et i-Bu représentent respectivement des groupes méthyle, éthyle, n-propyle, isopropyle, tert-butyle et isobutyle,
- « PF » représente le point de fusion du composé, exprimé en degrés Celsius (°C). « puis déc. » signifie « puis décomposition du composé »
- « MH⁺ » représente la masse M+H du composé, obtenue par LC-MS (abréviation de Liquid Chromatography - Mass Spectroscopy).
- « ¤ » dans les colonnes « MH+ » ou « PF » indique que la mesure n'a pas été faite.

**TABLEAU I**

| | | | | | | | | |
|---|---|---|---|---|---|---|---|---|
| 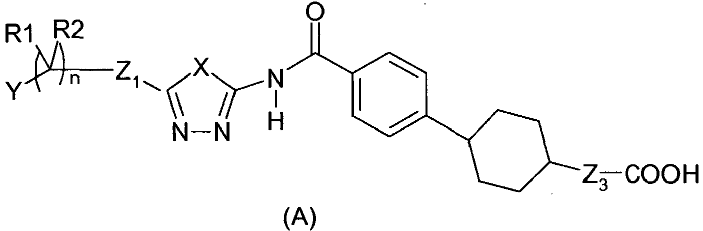 | | | | | | | | |

| **N°** | **n** | **R1** | **R2** | **X** | **Y** | **Z1/Z3** | **MH+** | **PF** |
|---|---|---|---|---|---|---|---|---|
| **1** | 1 | H | H | S | 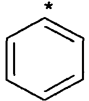 | -/CH₂ | 436 | 279°C |
| **2** | 1 | H | H | S | 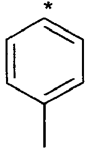 | -/CH₂ | 450 | >250°C |
| **3** | 1 | H | H | S | 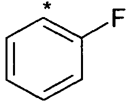 | -/CH₂ | 454 | 271 |
| **4** | 1 | H | H | S | 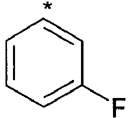 | -/CH₂ | 454 | 277 |
| **5** | 1 | H | H | S | 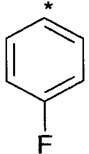 | -/CH₂ | 454 | >250°C |
| **6** | 1 | H | H | S | 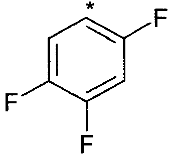 | -/CH₂ | 490 | 242 |
| **7** | 1 | 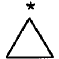 | | S | 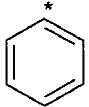 | -/CH₂ | 462 | >250°C |
| **8** | 1 | 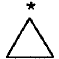 | | S | 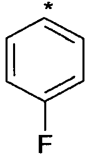 | -/CH₂ | 480 | >250°C |
| **9** | 1 | 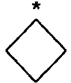 | | S | 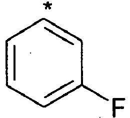 | -/CH₂ | 494 | 275 |
| **10** | 1 | H | H | S | 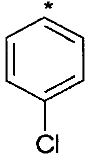 | -/CH₂ | 470 | >250°C |
| **11** | 1 | H | H | S | 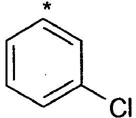 | -/CH₂ | 470 | 255-260 |
| **12** | 1 | H | H | S | 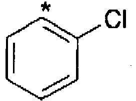 | -/CH₂ | 470 | 245-250 |
| **13** | 1 | H | H | S | 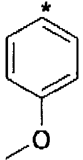 | -/CH₂ | 466 | 254 |
| **14*** | 0 | - | - | S | 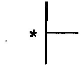 | -/CH₂ | 402 | 298 |
| **15*** | 0 | - | - | S | 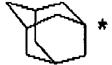 | -/CH₂ | 480 | 320 |
| **16*** | 0 | - | - | S | 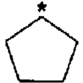 | -/CH₂ | 414 | 274 |
| **17** | 1 | - | - | S | 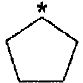 | -/CH₂ | 428 | 268 |
| **18** | 2 | H | H | S | 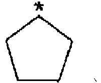 | -/CH₂ | 442 | 235 puis dec. |
| **19*** | 0 | - | - | S | -iBu | -/CH₂ | 402 | 263 |
| **20** | 2 | H | H | S | 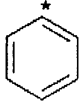 | -/CH₂ | 450 | 270 |
| **21** | 2 | H | H | S | 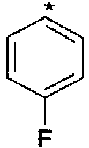 | -/CH₂ | 468 | 270 |
| **22** | 1 | H | H | S | 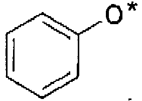 | -/CH₂ | 452 | 261 |
| **23** | 3 | H | H | S | 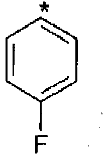 | -/CH₂ | 482 | 229 |
| **24** | 1 | H | H | S | 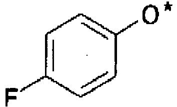 | -/CH₂ | 470 | 275 |
| **25*** | 0 | - | - | S | 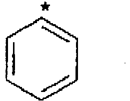 | -/CH₂ | 422 | 240 puis dec. |
| **26*** | 0 | - | - | S | 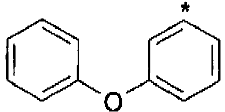 | -/CH₂ | 514 | 240 puis dec. |
| **27*** | 0 | - | - | S | 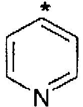 | -/CH₂ | 423 | >250 |
| **28*** | 0 | - | - | S | 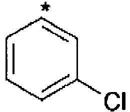 | -/CH₂ | 456 | >250 |
| **29*** | 0 | - | - | S | 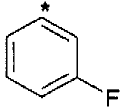 | -/CH₂ | 440 | >250 |
| **30*** | 0 | - | - | S | 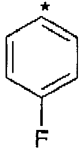 | -/CH₂ | 440 | >250 |
| **31*** | 0 | - | - | S | 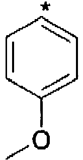 | -/CH₂ | 452 | >250 |
| **32** | 1 | H | H | O | 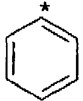 | -/CH₂ | 420 | € |
| **33** | 1 | H | H | O | 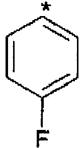 | -/CH₂ | 438 | 215 |
| **34 cis** | 1 | H | H | S | 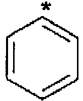 | -/CH₂ | 436 | 277 |
| **35*** | 0 | - | - | S | 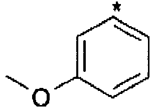 | -/CH₂ | 452 | 264-282 |
| **36*** | 0 | - | - | S | 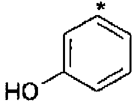 | -/CH₂ | 438 | 286 puis dec. |
| **37** | 1 | H | H | S | 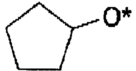 | -/CH₂ | 444 | >250 |
| **38** | 2 | H | H | S | 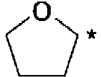 | -/CH₂ | 444 | 249 |
| **39** | 2 | H | H | S | 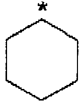 | -/CH₂ | 456 | >260 |
| **40** | 1 | H | H | S | 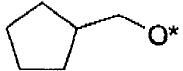 | CH₂/CH₂ | 458 | 241 |
| **41** | 1 | H | H | S | 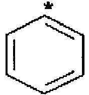 | S/CH₂ | 468 | € |
| **44** | 1 | H | H | S | 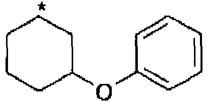 | -/CH₂ | 520 | 245 |
| **45** | 1 | H | H | S | 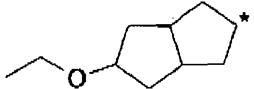 | -/CH₂ | 511 | 201 puis dec. |
| **47*** | 0 | - | - | S | Br | -/CH₂ | 424 | € |
| **48*** | 2 | H | H | S | 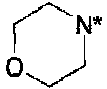 | -/CH₂ | 459 | 213 |
| **49*** | 0 | - | - | S | 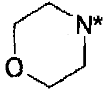 | -/CH₂ | 431 | >250 |
| **50*** | 0 | - | - | S | COOH | -/CH₂ | € | 275-280 |
| **51** | 1 | H | H | S | 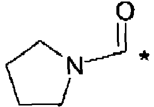 | -/CH₂ | 457 | >260 |

**TABLEAU II**

| | | | | | | | | |
|---|---|---|---|---|---|---|---|---|
| 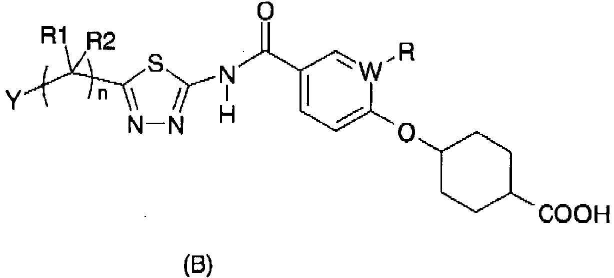 | | | | | | | | |

| **N°** | **n** | **R** | **R1** | **R2** | **W** | **Y** | **MH+** | **PF** |
|---|---|---|---|---|---|---|---|---|
| **52 cis** | 1 | H | H | H | C | 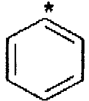 | 438 | 266 |
| **53 trans** | 1 | H | H | H | C | 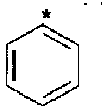 | 438 | 270 |
| **54 trans*** | 0 | H | - | - | C | 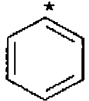 | 424 | 306 |
| **55 cis** | 1 | F | H | H | C | 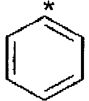 | 456 | 290 |
| **56 cis** | 1 | Cl | H | H | C | 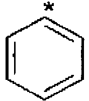 | 472 | 263 |
| **57 cis** | 2 | H | H | H | C | 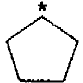 | 444 | 270 |
| **58 cis*** | 0 | H | - | - | C | 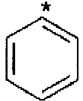 | 424 | 291 |
| **59 cis*** | 0 | H | - | - | C | 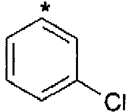 | 458 | 296 |
| **60 cis*** | 0 | H | - | - | C | 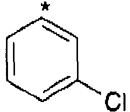 | 476 | 288 |
| **61 cis*** | 0 | H | - | - | C | H | 348 | 277 |
| **62 cis** | 1 | H | H | H | N | 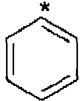 | 439 | 252-257 |
| **63 cis** | 1 | H | H | H | N | 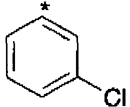 | 473 | 256 |
| **64 cis** | 2 | H | H | H | N | 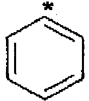 | 453 | 255-260 |
| **65 cis*** | 0 | H | - | - | N | 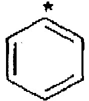 | 425 | >265 |
| **66 cis*** | 0 | H | - | - | N | 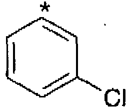 | 459 | 277-280 |
| **67 cis** | 1 | H | H | H | C | 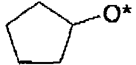 | 445 | 272 |

**TABLEAU III**

| | | | |
|---|---|---|---|
| 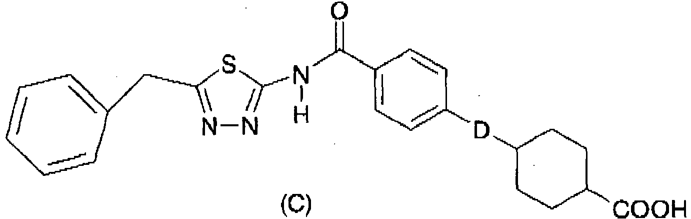 | | | |

| **N°** | **D** | **MH+** | **PF** |
|---|---|---|---|
| **68 cis** | NH | 437 | 248 |
| **69 trans** | NH | 437 | 265 |
| **70 trans** | liaison | 422 | 247 puis dec. |

**TABLEAU IV**

| | | | | | |
|---|---|---|---|---|---|
| 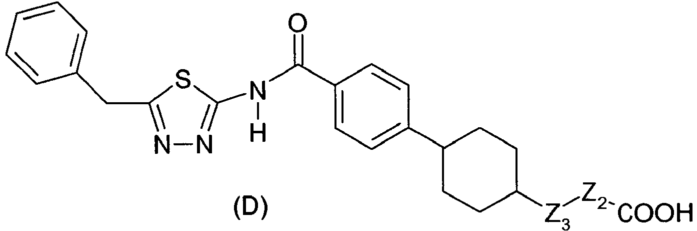 | | | | | |

| **N°** | **Z2** | **Z3** | **Motif formé Entre Z2 et Z3** | **MH+** | **PF** |
|---|---|---|---|---|---|
| 71 | -CH= | =CH- | Double liaison | 448 | >250 |
| 72 | 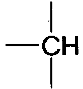 | 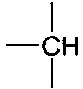 | 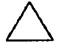 | 462 | >250 |
| 73 trans | -CH2- | -CH2- | Simple liaison | 450 | 275-280 |

**TABLEAU V**

| **N°** | **Molécules (I)** | **MH+** | **PF** |
|---|---|---|---|
| **74** | 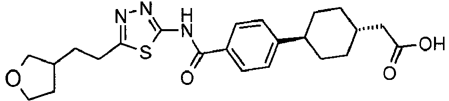 | 444 | >260 |
| **75** | 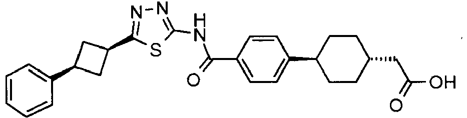 | 476 | 258 |
| **76** | 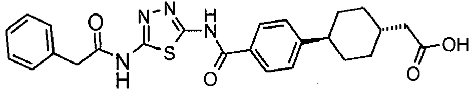 | 479 | 291 |
| **78** | 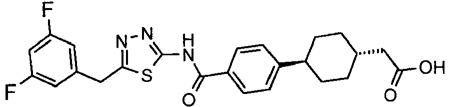 | 472 | 294 |
| **79** | 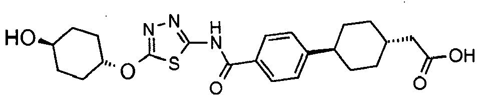 | 458 | >250 |
| **80** | 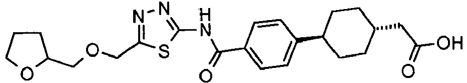 | 460 | 220 puis déc. |
| **81** | 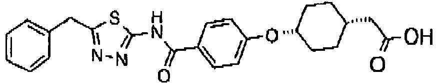 | 452 | 230-250 |
| **82** | 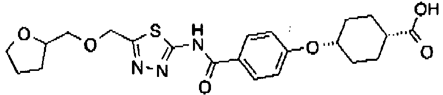 | 462 | 217-223 |
| **83** | 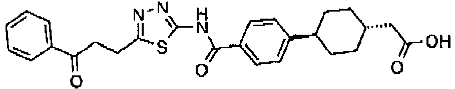 | 478 | 240-268 |
| **84** | 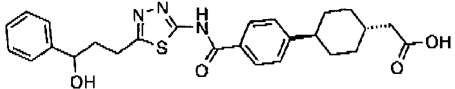 | 480 | 239-242 |
| **85*** | 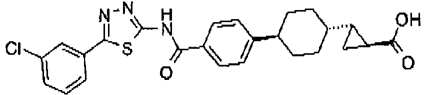 | 482 | >250 |
| **86** | 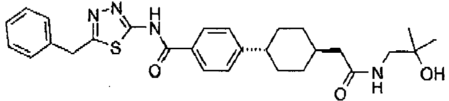 | 507 | 260 |
| **87** | 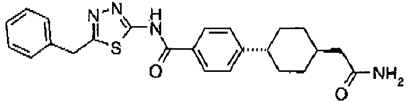 | 435 | 280 |
| **88** | 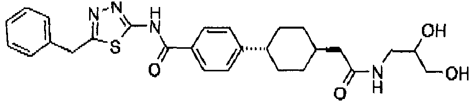 | 509 | 224 |
| **89** | 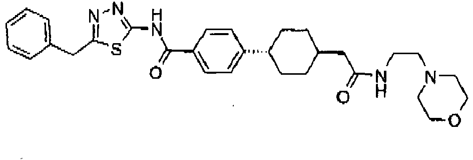 | 548 | 227 |
| **90** | 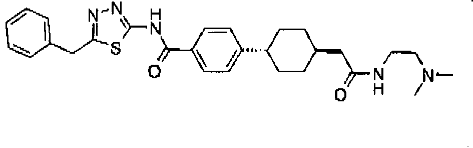 | 506 | 256 |
| **91** | 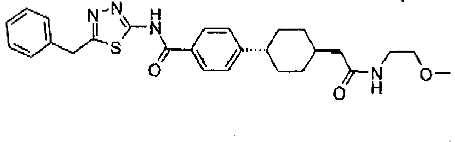 | 493 | 268 |
| **92*** | 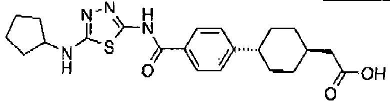 | 429 | 335 |
| **93** | 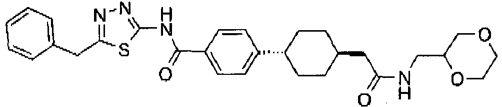 | 535 | 281 |
| **94** | 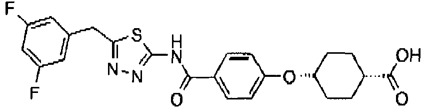 | 474 | 235-237 |
| **95** | 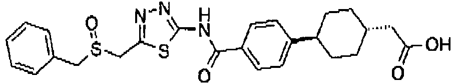 | 498 | 241 |
| **96** | 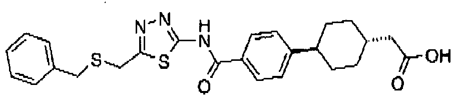 | 482 | 234 |
| **97** | 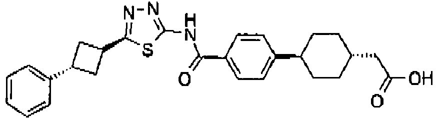 | 476 | 247 |
| **98** | 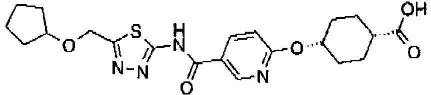 | 447 | € |
| **101*** | 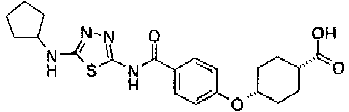 | 431 | 294 |
| **102** | 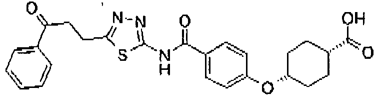 | 480 | 220-260 |
| **42** | 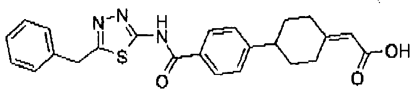 | 434 | 255 |
| **43** | 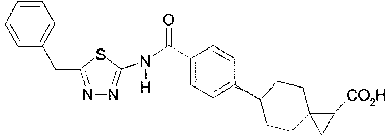 | 448 | 255 |
| **46** | 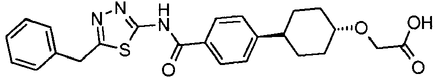 | 452 | 250-255 |

Les composés selon l'invention ont fait l'objet d'essais pharmacologiques permettant de déterminer leur effet inhibiteur de la biosynthèse des triglycérides.

Ces essais ont consisté à mesurer l'activité inhibitrice *in vitro* des composés de l'invention sur un test cellulaire.

Les cellules Chang Liver à 80% de confluence sont décollées à la trypsine-EDTA, 4 ml par flasque de 175 cm². Après centrifugation 1300 g pendant 5 min, le culot cellulaire est lavé une fois par du PBS avant d'être remis en suspension dans du milieu complet. Le nombre de cellules et leur viabilité sont déterminés sur cellules de Mallassez par la méthode d'exclusion au bleu trypan.
150 000 cellules sont ensemencées par puits d'une plaque 24 puits pendant 3 h minimum dans le milieu DMEM 4.5 g/l de glucose additionné de 10% de SVF et d'antibiotiques et sont maintenues à 37°C dans l'incubateur à CO₂ (5%).
Après 3 h, les cellules ont adhéré, le milieu est éliminé et remplacé pendant une nuit par du milieu DMEM 4,5 g/l de glucose avec 2 % de BSA/oléate.
A l'issue de ces 18h de culture sans sérum, les composés à tester sont incubés pendant 30 min (de 1,3, 10, 30, 100, 300 et 1000 nM) avec les cellules avant d'ajouter le [¹⁴C] glycérol (0,4 µCi/ml/puit) pour une durée d'incorporation de 6 h.
Le surnageant est aspiré et les cellules sont récupérées par traitement à la trypsine-EDTA, 100 µl/puits, 5 min à 37°C. Cette suspension cellulaire est ensuite récupérée dans un tube Eppendorf et est lavée par 2 lavages de 500 µl de PBS. Une centrifugation à 1300 g pendant 5 min permet de récupérer les cellules en culot qui peuvent être congelées à -20°C. Afin d'extraire les lipides à partir du culot cellulaire, 400 µl d'un mélange méthanol/dichlorométhane/trifluoroacétique acide (50/50/0,1%) est utilisé pour resuspendre les cellules. Puis, les membranes cellulaires sont détruites par sonication au bain-marie, 30 min. Les échantillons sont filtrés sur 0,45 µm avant injection sur HPLC sur une colonne C18 de 4,6 X 75 mm, 3 µm avec une phase mobile 5% (H₂O + 0,1%TFA), 70% méthanol, 25% dichlorométhane avec un débit de 1,5 ml/minute. La radioactivité est mesurée à l'aide d'un Flo One C625TR (Perkin Elmer).

L'activité inhibitrice vis-à-vis de la biosynthèse des triglycérides est donnée par la concentration qui inhibe 50% de l'activité.

Les activités des composés selon l'invention sont généralement comprises entre 0,01 µM et 10 µM et plus particulièrement entre 0,01 et 1 µM.
Par exemple, les activités des composés n° 1 ; 4 ; 23 ; 25 ; 37 ; 40 ; 63 et 68 sont respectivement de 0,029 ; 0,046 ; 0,181 ; 0,590 ; 0,018 ; 0,051 ; 0,085 et 0,271 µM.
Il apparaît donc que les composés selon l'invention ont une activité inhibitrice de la biosynthèse des triglycérides.

Les composés selon l'invention peuvent donc être utilisés pour la préparation de médicaments, en particulier de médicaments inhibiteurs de la biosynthèse des triglycérides.

Ainsi, selon un autre de ses aspects, l'invention a pour objet des médicaments qui comprennent un composé de formule (I), ou un sel d'addition de ce dernier à un acide ou à une base pharmaceutiquement acceptable du composé de formule (I).

Ces médicaments trouvent leur emploi en thérapeutique, notamment dans le traitement et/ou la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique, des maladies coronariennes, de l'hypertension, des maladies de peau, de la maladie d'Alzheimer, des maladies immunomodulatrices, de l'infection à VIH, du syndrome inflammatoire de l'intestin, de certains cancers, avantageusement pour la préparation d'un médicament destiné au traitement ou à la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique.

Selon un autre de ses aspects, la présente invention concerne des compositions pharmaceutiques comprenant, en tant que principe actif, un composé selon l'invention. Ces compositions pharmaceutiques comprennent une dose efficace d'au moins un composé selon l'invention ou un sel pharmaceutiquement acceptable dudit composé, ainsi qu'au moins un excipient pharmaceutiquement acceptable. Lesdits excipients sont choisis selon la forme pharmaceutique et le mode d'administration souhaités, parmi les excipients habituels qui sont connus de l'homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale, telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composé répondant à la **formule (I)** 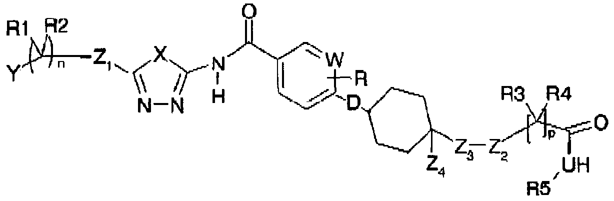 dans laquelle
• **U** représente un atome d'oxygène ou un atome d'azote, sachant que lorsque U représente un atome d'oxygène alors R5 est absent ;
• **n** est égale à 1, 2 ou 3 ;
• **p** est égal à 0, 1 ou 2 ;
• **D** représente un atome d'oxygène, un groupe -NH- ou une liaison ;
• **W** représente un atome de carbone ou d'azote ;
• **X** représente un hétéroatome choisi parmi un atome d'oxygène ou un atome de soufre ;
• **R1, R2, R3 et R4** représentent, indépendamment l'un de l'autre,
○ un atome d'hydrogène,
○ un groupe -(C1-C6)alkyle ou bien,
○ (i) R1 et R2 peuvent former avec l'atome de carbone auquel ils sont rattachés un groupe -(C3-C10)cycloalkyle- et/ou (ii) R3 et R4 peuvent former avec l'atome de carbone auquel ils sont rattachés un groupe -(C3-C10)cycloalkyle- ;
• **Y** représente un atome d'hydrogène, un groupe -(C1-C6)alkyle, -(C3-C10)cycloalkyle-, (C3-C10)cycloalkyloxy-, (C3-C10)cycloalkyl-(C1-C6)alkyloxy-, heterocycloalkyle-(C1-C6)alkyloxy-, un groupe -COOR1, aryle, arylalkyle, hétéroaryle, hétérocycloalkyle, aryloxy, -C(O)-hétérocycloalkyle, -C(O)aryle, -CH(OH)aryle, -NH-cycloalkyle lesdits groupes étant éventuellement substitués par un ou plusieurs substituants choisis parmi un atome d'halogène, un groupe hydroxyl, un groupe (C1-C6)alkyle, (C1-C6)alcoxy, hétérocycloalkyle ou un groupe aryloxy ;
• **R** représente un atome d'hydrogène ou d'halogène ;
• **Z1** est absent ou représente un atome de soufre, une fonction-NH-, -NHC(O)-, un groupe -S(O)-CH₂-, -SCH₂-, méthylène ou un groupe éthylène ;
• **Z2** est absent ou représente un groupe méthylène, un groupe 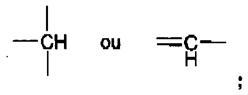
• **Z3** est absent ou représente un atome d'oxygène ou un groupe méthylène, un groupe 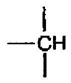 ou 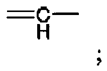 Sachant que **Z2** ne représente un groupe 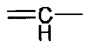 que lorsque **Z3** est présent et qu'il représente un groupe 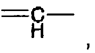 et réciproquement, **Z2 et Z3** formant ainsi une double liaison ;
Sachant que **Z2** et **Z3,** lorsqu'ils sont présents, peuvent être compris dans un groupe cycloalkyle ;
• **Z4** est
○ un atome d'hydrogène,
○ un atome de carbone formant éventuellement avec Z3 un groupe -(C3-C10)cycloalkyle- lorsque Z3 est un groupe 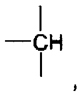 ou
○ est absent, Z3 étant alors un groupe 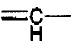 formant une double liaison avec le carbone du cyclohexyle qui lui est adjacent;
• **R5** représente un atome d'hydrogène ou un groupe alkyle éventuellement substitué par au moins un groupe hydroxyle, hétérocycloalkyle-(C1-C6)alkyle, amine ou alkyloxy,
sous forme d'acide, de base ou de sel d'addition à un acide ou à une base.

2. Composé de formule (I) selon la revendication 1, **caractérisé en ce que** :
• **U** représente un atome d'oxygène ou un atome d'azote, sachant que lorsque **U** représente un atome d'oxygène alors **R5** est absent ;
et/ou
• **n** est égale à 1, 2 ou 3 ;
et/ou
• **p** est égal à 0, 1 ou 2 ;
et/ou
• **D** représente un atome d'oxygène, un groupe -NH- ou une liaison ;
et/ou
• **W** représente un atome de carbone ou d'azote ;
et/ou
• **X** représente un hétéroatome choisi parmi un atome d'oxygène ou un atome de soufre ;
et/ou
• **R1, R2, R3 et R4** représentent, indépendamment l'un de l'autre,
○ un atome d'hydrogène,
○ un groupe -(C1-C6)alkyle ou bien,
○ (i) R1 et R2 peuvent former avec l'atome de carbone auquel ils sont rattachés un groupe -(C3-C10)cycloalkyle- et/ou (ii) R3 et R4 peuvent former avec l'atome de carbone auquel ils sont rattachés un groupe -(C3-C10)cycloalkyle- ;
et/ou
• **Y** représente un atome d'hydrogène, un groupe -(C1-C6)alkyle, -(C3-C10)cycloalkyle-, (C3-C10)cycloalkyloxy-, (C3-C10)cycloalkyl-(C1-C6)alkyloxy-, heterocycloalkyle-(C1-C6)alkyloxy-, un groupe -COOR1, aryle, arylalkyle, hétéroaryle, hétérocycloalkyle, aryloxy, -C(O)-hétérocycloalkyle, -C(O)aryle, -CH(OH)aryle, -NH-cycloalkyle lesdits groupes étant éventuellement substitués par un ou plusieurs substituants choisis parmi un atome d'halogène, un groupe hydroxyl, un groupe (C1-C6) alkyle, (C1-C6) alcoxy, hétérocycloalkyle ou un groupe aryloxy :
et/ou
• **R** représente un atome d'hydrogène ou d'halogène ;
et/ou
• **Z1** est absent ou représente un atome de soufre, une fonction-NH-, -NHC(O)-, un groupe -S(O)-CH₂-, -SCH₂-, méthylène ou un groupe éthylène ;
et/ou
• **Z2** est absent ou représente un groupe méthylène, un groupe 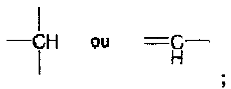 et/ou
• **Z3** est absent ou représente un atome d'oxygène ou un groupe méthylène, un groupe 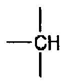 ou 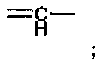 et/ou
• **Z2** et **Z3** sont présents et peuvent représenter chacun un groupe 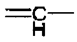 et forment ainsi une double liaison ;
et/ou
• **Z2** et **Z3** sont présents et peuvent être compris dans un groupe cycloalkyle ;
et/ou
• **Z4** est
○ un atome d'hydrogène.
○ un atome de carbone formant éventuellement avec Z3 un groupe -(C3-C10)cycloalkyle- lorsque Z3 est un groupe 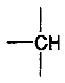 ou
○ est absent, Z3 étant un groupe 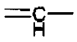 formant une double liaison avec le carbone du cyclohexyle qui lui est adjacent;
et/ou
• **R5** représente un atome d'hydrogène ou un groupe alkyle éventuellement substitué par au moins un groupe hydroxyle, heterocycloalkyle-(C1-C6)alkyle, amine ou alkyloxy,
et/ou
• ledit composé (I) se présente sous forme d'acide, de base ou de sel d'addition à un acide ou à une base.

3. Composé de formule (I) selon la revendication 1 ou 2, **caractérisé en ce qu'**il s'agit du composé (I') de formule suivante : 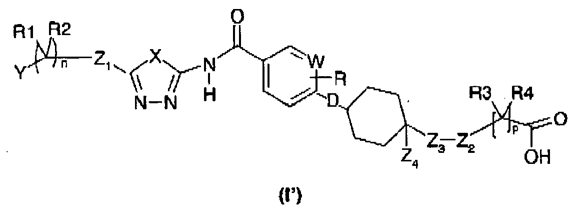
dans laquelle **Y, R1, R2**, **n, Z1**, **X, W, R, D, Z4, Z3, Z2, R3, R4 et p** sont tels que définis dans l'une quelconque des revendications 1 ou 2.

4. Composé de formule (I) selon la revendication 1 ou 2, **caractérisé en ce qu'**il s'agit du composé (I") de formule suivante : 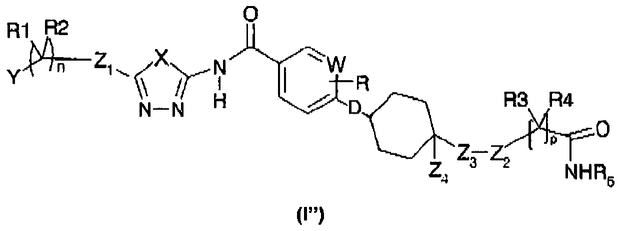
dans laquelle **Y, R1, R2, n, Z1**, **X, W, R, D, Z4, Z3, Z2, R3, R4, p et R5** sont tels que définis dans l'une quelconque des revendications 1 ou 2.

5. Composé selon l'une quelconque des revendications 1 à 4, **caractérisé en ce que** D représente une liaison.

6. Composé selon l'une quelconque des revendications 1 à 4, **caractérisé en ce que** D représente un atome d'oxygène.

7. Composé selon quelconque l'une quelconque des revendications 1 à 6, **caractérisé en ce que p** est égale à 0.

8. Composé selon l'une quelconque des revendications 1 à 7, **caractérisé en ce que** :
• **Z3** et **Z2** représente chacun un méthylène, ou
• **Z3** représent un méthylène et **Z2** est absent, ou
• **Z3** et **Z2** sont absents.

9. Composé selon l'une quelconque des revendications 1 à 7, **caractérisé en ce que** :
• **Z3** et **Z2** sont compris dans un groupe cycloalkyle, avantageusement un cyclopropyle, ou
• **Z3** et **Z2** forment ensemble une double liaison.

10. Composé selon l'une quelconque des revendications 1 à 9, **caractérisé en ce que W** représente un atome de carbone.

11. Composé selon l'une quelconque des revendications 1 à 10, **caractérisé en ce que X** représente un atome de soufre.

12. Composé selon l'une des revendications précédentes, **caractérisé en ce qu'**il s'agit de :
- acide {4-[4-(5-benzyl-[1,3,4]thiadiazol-2-ylcarbamoyl)-phényl]-cyclohexyl}-acétique
- acide (4-{4-[5-(4-méthyl-benzyl)-[1,3,4]thiadiazol-2-ylcarbamoyl]-phényl}-cyclohexyl)-acétique
- acide (4-{4-[5-(2-fluoro-benzyl)-[1,3,4]thiadiazol-2-ylcarbamoyl]-phényl}-cyclohexyl)-acétique
- acide (4-{4-[5-(3-fluoro-benzyl)-[1,3,4]thiadiazol-2-ylcarbamoyl]-phényl}-cyclohexyl)-acétique
- acide (4-{4-[5-(4-fluoro-benzyl)-[1,3,4]thiadiazol-2-ylcarbamoyl]-phényl}-cyclohexyl)-acétique
- acide (4-{4-[5-(2,4,5-trifluoro-benzyl)-[1,3,4]thiadiazol-2-ylcarbamoyl]-phényl}-cyclohexyl)-acétique
- acide [4-(4-{5-[1-(phényl)-cyclopropyl]-[1,3,4]thiadiazol-2-ylcarbamoyl}-phényl)-cyclohexyl]-acétique
- acide [4-(4-{5-[1-(4-fluoro-phényl)-cyclopropyl]-[1,3,4]thiadiazol-2-ylcarbamoyl}-phényl)-cyclohexyl]-acétique
- acide [4-(4-{5-[1-(3-fluoro-phényl)-cyclobutyl]-[1,3,4]thiadiazol-2-ylcarbamoyl}-phényl)-cyclohexyl]-acétique
- acide (4-{4-[5-(4-chloro-benzyl)-[1,3,4]thiadiazol-2-ylcarbamoyl]-phényl}-cyclohexyl)-acétique
- acide (4-{4-[5-(3-chloro-benzyl)-[1,3,4]thiadiazol-2-ylcarbamoyl]-phényl}-cyclohexyl)-acétique
- acide (4-{4-[5-(2-chloro-benzyl)-[1,3,4]thiadiazol-2-ylcarbamoyl]-phényl}-cyclohexyl)-acétique
- acide (4-{4-[5-(4-méthoxy-benzyl)-[1,3,4]thiadiazol-2-ylcarbamoyl]-phényl}-cyclohexyl)-acétique
- acide {4-[4-(5-tert-butyl-[1,3,4]thiadiazol-2-ylcarbamoyl)-phényl]-cyclohexyl}-acétique
- acide {4-[4-(5-cyclopentylméthyl-[1,3,4]thiadiazol-2-ylcarbamoyl)-phényl]-cyclohexyl}-acétique
- acide (4-{4-[5-(2-cyclopentyl-éthyl)-[1,3,4]thiadiazol-2-ylcarbamoyl]-phényl}-cyclohexyl)-acétique
- acide {4-[4-(5-isobutyl-[1,3,4]thiadiazol-2-ylcarbamoyl)-phényl]-cyclohexyl}-acétique
- acide {4-[4-(5-phénéthyl-[1,3,4]thiadiazol-2-ylcarbamoyl)-phényl]-cyclohexyl}-acetique
- acide [4-(4-{5-[2-(4-fluoro-phényl)-éthyl]-[1,3,4]thiadiazol-2-ylcarbamoyl}-phényl)-cyclohexyl]-acétique
- acide {4-[4-(5-phénoxyméthyl-[1,3,4]thiadiazol-2-ylcarbamoyl)-phényl]-cyclohexyl}-acetique
- acide [4-(4-{5-[3-(4-fluoro-phényl)-propyl]-[1,3,4]thiadiazol-2-ylcarbamoyl}-phényl)-cyclohexyl]-acétique
- acide (4-{4-[5-(4-fluoro-phénoxyméthyl)-[1,3,4]thiadiazol-2-ylcarbamoyl]-phényl}-cyclohexyl)-acétique
- acide (4-{4-[5-(benzyl)-[1,3,4]oxadiazol-2-ylcarbamoyl]-phényl}-cyclohexyl)-acétique
- acide (4-{4-[5-(4-fluoro-benzyl)-[1,3,4]oxadiazol-2-ylcarbamoyl]-phényl}-cyclohexyl)-acétique
- acide cis-4-[4-(5-benzyl-[1,3,4]thiadiazol-2-ylcarbamoyl)-phénoxy]-cyclohexane-carboxylique
- acide trans-4-[4-(5-benzyl-[1,3,4]thiadiazol-2-ylcarbamoyl)-phénoxy]-cyclohexane-carboxylique
- acide cis-4-[4-(5-benzyl-[1,3,4]thiadiazol-2-ylcarbamoyl-2-fluoro-phénoxy]-cyclohexanecarboxylique
- acide cis-4-[4-(5-benzyl-[1,3,4]thiadiazol-2-ylcarbamoyl)-2-chloro-phénoxy]-cyclohexanecarboxylique
- acide cis-4-{4-[5-(2-cyclopentyl-éthyl)-[1,3,4]thiadiazol-2-ylcarbamoyl]-phénoxy}-cyclohexanecarboxylique
- acide cis-4-[5-(5-benzyl-[1,3,4]thiadiazol-2-ylcarbamoyl)-pyridin-2-yloxy]-cyclohexanecarboxylique
- acide cis-4-{5-[5-(3-chloro-benzyl)-[1,3,4]thiadiazol-2-ylcarbamoyl]-pyridin-2-yloxy}-cyclohexanecarboxylique
- acide cis-4-[5-(5-phénéthyl-[1,3,4]thiadiazol-2-ylcarbamoyl)-pyridin-2-yloxy]-cyclohexanecarboxylique
- acide cis-4-[4-(5-benzyl-[1,3,4]thiadiazol-2-ylcarbamoyl)-phénylamino]-cyclohexane-carboxylique
- acide trans-4-[4-(5-benzyl-[1,3,4]thiadiazol-2-ylcarbamoyl)-phénylamino]-cyclohexane-carboxylique
- acide trans-{4-[4-(5-benzyl-[1,3,4]thiadiazol-2-ylcarbamoyl)-phényl]-cyclohexyl}-acétique
- acide trans-4-[4-(5-benzyl-[1,3,4]thiadiazol-2-ylcarbamoyl)-phényl]-cyclohexane-carboxylique
- acide trans-{4-[4-(5-cyclopentyloxyméthyl-[1,3,4]thiadiazol-2-ylcarbamoyl)-phényl]-cyclohexyl}-acétique
- acide trans-[4-(4-{5-[2-(tetrahydro-furan-2-yl)-éthyl]-[1,3,4]thiadiazol-2-ylcarbamoyl}-phényl)-cyclohexyl]-acétique
- acide trans-(4-{4-[5-(2-cyclohexyl-éthyl)-[1,3,4]thiadiazol-2-ylcarbamoyl]-phényl}-cyclohexyl)-acétique
- acide trans-{4-[4-(5-cyclopentylméthoxyméthyl-[1,3,4]thiadiazol-2-ylcarbamoyl)-phényl]-cyclohexyl}-acétique
- acide trans-{4-[4-(5-benzylsulfanyl-[1,3,4]thiadiazol-2-ylcarbamoyl)-phényl]-cyclohexyl}-acétique
- acide {4-[4-(5-benzyl-[1,3,4]thiadiazol-2-ylcarbamoyl)-phényl]-cyclohexylidène}-acétique
- acide 6-[4-(5-benzyl-[1,3,4]thiadiazol-2-ylcarbamoyl)-phényl]-spiro[2.5]octane-1-carboxylique
- acide (E)-3-{4-[4-(5-benzyl-[1,3,4]thiadiazol-2-ylcarbamoyl)-phényl]-cyclohexyl}-acrylique
- acide trans-(1R,2S/1S,2R)-2-{4-[4-(5-benzyl-[1,3,4]thiadiazol-2-ylcarbamoyl)-phényl]-cyclohexyl}-cyclopropanecarboxylique
- acide trans-3-{4-[4-(5-benzyl-[1,3,4]thiadiazol-2-ylcarbamoyl)-phényl]-cyclohexyl}-propionique
- acide (4-(4-[5-((1S,3S/1R,3R)-3-phénoxy-cyclohexyl)-[1,3,4]thiadiazol-2-ylcarbamoyl]-phényl}-cyclohexyl)-acétique
- acide trans-(4-{4-[(5-{[(3R,6S/3S,6R)-5-éthoxyoctahydropentalèn-2-yl]méthyl}-1,3,4-thiadiazol-2-yl)carbamoyl]phényl}cyclohexyl)acétique
- acide trans-{4-[4-(5-benzyl-[1,3,4]thiadiazol-2-ylcarbamoyl)-phényl]-cyclohexyloxy}-acétique
- acide trans-(4-{4-[5-(2-morpholin-4-yl-éthyl)-[1,3,4]thiadiazol-2-ylcarbamoyl]-phényl)-cyclohexyl)-acétique
- acide trans-(4-{4-[5-(2-oxo-2-pyrrolidin-1-yl-éthyl)-[1,3,4]thiadiazol-2-ylcarbamoyl]-phényl}-cyclohexyl)-acétique
- acide cis-4-[4-(5-cyclopentyloxyméthyl-[1,3,4]thiadiazol-2-ylcarbamoyl)-phénoxy]-cyclohexanecarboxylique.
- acide trans-[4-(4-{5-[2-(tetrahydro-furan-3-yl)-éthyl]-[1,3,4]thiadiazol-2-ylcarbamoyl}-phényl)-cyclohexyl]-acétique
- acide trans-(4-{4-[5-(3-phényl-cyclobutyl)-[1,3,4]thiadiazol-2-ylcarbamoyl]-phényl}-cyclohexyl)-acétique
- acide {4-[4-(5-Phenylacetylamino-[1,3,4]thiadiazol-2-ylcarbamoyl)-phenyl]-cyclohexyl}-acetique
- acide trans-{4-[4-(5-cyclopentyloxyméthyl-[1,3,4]thiadiazol-2-ylcarbamoyl)-phényl]-cyclohexyl}-acétique
- acide trans-(4-{4-[5-(3,6-difluoro-benzyl)-[1,3,4]thiadiazol-2-ylcarbamoyl]-phényl}-cyclohexyl)-acétique
- acide trans-(4-{4-[5-(4-hydroxy-cyclohexylméthyl)-[1,3,4]thiadiazol-2-ylcarbamoyl]-phényl}-cyclohexyl)-acétique
- acide trans-(4-{4-[5-(tetrahydro-furan-2-ylméthoxyméthyl)-[1,3,4]thiadiazol-2-ylcarbamoyl]-phényl}-cyclohexyl}-acétique
- acide trans-{4-[4-(5-benzyl-[1,3,4]thiadiazol-2-ylcarbamoyl)-phénoxy]-cyclohexyl}-acétique
- acide trans-4-{4-[5-(tetrahydro-furan-2-ylméthoxyméthyl)-[1,3,4]thiadiazol-2-ylcarbamoyl]-phénoxy}-cyclohexanecarboxylique
- acide trans-(4-{4-[5-(3-oxo-3-phényl-propyl)-[1,3,4]thiadiazol-2-ylcarbamoyl]-phényl}-cyclohexyl)-acétique
- acide trans-(4-{4-[5-(3-hydroxy-3-phényl-propyl)-[1,3,4]thiadiazol-2-ylcarbamoyl]-phényl}-cyclohexyl)-acétique
- trans-N-(5-benzyl-[1,3,4]thiadiazol-2-yl)-4-{4-[(2-hydroxy-2-méthyl-propylcarbamoyl)-méthyl]-cyclohexyl}-benzamide
- trans-N-(5-benzyl-[1,3,4]thiadiazol-2-yl)-4-(4-carbamoylméthyl-cyclohexyl)-benzamide
- trans-N-(5-benzyl-[1,3,4]thiadiazol-2-yl)-4-{4-[(2,3-dihydroxy-propylcarbamoyl)-méthyl]-cyclohexyl}-benzamide
- trans-N-(5-benzyl-[1,3,4]thiadiazol-2-yl)-4-{4-[(2-morpholin-4-yl-éthylcarbamoyl)-méthyl]-cyclohexyl}-benzamide
- trans-N-(5-benzyl-[1,3,4]thiadiazol-2-yl)-4-{4-[(2-diméthylamino-éthylcarbamoyl)-méthyl]-cyclohexyl}-benzamide
- trans-N-(5-benzyl-[1,3,4]thiadiazol-2-yl)-4-{4-[(2-méthoxy-éthylcarbamoyl)-méthyl]-cyclohexyl}-benzamide
- trans-N-(5-benzyl-[1,3,4]thiadiazol-2-yl)-4-(4-{[([1,4]dioxan-2-ylméthyl)-carbamoyl]-méthyl}-cyclohexyl)-benzamide
- acide trans-4-{4-[5-(3,5-difluoro-benzyl)-[1,3,4]thiadiazol-2-ylcarbamoyl]-phénoxy}-cyclohexanecarboxylique
- acide trans-{4-[4-(5-phénylméthanesulfinylméthyl-[1,3,4]thiadiazol-2-ylcarbamoyl)-phényl]-cyclohexyl}-acétique
- acide trans-{4-[4-(5-benzylsulfanylméthyl-[1,3,4]thiadiazol-2-ylcarbamoyl)-phényl]-cyclohexyl}-acétique
- acide trans- (4-{4-[5-(3-phényl-cyclobutyl)-[1,3,4]thiadiazol-2-ylcarbamoy]-phényl}-cyclohexyl)-acétique
- acide cis-4-[5-(5-cyclopentyloxyméthyl-[1,3,4]thiadiazol-2-ylcarbamoyl)-pyridin-2-yloxy]-cyclohexanecarboxylique
- acide trans-(4-(4-[5-(2-cyclopentylamino-éthyl)-[1,3,4]thiadiazol-2-ylcarbamoyl]-phényl}-cyclohexyl)-acétique
- acide cis-4-(4-{5-[2-(3-morpholin-4-yl-cyclopentyl)-éthyl]-[1,3,4]thiadiazol-2-ylcarbamoyl}-phénoxy)-cyclohexanecarboxylique
- acide cis-4-{4-[5-(3-oxo-3-phényl-propyl)-[1,3,4]thiadiazol-2-ylcarbamoyl]-phénoxy}-cyclohexanecarboxylique
- acide cis-4-{4-[5-(3,5-difluoro-benzyl)-[1,3,4]thiadiazol-2-ylcarbamoyl]-phénoxy}-cyclohexanecarboxylique.

13. Procédé de préparation d'un composé de formule (I) selon l'une quelconque des revendications 1 à 12, **caractérisé en ce que** l'on déprotège la fonction ester d'un composé choisi parmi
(i) un composé de formule M 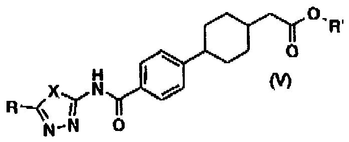
dans laquelle R représente un groupe -C(R1R2)n-Y et X, Y, R1, R2 et n sont tels que définis dans la revendication 1 et R' représente un groupe protecteur ;et
(ii) un composé de formule (XXIII) : 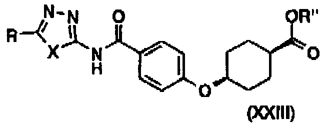
dans laquelle R représente un groupe -C(R1R2)n-Y et X, Y, R1, R2 et n sont tels que définis dans la revendication 1 et R" représente un groupe protecteur.

14. Procédé de préparation selon la revendication 13, **caractérisé en ce que** l'obtention du composé de formule (V), se fait par réaction (i) d'un composé de formule (II) : 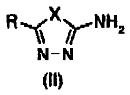
dans laquelle R représente un groupe -C(R1R2)n-Y et X, Y, R1, R2 et n sont tels que définis dans la revendication 1,
avec (ii) un composé de formule (III) 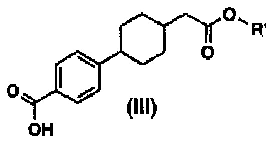
dans laquelle R' représente un groupe protecteur.

15. Procédé de préparation d'un composé de formule (I) selon selon la revendication 13, **caractérisé en ce que** l'obtention du composé de formule (XXIII), se fait par réaction (i) d'un composé de formule (II) : 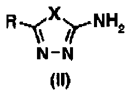
dans laquelle R représente un groupe -C(R1R2)n-Y et X, Y, R1, R2 et n sont tels que définis dans la revendication 1,
avec (ii) un composé de formule (XXI) 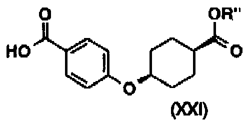
dans laquelle R" représente un groupe protecteur.

16. Composés de formule (III) : 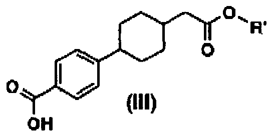
dans lesquelles R' représente un groupe protecteur.

17. Composés de formule (V) : 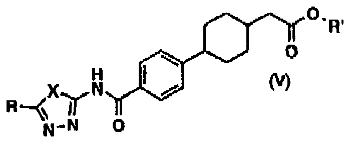
dans lesquelles R représente un groupe -C(R1R2)n-Y avec Y, R1, R2, n et X tels que définis dans la revendication 1 et R' représente un groupe protecteur.

18. Composés de formule (XXIII) : 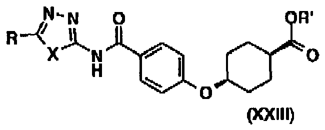
dans lesquelles R représente un groupe -C(R1R2)n-Y avec Y, R1, R2, n et X tels que définis dans la revendication 1 et R' représente un groupe protecteur.

19. Composés de formule (XXI) : 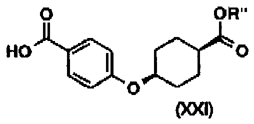
dans lesquelles R" représente un groupe protecteur, à l'exclusion du composé acide 4-(4-éthoxy-carbonyl-cyclohexyloxy)-benzoique.

20. Médicament, **caractérisé en ce qu'**il comprend un composé de formule (I) selon l'une quelconque des revendications 1 à 12, ou un sel d'addition de ce composé à un acide ou à une base pharmaceutiquement acceptable du composé de formule (I).

21. Composition pharmaceutique, **caractérisée en ce qu'**elle comprend un composé de formule (I) selon l'une quelconque des revendications 1 à 12, ou un sel pharmaceutiquement acceptable de ce composé, ainsi qu'au moins un excipient pharmaceutiquement acceptable.

22. Utilisation d'un composé de formule (I) selon l'une quelconque des revendications 1 à 12 pour la préparation d'un médicament destiné au traitement et/ou à la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique, des maladies coronariennes, de l'hypertension, des maladies de peau, de la maladie d'Alzheimer, des maladies immunomodulatrices, de l'infection à VIH, du syndrome inflammatoire de l'intestin, de certaines cancers, avantageusement pour la préparation d'un médicament destiné au traitement ou à la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique.

## Patentansprüche

1. Verbindung der Formel (I) 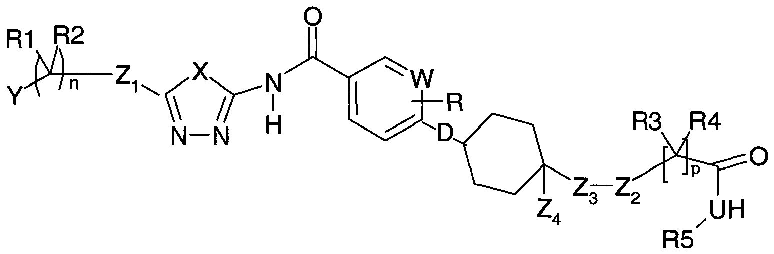 worin
• **U** für ein Sauerstoffatom oder ein Stickstoffatom steht, mit der Maßgabe, dass **R5** fehlt, wenn U für ein Sauerstoffatom steht;
• **n** gleich 1, 2 oder 3 ist;
• **p** gleich 0, 1 oder 2 ist;
• **D** für ein Sauerstoffatom, eine -NH-Gruppe oder eine Bindung steht;
• **W** für ein Kohlenstoff- oder Stickstoffatom steht;
• **X** für ein Heteroatom, das aus einem Sauerstoffatom oder einem Schwefelatom ausgewählt ist, steht;
• **R1, R2, R3** und **R4** unabhängig voneinander für
o ein Wasserstoffatom,
o eine -(C1-C6)-Alkylgruppe stehen oder
o (i) R1 und R2 mit dem Kohlenstoffatom, an das sie gebunden sind, eine -(C3-C10)-Cycloalkylgruppe bilden können und/oder (ii) R3 und R4 mit dem Kohlenstoffatom, an das sie gebunden sind, eine -(C3-C10)-Cycloalkylgruppe bilden können;
• **Y** für ein Wasserstoffatom, eine -(C1-C6)-Alkyl-, - (C3-C10)-Cycloalkyl-, (C3-C10)-Cycloalkyloxy-, (C3-C10)-Cycloalkyl-(C1-C6)-alkyloxy- oder Heterocycloalkyl-(C1-C6)-alkyloxygruppe, eine -COOR1-, Aryl-, Arylalkyl-, Heteroaryl-, Heterocycloalkyl-, Aryloxy-, -C(O)-Heterocycloalkyl-, -C(O)-Aryl, -CH(OH)-Aryl oder -NH-Cycloalkylgruppe steht, wobei diese Gruppen gegebenenfalls durch einen oder mehrere Substituenten, die aus einem Halogenatom, einer Hydroxylgruppe, einer (C1-C6)-Alkyl-, (C1-C6)-Alkoxy-Heterocycloalkyl oder Aryloxygruppe ausgewählt sind, substituiert sind;
• **R** für ein Wasserstoff- oder Halogenatom steht;
• **z1** fehlt oder für ein Schwefelatom, eine -NH- oder -NHC(O)-Funktion, eine -S(O)-CH₂-, -SCH₂-, Methylen-oder Ethylengruppe steht;
• **Z2** fehlt oder für eine Methylengruppe oder eine Gruppe 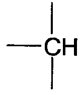 oder 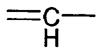 steht;
• **Z3** fehlt oder für ein Sauerstoffatom oder eine Methylengruppe oder eine Gruppe 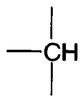 oder 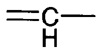 steht;
mit der Maßgabe, dass **Z2** nur für eine Gruppe 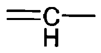 steht, wenn **Z3** vorhanden ist und für eine Gruppe 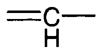 steht, und umgekehrt, wobei z**2** und **z3** somit eine Doppelbindung bilden;
mit der Maßgabe, dass **z2** und **z3,** wenn sie vorhanden sind, in einer Cycloalkylgruppe enthalten sein können;
• **z4** für
o ein Wasserstoffatom,
o ein Kohlenstoffatom, das gegebenenfalls mit Z3 eine -(C3-C10)-Cycloalkylgruppe bildet, wenn
z3 für eine Gruppe 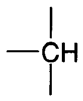 steht, steht oder
o fehlt, wobei Z3 dann eine Gruppe der Formel 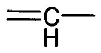 ist, die mit dem Cyclohexyl-Kohlenstoff, dem es benachbart ist, eine Doppelbindung bildet;
• **R5** für ein Wasserstoffatom oder eine Alkylgruppe, die gegebenenfalls durch mindestens eine Hydroxyl-, Heterocycloalkyl(C1-C6)-alkyl-, Amin- oder Alkyloxygruppe substituiert ist, steht,
und/oder
die Verbindung (I) in Säure-, Basen- oder Säure- oder Basenadditionssalzform vorliegt.

2. Verbindung der Formel (I) nach Anspruch 1, **dadurch gekennzeichnet, dass**:
• **U** für ein Sauerstoffatom oder ein Stickstoffatom steht, mit der Maßgabe, dass **R5** fehlt, wenn **U** für ein Sauerstoffatom steht;
und/oder
• **n** gleich 1, 2 oder 3 ist;
und/oder
• **p** gleich 0, 1 oder 2 ist;
und/oder
• **D** für ein Sauerstoffatom, eine -NH-Gruppe oder eine Bindung steht;
und/oder
• **W** für ein Kohlenstoff- oder Stickstoffatom steht;
und/oder
• **X** für ein Heteroatom, das aus einem Sauerstoffatom oder einem Schwefelatom ausgewählt ist, steht;
und/oder
• **R1, R2, R3** und **R4** unabhängig voneinander für
o ein Wasserstoffatom,
o eine -(C1-C6)-Alkylgruppe stehen oder
o (i) R1 und R2 mit dem Kohlenstoffatom, an das sie gebunden sind, eine -(C3-C10)-Cycloalkylgruppe bilden können und/oder (ii) R3 und R4 mit dem Kohlenstoffatom, an das sie gebunden sind, eine -(C3-C10)-Cycloalkylgruppe bilden können;
und/oder
• **Y** für ein Wasserstoffatom, eine -(C1-C6)-Alkyl-, - (C3-C10)-Cycloalkyl-, (C3-C10)-Cycloalkyloxy-, (C3-C10)-Cycloalkyl-(C1-C6)-alkyloxy- oder Heterocycloalkyl-(C1-C6)-alkyloxygruppe, eine -COOR1-, Aryl-, Arylalkyl-, Heteroaryl-, Heterocycloalkyl-, Aryloxy-, -C(O)-Heterocycloalkyl-, -C(O)-Aryl, -CH(OH)-Aryl oder -NH-Cycloalkylgruppe steht, wobei diese Gruppen gegebenenfalls durch einen oder mehrere Substituenten, die aus einem Halogenatom, einer Hydroxylgruppe, einer (C1-C6)-Alkyl-, (C1-C6)-Alkoxy-Heterocycloalkyl oder Aryloxygruppe ausgewählt sind, substituiert sind;
und/oder
• **R** für ein Wasserstoff- oder Halogenatom steht;
und/oder
• **Z1** fehlt oder für ein Schwefelatom, eine -NH- oder -NHC(O)-Funktion, eine -S(O)-CH₂-, -SCH₂-, Methylenoder Ethylengruppe steht;
und/oder
• **Z2** fehlt oder für eine Methylengruppe oder eine Gruppe 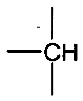 oder 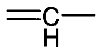 steht;
und/oder
• **Z3** fehlt oder für ein Sauerstoffatom oder eine Methylengruppe oder eine Gruppe 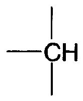 oder 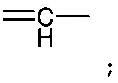 steht;
und/oder
• **Z2** und **Z3** vorhanden sind und jeweils für eine Gruppe der Formel 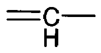 stehen können und somit eine Doppelbindung bilden;
und/oder
• **Z2** und **Z3** vorhanden sind und in einer Cycloalkylgruppe enthalten sein können;
und/oder
• **Z4** für
o ein Wasserstoffatom,
o ein Kohlenstoffatom, das gegebenenfalls mit Z3 eine -(C3-C10)-Cycloalkylgruppe bildet, wenn z3 für eine Gruppe 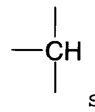 steht, steht oder
• fehlt, wobei Z3 dann eine Gruppe der Formel 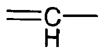 ist, die mit dem Cyclohexyl-Kohlenstoff, dem es benachbart ist, eine Doppelbindung bildet
und/oder
• R5 für ein Wasserstoffatom oder eine Alkylgruppe, die gegebenenfalls durch mindestens eine Hydroxyl-, Heterocycloalkyl(C1-C6)-alkyl-, Amin- oder Alkyloxygruppe substituiert ist, steht,
und/oder
die Verbindung (I) in Säure-, Basen- oder Säure- oder Basenadditionssalzform vorliegt.

3. Verbindung der Formel (I) nach Anspruch 1 oder 2, **dadurch gekennzeichnet, dass** es sich dabei um die Verbindung (I') der folgenden Formel handelt: 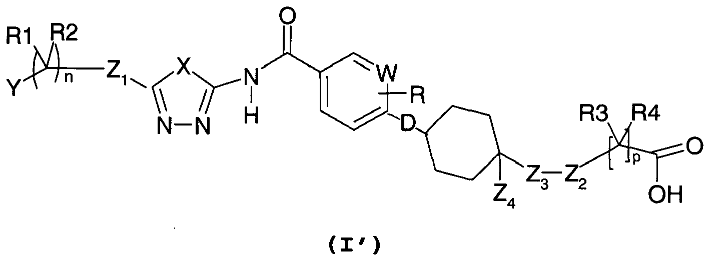
worin **Y, R1, R2, n**, **z1, X, W, R, D, Z4, Z3, Z2, R3, R4 und p** wie in einem der Ansprüche 1 oder 2 definiert sind.

4. Verbindung der Formel (I) nach Anspruch 1 oder 2, **dadurch gekennzeichnet, dass** es sich dabei um die Verbindung (I'') der folgenden Formel handelt: 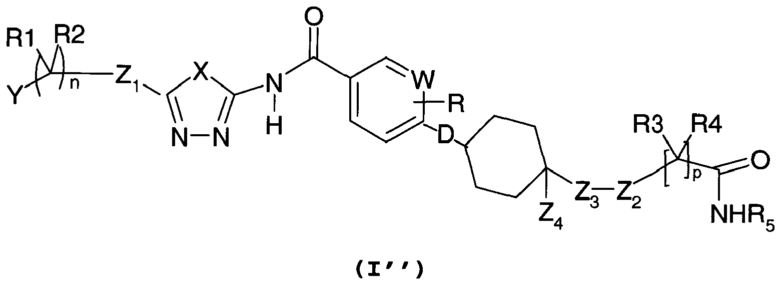
worin **Y, R1, R2, n**, **Z1, X, W, R, D, z4, Z3, Z2, R3, R4, p und R5** wie in einem der Ansprüche 1 oder 2 definiert sind.

5. Verbindung nach einem der Ansprüche 1 bis 4, **dadurch gekennzeichnet, dass D** für eine Bindung steht.

6. Verbindung nach einem der Ansprüche 1 bis 4, **dadurch gekennzeichnet, dass D** für ein Sauerstoffatom steht.

7. Verbindung nach einem der Ansprüche 1 bis 6, **dadurch gekennzeichnet, dass p** gleich 0 ist.

8. Verbindung nach einem der Ansprüche 1 bis 7, **dadurch gekennzeichnet, dass**:
• **Z3** und **Z2** jeweils für ein Methylen stehen oder
• **Z3** für ein Methylen steht und **Z2** fehlt oder
• **Z3** und **Z2** fehlen.

9. Verbindung nach einem der Ansprüche 1 bis 7, **dadurch gekennzeichnet, dass**:
• **Z3** und **Z2** in einer Cycloalkylgruppe, vorzugsweise Cyclopropyl, enthalten sind oder
• **Z3** und **Z2** zusammen eine Doppelbindung bilden.

10. Verbindung nach einem der Ansprüche 1 bis 9, **dadurch gekennzeichnet, dass W** für ein Kohlenstoffatom steht.

11. Verbindung nach einem der Ansprüche 1 bis 10, **dadurch gekennzeichnet, dass X** für ein Schwefelatom steht.

12. Verbindung nach einem der vorhergehenden Ansprüche, **dadurch gekennzeichnet, dass** es sich dabei um:
- {4-[4-(5-Benzyl[1,3,4]thiadiazol-2-ylcarbamoyl)phenyl]cyclohexyl}essigsäure
- (4-{4-[5-(4-Methylbenzyl)[1,3,4]thiadiazol-2-ylcarbamoyl]phenyl}cyclohexyl)essigsäure
- (4-{4-[5-(2-Fluorbenzyl)[1,3,4]thiadiazol-2-ylcarbamoyl]phenyl}cyclohexyl)essigsäure
- (4-{4-[5-(3-Fluorbenzyl)[1,3,4]thiadiazol-2-ylcarbamoyl]phenyl}cyclohexyl)essigsäure
- (4-{4-[5-(4-Fluorbenzyl)[1,3,4]thiadiazol-2-ylcarbamoyl]phenyl}cyclohexyl)essigsäure
- (4-{4-[5-(2,4,5-Trifluorbenzyl)[1,3,4]thiadiazol-2-ylcarbamoyl]phenyl}cyclohexyl)essigsäure
- [4-(4-{5-[1-(Phenyl)cyclopropyl][1,3,4]thiadiazol-2-ylcarbamoyl}phenyl)cyclohexyl]essigsäure
- [4-(4-{5-[1-(4-Fluorphenyl)cyclopropyl][1,3,4]-thiadiazol-2-ylcarbamoyl}phenyl)cyclohexyl]essigsäure
- [4-(4-{5-[1-(3-Fluorphenyl)cyclobutyl][1,3,4]thia-diazol-2-ylcarbamoyl}phenyl)cyclohexyl]essigsäure
- (4-{4-[5-(4-Chlorbenzyl)[1,3,4]thiadiazol-2-ylcarbamoyl]phenyl}cyclohexyl)essigsäure
- (4-{4-[5-(3-Chlorbenzyl)[1,3,4]thiadiazol-2-ylcarbamoyl]phenyl}cyclohexyl)essigsäure
- (4-{4-[5-(2-Chlorbenzyl)[1,3,4]thiadiazol-2-ylcarbamoyl]phenyl}cyclohexyl)essigsäure
- (4-{4-[5-(4-Methoxybenzyl)[1,3,4]thiadiazol-2-ylcarbamoyl]phenyl}cyclohexyl)essigsäure
- {4-[4-(5-tert.-Butyl[1,3,4]thiadiazol-2-ylcarbamoyl)phenyl]cyclohexyl}essigsäure
- {4-[4-(5-Cyclopentylmethyl[1,3,4]thiadiazol-2-ylcarbamoyl)phenyl]cyclohexyl}essigsäure
- (4-{4-[5-(2-Cyclopentylethyl)[1,3,4]thiadiazol-2-ylcarbamoyl]phenyl}cyclohexyl)essigsäure
- {4-[4-(5-Isobutyl[1,3,4]thiadiazol-2-ylcarbamoyl)phenyl]cyclohexyl}essigsäure
- {4-[4-(5-Phenethyl[1,3,4]thiadiazol-2-ylcarbamoyl)phenyl]cyclohexyl}essigsäure
- [4-(4-{5-[2-(4-Fluorphenyl)ethyl][1,3,4]thiadiazol-2-ylcarbamoyl}phenyl)cyclohexyl]essigsäure
- {4-[4-(5-Phenoxymethyl[1,3,4]thiadiazol-2-ylcarbamoyl)phenyl]cyclohexyl}essigsäure
- [4-(4-{5-[3-(4-Fluorphenyl)propyl][1,3,4]thiadiazol-2-ylcarbamoyl}phenyl)cyclohexyl]essigsäure
- (4-{4-[5-(4-Fluorphenoxymethyl)[1,3,4]thiadiazol-2-ylcarbamoyl]phenyl}cyclohexyl)essigsäure
- (4-{4-[5-(Benzyl)[1,3,4]oxadiazol-2-ylcarbamoyl]phenyl}cyclohexyl)essigsäure
- (4-{4-[5-(4-Fluorbenzyl)[1,3,4]oxadiazol-2-ylcarbamoyl]phenyl}cyclohexyl)essigsäure
- cis-4-[4-(5-Benzyl)[1,3,4]thiadiazol-2-ylcarbamoyl)phenoxy]cyclohexancarbonsäure
- trans-4-[4-(5-Benzyl[1,3,4]thiadiazol-2-ylcarbamoyl)phenoxy]cyclohexancarbonsäure
- cis-4-[4-(5-Benzyl[1,3,4]thiadiazol-2-ylcarbamoyl)-2-fluorphenoxy]cyclohexancarbonsäure
- cis-4-[4-(5-Benzyl[1,3,4]thiadiazol-2-ylcarbamoyl)-2-chlorphenoxy]cyclohexancarbonsäure
- cis-4-{4-[5-(2-Cyclopentylethyl)[1,3,4]thiadiazol-2-ylcarbamoyl]phenoxy}cyclohexancarbonsäure
- cis-4-[5-(5-Benzyl[1,3,4]thiadiazol-2-ylcarbamoyl)pyridin-2-yloxy]cyclohexancarbonsäure
- cis-4-{5-[5-(3-Chlorbenzyl)[1,3,4]thiadiazol-2-ylcarbamoyl]pyridin-2-yloxy}cyclohexancarbonsäure
- cis-4-[5-(5-Phenethyl[1,3,4]thiadiazol-2-ylcarbamoyl)pyridin-2-yloxy]cyclohexancarbonsäure
- cis-4-[4-(5-Benzyl[1,3,4]thiadiazol-2-ylcarbamoyl)phenylamino]cyclohexancarbonsäure
- trans-4-[4-(5-Benzyl[1,3,4]thiadiazol-2-ylcarbamoyl)phenylamino]cyclohexancarbonsäure
- trans-{4-[4-(5-Benzyl[1,3,4]thiadiazol-2-ylcarbamoyl)phenyl]cyclohexyl}essigsäure
- trans-4-[4-(5-Benzyl[1,3,4]thiadiazol-2-ylcarbamoyl)phenyl]cyclohexancarbonsäure
- trans-{4-[4-(5-Cyclopentyloxymethyl[1,3,4]thiadiazol-2-ylcarbamoyl)phenyl]cyclohexyl}essigsäure
- trans-[4-(4-{5-[2-(Tetrahydrofuran-2-yl)ethyl][1,3,4]thiadiazol-2-ylcarbamoyl}phenyl)cyclo-hexyl]essigsäure
- trans-(4-{4-[5-(2-Cyclohexylethyl)[1,3,4]thiadiazol-2-ylcarbamoyl]phenyl}cyclohexyl)essigsäure
- trans-{4-[4-(5-Cyclopentylmethoxymethyl[1,3,4]-thiadiazol-2-ylcarbamoyl)phenyl]cyclohexyl}essigsäure
- trans-{4-[4-(5-Benzylsulfanyl[1,3,4]thiadiazol-2-ylcarbamoyl)phenyl]cyclohexyl}essigsäure
- {4-[4-(5-Benzyl[1,3,4]thiadiazol-2-ylcarbamoyl)phenyl]cyclohexyliden}essigsäure
- 6-[4-(5-Benzyl[1,3,4]thiadiazol-2-ylcarbamoyl)phenyl]spiro[2.5]octan-1-carbonsäure
- (E)-3-{4-[4-(5-Benzyl[1,3,4]thiadiazol-2-ylcarbamoyl)phenyl]cyclohexyl}acrylsäure
- trans-(1R,2S/1S,2R)-2-{4-[4-(5-Benzyl[1,3,4]thiadiazol-2-ylcarbamoyl)phenyl]cyclohexyl}cyclopropancarbonsäure
- trans-3-{4-[4-(5-Benzyl[1,3,4]thiadiazol-2-ylcarbamoyl)phenyl]cyclohexyl}propionsäure
- (4-{4-[5-((1S,3S/1R,3R)-3-Phenoxycyclohexyl)[1,3,4]thiadiazol-2-ylcarbamoyl]phenyl}cyclohexyl)essigsäure
- trans-(4-{4-[(5-{[(3R,6S/3S,6R)-5-Ethoxyoctahydropentalen-2-yl]methyl}-1,3,4-thiadiazol-2-yl)carbamoyl]phenyl}cyclohexyl)essigsäure
- trans-{4-[4-(5-Benzyl[1,3,4]thiadiazol-2-ylcarbamoyl)phenyl]cyclohexyloxy}essigsäure
- trans-(4-{4-[5-(2-Morpholin-4-ylethyl)[1,3,4]thiadiazol-2-ylcarbamoyl]phenyl}cyclohexyl)essigsäure
- trans-(4-{4-[5-(2-Oxo-2-pyrrolidin-1-ylethyl)[1,3,4]thiadiazol-2-ylcarbamoyl]phenyl}cyclohexyl)essigsäure
- cis-4-[4-(5-Cyclopentyloxymethyl[1,3,4]thiadiazol-2-ylcarbamoyl)phenoxy]cyclohexancarbonsäure
- trans-[4-(4-{5-[2-(Tetrahydrofuran-3-yl)ethyl][1,3,4]thiadiazol-2-ylcarbamoyl}phenyl)cyclohexyl]essigsäure
- trans-(4-{4-[5-(3-Phenylcyclobutyl)[1,3,4]thiadiazol-2-ylcarbamoyl]phenyl}cyclohexyl)essigsäure
- {4-[4-(5-Phenylacetylamino[1,3,4]thiadiazol-2-ylcarbamoyl)phenyl]cyclohexyl}essigsäure
- trans-{4-[4-(5-Cyclopentyloxymethyl[1,3,4]thiadiazol-2-ylcarbamoyl)phenyl]cyclohexyl}essigsäure
- trans-(4-{4-[5-(3,5-Difluorbenzyl)[1,3,4]thiadiazol-2-ylcarbamoyl]phenyl}cyclohexyl)essigsäure
- trans-(4-{4-[5-(4-Hydroxycyclohexylmethyl)[1,3,4]-thiadiazol-2-ylcarbamoyl]phenyl}cyclohexyl)essigsäure
- trans-(4-{4-[5-(Tetrahydrofuran-2-ylmethoxymethyl)[1,3,4]thiadiazol-2-ylcarbamoyl]phenyl}cyclohexyl)essigsäure
- trans-{4-[4-(5-Benzyl[1,3,4]thiadiazol-2-ylcarbamoyl)phenoxy]cyclohexyl}essigsäure
- trans-4-{4-[5-(Tetrahydrofuran-2-ylmethoxymethyl)[1,3,4]thiadiazol-2-ylcarbamoyl]phenoxy}cyclohexancarbonsäure
- trans-(4-{4-[5-(3-Oxo-3-phenylpropyl)[1,3,4]thiadiazol-2-ylcarbamoyl]phenyl}cyclohexyl)essigsäure
- trans-(4-{4-[5-(3-Hydroxy-3-phenylpropyl)[1,3,4]thiadiazol-2-ylcarbamoyl]phenyl}cyclohexyl)essigsäure
- trans-N-(5-Benzyl[1,3,4]thiadiazol-2-yl)-4-{4-[(2-hydroxy-2-methylpropylcarbamoyl)methyl]cyclohexyl}benzamid
- trans-N-(5-Benzyl[1,3,4]thiadiazol-2-yl)-4-(4-carbamoylmethylcyclohexyl)benzamid
- trans-N-(5-Benzyl[1,3,4]thiadiazol-2-yl)-4-{4-[(2,3-dihydroxypropylcarbamoyl)methyl]cyclohexyl}benzamid
- trans-N-(5-Benzyl[1,3,4]thiadiazol-2-yl)-4-{4-[(2-morpholin-4-ylethylcarbamoyl)methyl]cyclohexyl}benzamid
- trans-N-(5-Benzyl[1,3,4]thiadiazol-2-yl)-4-{4-[(2-dimethylaminoethylcarbamoyl)methyl]cyclohexyl}benzamid
- trans-N-(5-Benzyl[1,3,4]thiadiazol-2-yl)-4-{4-[(2-methoxyethylcarbamoyl)methyl]cyclohexyl}benzamid
- trans-N-(5-Benzyl[1,3,4]thiadiazol-2-yl)-4-(4-{[([1,4]dioxan-2-ylmethyl)carbamoyl]methyl}cyclo-hexyl)benzamid
- trans-4-{4-[5-(3,5-Difluorbenzyl)[1,3,4]thiadiazol-2-ylcarbamoyl]phenoxy}cyclohexancarbonsäure
- trans-{4-[4-(5-Phenylmethansulfinylmethyl[1,3,4]-thiadiazol-2-ylcarbamoyl)phenyl]cyclohexyl}essigsäure
- trans-{4-[4-(5-Benzylsulfanylmethyl[1,3,4]thiadiazol-2-ylcarbamoyl)phenyl]cyclohexyl}essigsäure
- trans-(4-{4-[5-(3-Phenylcyclobutyl)[1,3,4]thiadiazol-2-ylcarbamoyl]phenyl}cyclohexyl)essigsäure
- cis-4-[5-(5-Cyclopentyloxymethyl[1,3,4]thiadiazol-2-ylcarbamoyl)pyridin-2-yloxy]cyclohexancarbonsäure
- trans-(4-{4-[5-(2-Cyclopentylaminoethyl)[1,3,4]-thiadiazol-2-ylcarbamoyl]phenyl}cyclohexyl)essigsäure
- cis-4-(4-{5-[2-(3-Morpholin-4-ylcyclopentyl)ethyl][1,3,4]thiadiazol-2-ylcarbamoyl}phenoxy)cyclohexancarbonsäure
- cis-4-{4-[5-(3-Oxo-3-phenylpropyl)[1,3,4]thiadiazol-2-ylcarbamoyl]phenoxy}cyclohexancarbonsäure
- cis-4-{4-[5-(3,5-Difluorbenzyl)[1,3,4]thiadiazol-2-ylcarbamoyl]phenoxy}cyclohexancarbonsäure handelt.

13. Verfahren zur Herstellung einer Verbindung der Formel (I) nach einem der Ansprüche 1 bis 12, **dadurch gekennzeichnet, dass** man die Esterfunktion einer aus
(i) einer Verbindung der Formel (V) 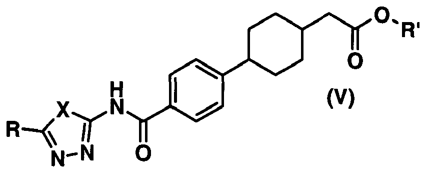
worin R für eine -C(R1R2)n-Y-Gruppe steht und X, Y, R1, R2 und n wie in Anspruch 1 definiert sind und R' für eine Schutzgruppe steht; und
(ii) einer Verbindung der Formel (XXIII) : 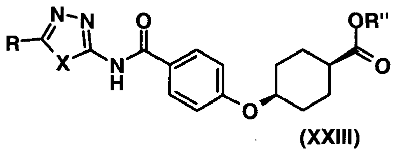
worin R für eine -C(R1R2)n-Y-Gruppe steht und X, Y, R1, R2 und n wie in Anspruch 1 definiert sind und R'' für eine Schutzgruppe steht,
ausgewählten Verbindung entschützt.

14. Herstellungsverfahren nach Anspruch 13, **dadurch gekennzeichnet, dass** man die Verbindung der Formel (V) durch Umsetzung von (i) einer Verbindung der Formel (II) : 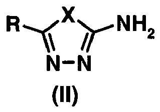
worin R für eine -C(R1R2)n-Y-Gruppe steht und X, Y, R1, R2 und n wie in Anspruch 1 definiert sind,
mit (ii) einer Verbindung der Formel (III) 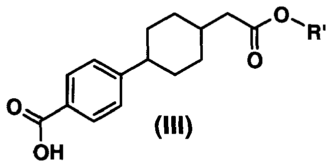
worin R' für eine Schutzgruppe steht,
erhält.

15. Verfahren zur Herstellung einer Verbindung der Formel (I) nach Anspruch 13, **dadurch gekennzeichnet, dass** man die Verbindung der Formel (XXIII) durch Umsetzung von (i) einer Verbindung der Formel (II) : 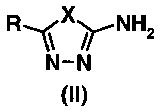
worin R für eine -C(R1R2)n-Y-Gruppe steht und X, Y, R1, R2 und n wie in Anspruch 1 definiert sind,
mit (ii) einer Verbindung der Formel (XXI) 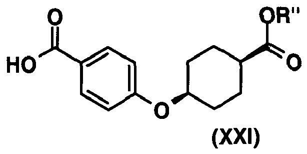
worin R'' für eine Schutzgruppe steht, erhält.

16. Verbindungen der Formel (III) : 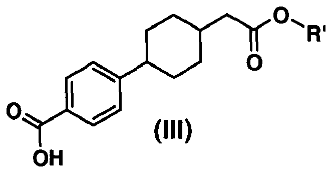
worin R' für eine Schutzgruppe steht.

17. Verbindungen der Formel (V): 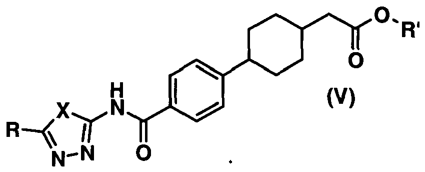
worin R für eine -C(R1R2)n-Y-Gruppe steht, wobei Y, R1, R2, n und X wie in Anspruch 1 definiert sind, und R' für eine Schutzgruppe steht.

18. Verbindungen der Formel (XXIII) : 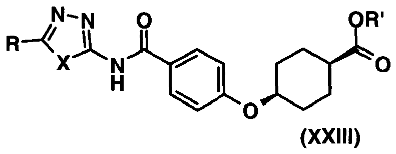
worin R für eine -C(R1R2)n-Y-Gruppe steht, wobei Y, R1, R2, n und X wie in Anspruch 1 definiert sind, und R' für eine Schutzgruppe steht.

19. Verbindungen der Formel (XXI) : 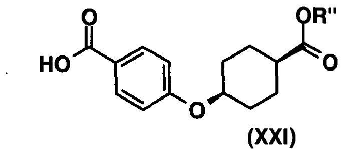
worin R'' für eine Schutzgruppe steht, mit Ausnahme der Verbindung 4-(4-Ethoxycarbonylcyclohexyloxy)benzoesäure.

20. Medikament, **dadurch gekennzeichnet, dass** es eine Verbindung der Formel (I) nach einem der Ansprüche 1 bis 12 oder ein Additionssalz dieser Verbindung mit einer pharmazeutisch unbedenklichen Säure oder Base der Verbindung der Formel (I) umfasst.

21. Pharmazeutische Zusammensetzung, **dadurch gekennzeichnet, dass** sie eine Verbindung der Formel (I) nach einem der Ansprüche 1 bis 12 oder ein pharmazeutisch unbedenkliches Salz dieser Verbindung sowie mindestens einen pharmazeutisch unbedenklichen Hilfsstoff umfasst.

22. Verwendung einer Verbindung der Formel (I) nach einem der Ansprüche 1 bis 12 zur Herstellung eines Arzneimittels zur Behandlung und/oder Prävention von Obesitas, Dyslipidämie, Leiden mit gestörter Nüchternglukose, metabolischer Azidose, Ketose, Lebersteatose, Insulinresistenz, Typ-II-Diabetes und sich daraus ergebenden Komplikationen, Lipotoxizität, Akkumulation und Überschuss von Triacylglyceriden im Fettgewebe (WAT), metabolischem Syndrom, Koronarerkrankungen, Hypertonie, Hauterkrankungen, Alzheimer-Krankheit, immunmodulatorischen Erkrankungen, HIV-Infektion, Reizkolon, bestimmten Krebserkrankungen, vorteilhafterweise zur Herstellung eines Medikaments zur Behandlung oder Prävention von Obesitas, Dyslipidämie, Leiden mit gestörter Nüchternglukose, metabolischer Azidose, Ketose, Lebersteatose, Insulinresistenz, Typ-II-Diabetes und sich daraus ergebenden Komplikationen, Lipotoxizität, der Akkumulation und dem Überschuss von Triacylglyceriden im Fettgewebe (WAT), metabolischem Syndrom.

## Claims

1. Compound corresponding to formula (I) 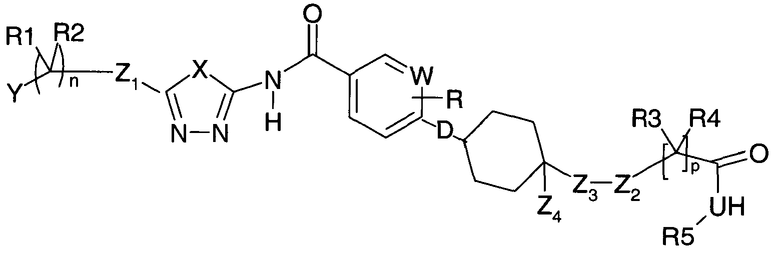 in which
• **U** represents an oxygen atom or a nitrogen atom, given that when **U** represents an oxygen atom, then **R5** is absent;
• **n** is equal to 0, 1, 2 or 3;
• **p** is equal to 0, 1 or 2;
• **D** represents an oxygen atom, a group -NH- or a bond;
• **W** represents a carbon or nitrogen atom;
• **X** represents a heteroatom chosen from an oxygen atom and a sulfur atom;
• **R1, R2, R3** and **R4** represent, independently of each other,
o a hydrogen atom,
o a group -(C1-C6)alkyl, or alternatively,
o (i) R1 and R2 may form, with the carbon atom to which they are attached, a group -(C3-C10)cycloalkyl- and/or (ii) R3 and R4 may form, with the carbon atom to which they are attached, a group -(C3-C10)cycloalkyl-;
• **Y** represents a hydrogen atom, a group -(C1-C6)alkyl, -(C3-C10)cycloalkyl-, (C3-C10)cycloalkyloxy-, (C3-C10)cycloalkyl-(C1-C6)alkyloxy-, heterocycloalkyl-(C1'-C6)alkyloxy-, a group -COOR1, aryl, arylalkyl, heteroaryl, heterocycloalkyl, aryloxy, -C(O)-heterocycloalkyl, -C(O)aryl, -CH(OH)aryl or -NH-cycloalkyl, the said groups being optionally substituted with one or more substituents chosen from a halogen atom, a hydroxyl group, a group (C1-C6)alkyl, (C1-C6)alkoxy or heterocycloalkyl or an aryloxy group;
• **R** represents a hydrogen or halogen atom;
• **Z1** is absent or represents a sulfur atom, a function -NH- or -NHC(O)-, a group -S(O)-CH₂-, -SCH₂-, methylene or an ethylene group;
• **Z2** is absent or represents a methylene group, a group 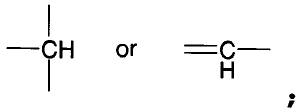
• **Z3** is absent or represents an oxygen atom or a methylene group, a group 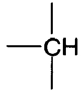 or 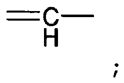 Given that **Z2** represents a group 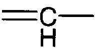 only when **Z3** is present and that it represents a group 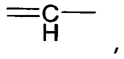 and vice versa, **Z2** and **Z3** thus forming a double bond;
Given that **Z2** and **Z3,** when they are present, may be included in a cycloalkyl group;
• **Z4** is
○ a hydrogen atom,
○ a carbon atom optionally forming with Z3 a group -(C3-C10)cycloalkyl- when Z3 is a group 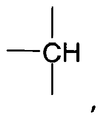 or
○ is absent, z3 then being a group 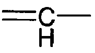 forming a double bond with the cyclohexyl carbon adjacent thereto;
• **R5** represents a hydrogen atom or an alkyl group optionally substituted with at least one hydroxyl, heterocycloalkyl(C1-C6)alkyl, amine or alkyloxy group, in the form of the acid or the base or of an addition salt with an acid or with a base.

2. Compound of formula (I) according to Claim 1, **characterized in that**:
• **U** represents an oxygen atom or a nitrogen atom, given that when **U** represents an oxygen atom, then **R5** is absent;
and/or
• **n** is equal to 0, 1, 2 or 3;
and/or
• **p** is equal to 0, 1 or 2;
and/or
• **D** represents an oxygen atom, a group -NH- or a bond;
and/or
• **W** represents a carbon or nitrogen atom;
and/or
• **X** represents a heteroatom chosen from an oxygen atom and a sulfur atom;
and/or
• **R1, R2, R3** and **R4** represent, independently of each other,
o a hydrogen atom,
o a group -(C1-C6)alkyl, or alternatively,
o (i) R1 and R2 may form, with the carbon atom to which they are attached, a group -(C3-C10)cycloalkyl- and/or (ii) R3 and R4 may form, with the carbon atom to which they are attached, a group -(C3-C10)cycloalkyl-;
and/or
• **Y** represents a hydrogen atom, a group -(C1-C6)alkyl, -(C3-C10)cycloalkyl-, (C3-C10)cycloalkyloxy-, (C3-C10)cycloalkyl-(C1-C6)alkyloxy-, heterocycloalkyl-(C1-C6)alkyloxy-, a group -COOR1, aryl, arylalkyl, heteroaryl, heterocycloalkyl, aryloxy, -C(O)-heterocycloalkyl, -C(O)aryl, -CH(OH)aryl or -NH-cycloalkyl, the said groups being optionally substituted with one or more substituents chosen from a halogen atom, a hydroxyl group, a group (C1-C6)alkyl, (C1-C6)alkoxy or heterocycloalkyl or an aryloxy group; and/or
• **R** represents a hydrogen or halogen atom;
and/or
• **Z1** is absent or represents a sulfur atom, a function -NH- or -NHC(O)-, a group -S(O)-CH₂-, -SCH₂-, methylene or an ethylene group;
and/or
• **Z2** is absent or represents a methylene group, a group 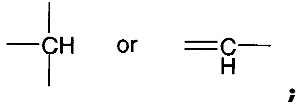 and/or
• **Z3** is absent or represents an oxygen atom or a methylene group, a group 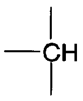 or 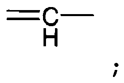 and/or
• **Z2** and **Z3** are present and may each represent a group 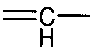 and thus form a double bond;
and/or
• **Z2** and **Z3** are present and may be included in a cycloalkyl group;
and/or
• **Z4** is
○ a hydrogen atom,
○ a carbon atom optionally forming with Z3 a group -(C3-C10)cycloalkyl- when Z3 is a group 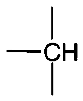 or
○ is absent, Z3 being a group 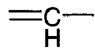 forming a double bond with the cyclohexyl carbon adjacent thereto;
and/or
• **R5** represents a hydrogen atom or an alkyl group optionally substituted with at least one hydroxyl, heterocycloalkyl(C1-C6)alkyl, amine or alkyloxy group, and/or
• the said compound (I) is in the form of the acid or the base or of an addition salt with an acid or with a base.

3. Compound of formula (I) according to Claim 1 or 2, **characterized in that** it is compound (I') having the following formula: 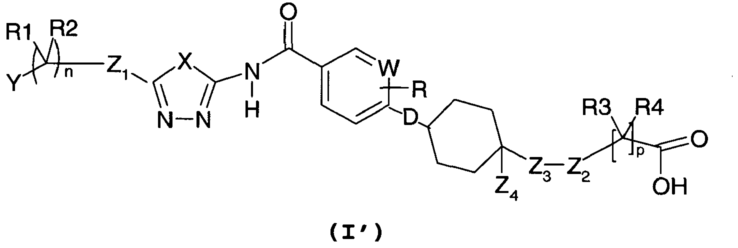
in which **Y, R1, R2, n**, **Z1, X, W, R, D, Z4, Z3, Z2, R3, R4** and **p** are as defined in either of Claims 1 and **2.**

4. Compound of formula (I) according to Claim 1 or 2, **characterized in that** it is compound (I'') having the following formula: 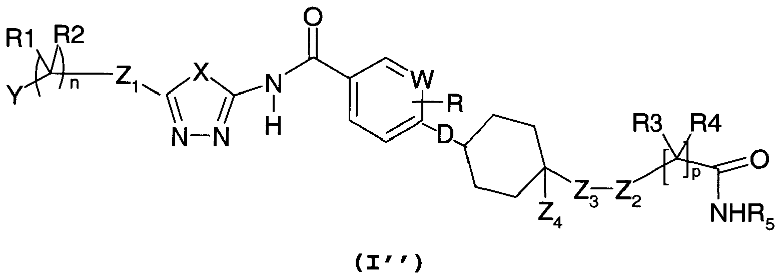
in which **Y, R1, R2, n**, **z1, X, W, R, D, Z4, Z3, Z2, R3, R4, p** and **R5** are as defined in either of Claims 1 and 2.

5. Compound according to any one of Claims 1 to 4, **characterized in that D** represents a bond.

6. Compound according to any one of Claims 1 to 4, **characterized in that D** represents an oxygen atom.

7. Compound according to any one of Claims 1 to 6, **characterized in that p** is equal to 0.

8. Compound according to any one of Claims 1 to 7, **characterized in that**:
• **Z3** and **Z2** each represents a methylene, or
• **Z3** represents a methylene and **Z2** is absent, or
• **Z3** and **Z2** are absent.

9. Compound according to any one of Claims 1 to 7, **characterized in that**:
• **Z3** and **Z2** are included in a cycloalkyl group, advantageously a cyclopropyl, or
• **Z3** and **Z2** together form a double bond.

10. Compound according to any one of Claims 1 to 9, **characterized in that W** represents a carbon atom.

11. Compound according to any one of Claims 1 to 10, **characterized in that X** represents a sulfur atom.

12. Compound according to one of the preceding claims, **characterized in that** it is:
- {4-[4-(5-benzyl[1,3,4]thiadiazol-2-ylcarbamoyl)-phenyl]cyclohexyl}acetic acid
- (4-{4-[5-(4-methylbenzyl)[1,3,4]thiadiazol-2-ylcarbamoyl]phenyl}cyclohexyl)acetic acid
- (4-{4-[5-(2-fluorobenzyl)[1,3,4]thiadiazol-2-ylcarbamoyl]phenyl}cyclohexyl)acetic acid
- (4-{4-[5-(3-fluorobenzyl)[1,3,4]thiadiazol-2-ylcarbamoyl]phenyl}cyclohexyl)acetic acid
- (4-{4-[5-(4-fluorobenzyl)[1,3,4]thiadiazol-2-ylcarbamoyl]phenyl}cyclohexyl)acetic acid
- (4-{4-[5-(2,4,5-trifluorobenzyl)[1,3,4]thiadiazol-2-ylcarbamoyl]phenyl}cyclohexyl)acetic acid
- [4-(4-{5-[1-(phenyl)cyclopropyl][1,3,4]thiadiazol-2-ylcarbamoyl}phenyl)cyclohexyl]acetic acid
- [4-(4-{5-[1-(4-fluorophenyl)cyclopropyl][1,3,4]thia-diazol-2-ylcarbamoyl}phenyl)cyclohexyl]acetic acid
- [4-(4-{5-[1-(3-fluorophenyl)cyclobutyl][1,3,4]thia-diazol-2-ylcarbamoyl}phenyl)cyclohexyl]acetic acid
- (4-{4-[5-(4-chlorobenzyl)[1,3,4]thiadiazol-2-ylcarbamoyl]phenyl}cyclohexyl)acetic acid
- (4-{4-[5-(3-chlorobenzyl)[1,3,4]thiadiazol-2-ylcarbamoyl]phenyl}cyclohexyl)acetic acid
- (4-{4-[5-(2-chlorobenzyl)[1,3,4]thiadiazol-2-ylcarbamoyl]phenyl}cyclohexyl)acetic acid
- (4-{4-[5-(4-methoxybenzyl)[1,3,4]thiadiazol-2-ylcarbamoyl]phenyl}cyclohexyl)acetic acid
- {4-[4-(5-tert-butyl[1,3,4]thiadiazol-2-ylcarbamoyl)-phenyl]cyclohexyl}acetic acid
- {4-[4-(5-cyclopentylmethyl[1,3,4]thiadiazol-2-ylcarbamoyl)phenyl]cyclohexyl}acetic acid
- (4-{4-[5-(2-cyclopentylethyl)[1,3,4]thiadiazol-2-ylcarbamoyl]phenyl}cyclohexyl)acetic acid
- {4-[4-(5-isobutyl[1,3,4]thiadiazol-2-ylcarbamoyl)-phenyl]cyclohexyl}acetic acid
- {4-[4-(5-phenethyl[1,3,4]thiadiazol-2-ylcarbamoyl)-phenyl]cyclohexyl}acetic acid
- [4-(4-{5-[2-(4-fluorophenyl)ethyl][1,3,4]thiadiazol-2-ylcarbamoyl}phenyl)cyclohexyl]acetic acid
- {4-[4-(5-phenoxymethyl[1,3,4]thiadiazol-2-ylcarbamoyl)phenyl]cyclohexyl}acetic acid
- [4-(4-{5-[3-(4-fluorophenyl)propyl][1,3,4]thiadiazol-2-ylcarbamoyl}phenyl)cyclohexyl]acetic acid
- (4-{4-[5-(4-fluorophenoxymethyl)[1,3,4]thiadiazol-2-ylcarbamoyl]phenyl}cyclohexyl)acetic acid
- (4-{4-[5-(benzyl)[1,3,4]oxadiazol-2-ylcarbamoyl]-phenyl}cyclohexyl)acetic acid
- (4-{4-[5-(4-fluorobenzyl)[1,3,4]oxadiazol-2-ylcarbamoyl]phenyl}cyclohexyl)acetic acid
- cis-4-[4-(5-benzyl[1,3,4]thiadiazol-2-ylcarbamoyl)-phenoxy]cyclohexanecarboxylic acid
- trans-4-[4-(5-benzyl[1,3,4]thiadiazol-2-ylcarbamoyl)-phenoxy]cyclohexanecarboxylic acid
- cis-4-[4-(5-benzyl[1,3,4]thiadiazol-2-ylcarbamoyl)-2-fluorophenoxy]cyclohexanecarboxylic acid
- cis-4-[4-(5-benzyl[1,3,4]thiadiazol-2-ylcarbamoyl)-2-chlorophenoxy]cyclohexanecarboxylic acid
- cis-4-{4-[5-(2-cyclopentylethyl)[1,3,4]thiadiazol-2-ylcarbamoyl]phenoxy}cyclohexanecarboxylic acid
- cis-4-[5-(5-benzyl[1,3,4]thiadiazol-2-ylcarbamoyl)-pyridin-2-yloxy]cyclohexanecarboxylic acid
- cis-4-{5-[5-(3-chlorobenzyl)[1,3,4]thiadiazol-2-ylcarbamoyl]pyridin-2-yloxy}cyclohexanecarboxylic acid
- cis-4-[5-(5-phenethyl[1,3,4]thiadiazol-2-ylcarbamoyl)pyridin-2-yloxy]cyclohexanecarboxylic acid
- cis-4-[4-(5-benzyl[1,3,4]thiadiazol-2-ylcarbamoyl)-phenylamino]cyclohexanecarboxylic acid
- trans-4-[4-(5-benzyl[1,3,4]thiadiazol-2-ylcarbamoyl)-phenylamino]cyclohexanecarboxylic acid
- trans-{4-[4-(5-benzyl[1,3,4]thiadiazol-2-ylcarbamoyl)phenyl]cyclohexyl}acetic acid
- trans-4-[4-(5-benzyl[1,3,4]thiadiazol-2-ylcarbamoyl)-phenyl]cyclohexanecarboxylic acid
- trans-{4-[4-(5-cyclopentyloxymethyl[1,3,4]thiadiazol-2-ylcarbamoyl)phenyl]cyclohexyl}acetic acid
- trans-[4-(4-{5-[2-(tetrahydrofuran-2-yl)ethyl]-[1,3,4]thiadiazol-2-ylcarbamoyl}phenyl)cyclohexyl]-acetic acid
- trans-(4-{4-[5-(2-cyclohexylethyl)[1,3,4]thiadiazol-2-ylcarbamoyl]phenyl}cyclohexyl)acetic acid
- trans-{4-[4-(5-cyclopentylmethoxymethyl[1,3,4]thia-diazol-2-ylcarbamoyl)phenyl]cyclohexyl}acetic acid
- trans-{4-[4-(5-benzylsulfanyl[1,3,4]thiadiazol-2-ylcarbamoyl)phenyl]cyclohexyl}acetic acid
- {4-[4-(5-benzyl[1,3,4]thiadiazol-2-ylcarbamoyl)-phenyl]cyclohexylidene}acetic acid
- 6-[4-(5-benzyl[1,3,4]thiadiazol-2-ylcarbamoyl)-phenyl]spiro[2.5]octane-1-carboxylic acid
- (E)-3-{4-[4-(5-benzyl[1,3,4]thiadiazol-2-ylcarbamoyl)phenyl]cyclohexyl}acrylic acid
- trans-(1R,2S/1S,2R)-2-{4-[4-(5-benzyl[1,3,4]thia-diazol-2-ylcarbamoyl)phenyl]cyclohexyl}cyclopropane-carboxylic acid
- trans-3-{4-[4-(5-benzyl[1,3,4]thiadiazol-2-ylcarbamoyl)phenyl]cyclohexyl}propionic acid
- (4-{4-[5-((1S,3S/1R,3R)-3-phenoxycyclohexyl)-[1,3,4]thiadiazol-2-ylcarbamoyl]phenyl}cyclohexyl)-acetic acid
- trans-(4-{4-[(5-{[(3R,6S/3S,6R)-5-ethoxyoctahydropentalen-2-yl]methyl}-1,3,4-thiadiazol-2-yl)carbamoyl]phenyl}cyclohexyl)acetic acid
- trans-{4-[4-(5-benzyl[1,3,4]thiadiazol-2-ylcarbamoyl)phenyl]cyclohexyloxy}acetic acid
- trans-(4-{4-[5-(2-morpholin-4-ylethyl)[1,3,4]thia-diazol-2-ylcarbamoyl]phenyl}cyclohexyl)acetic acid
- trans-(4-{4-[5-(2-oxo-2-pyrrolidin-1-ylethyl)-[1,3,4]thiadiazol-2-ylcarbamoyl]phenyl}cyclohexyl)-acetic acid
- cis-4-[4-(5-cyclopentyloxymethyl[1,3,4]thiadiazol-2-ylcarbamoyl)phenoxy]cyclohexanecarboxylic acid
- trans-[4-(4-{5-[2-(tetrahydrofuran-3-yl)ethyl]-[1,3,4]thiadiazol-2-ylcarbamoyl}phenyl)cyclohexyl]-acetic acid
- trans-(4-{4-[5-(3-phenylcyclobutyl)[1,3,4]thiadiazol-2-ylcarbamoyl]phenyl}cyclohexyl)acetic acid
- {4-[4-(5-phenylacetylamino[1,3,4]thiadiazol-2-ylcarbamoyl)phenyl]cyclohexyl}acetic acid
- trans-{4-[4-(5-cyclopentyloxymethyl[1,3,4]thiadiazol-2-ylcarbamoyl)phenyl]cyclohexyl}acetic acid
- trans-(4-{4-[5-(3,5-difluorobenzyl)[1,3,4]thiadiazol-2-ylcarbamoyl]phenyl}cyclohexyl)acetic acid
- trans-(4-{4-[5-(4-hydroxycyclohexylmethyl)-[1,3,4]thiadiazol-2-ylcarbamoyl]phenyl}cyclohexyl)-acetic acid
- trans-(4-{4-[5-(tetrahydrofuran-2-ylmethoxymethyl)-[1,3,4]thiadiazol-2-ylcarbamoyl]phenyl}cyclohexyl)-acetic acid
- trans-{4-[4-(5-benzyl[1,3,4]thiadiazol-2-ylcarbamoyl)phenoxy]cyclohexyl}acetic acid
- trans-4-{4-[5-(tetrahydrofuran-2-ylmethoxymethyl)-[1,3,4]thiadiazol-2-ylcarbamoyl]phenoxy}cyclohexane-carboxylic acid
- trans-(4-{4-[5-(3-oxo-3-phenylpropyl)[1,3,4]thiadiazol-2-ylcarbamoyl]phenyl}cyclohexyl)acetic acid
- trans-(4-{4-[5-(3-hydroxy-3-phenylpropyl)[1,3,4]thiadiazol-2-ylcarbamoyl]phenyl}cyclohexyl)acetic acid
- trans-N-(5-benzyl[1,3,4]thiadiazol-2-yl)-4-{4-[(2-hydroxy-2-methylpropylcarbamoyl)methyl]cyclohexyl}-benzamide
- trans-N-(5-benzyl[1,3,4]thiadiazol-2-yl)-4-(4-carbamoylmethylcyclohexyl)benzamide
- trans-N-(5-benzyl[1,3,4]thiadiazol-2-yl)-4-{4-[(2,3-dihydroxypropylcarbamoyl)methyl]cyclohexyl}benzamide
- trans-N-(5-benzyl[1,3,4]thiadiazol-2-yl)-4-{4-[(2-morpholin-4-ylethylcarbamoyl)methyl]cyclohexyl}-benzamide
- trans-N-(5-benzyl[1,3,4]thiadiazol-2-yl)-4-{4-[(2-dimethylaminoethylcarbamoyl)methyl]cyclohexyl}benzamide
- trans-N-(5-benzyl[1,3,4]thiadiazol-2-yl)-4-{4-[(2-methoxyethylcarbamoyl)methyl]cyclohexyl}benzamide
- trans-N-(5-benzyl[1,3,4]thiadiazol-2-yl)-4-(4-{[([1,4]dioxan-2-ylmethyl)carbamoyl]-methyl}cyclohexyl)benzamide
- trans-4-{4-[5-(3,5-difluorobenzyl)[1,3,4]thiadiazol-2-ylcarbamoyl]phenoxy}cyclohexanecarboxylic acid
- trans-{4-[4-(5-phenylmethanesulfinylmethyl[1,3,4]thiadiazol-2-ylcarbamoyl)phenyl]cyclohexyl}acetic acid
- trans-{4-[4-(5-benzylsulfanylmethyl[1,3,4]thiadiazol-2-ylcarbamoyl)phenyl]cyclohexyl}acetic acid
- trans-(4-{4-[5-(3-phenylcyclobutyl)[1,3,4]thiadiazol-2-ylcarbamoyl]phenyl}cyclohexyl)acetic acid
- cis-4-[5-(5-cyclopentyloxymethyl[1,3,4]thiadiazol-2-ylcarbamoyl)pyridin-2-yloxy]cyclohexanecarboxylic acid
- trans-(4-{4-[5-(2-cyclopentylaminoethyl)[1,3,4]thia-diazol-2-ylcarbamoyl]phenyl}cyclohexyl)acetic acid
- cis-4-(4-{5-[2-(3-morpholin-4-ylcyclopentyl)ethyl]-[1,3,4]thiadiazol-2-ylcarbamoyl}phenoxy)cyclohexane-carboxylic acid
- cis-4-{4-[5-(3-oxo-3-phenylpropyl)[1,3,4]thiadiazol-2-ylcarbamoyl]phenoxy}cyclohexanecarboxylic acid
- cis-4-{4-[5-(3,5-difluorobenzyl)[1,3,4]thiadiazol-2-ylcarbamoyl]phenoxy}cyclohexanecarboxylic acid.

13. Process for preparing a compound of formula (I) according to any one of Claims 1 to 12, **characterized in that** the ester function of a compound chosen from (i) a compound of formula (V) 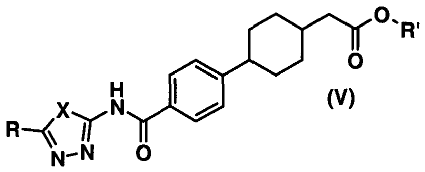
in which R represents a group -C(R1R2)n-Y and X, Y, R1, R2 and n are as defined in Claim 1 and R' represents a protecting group; and
(ii) a compound of formula (XXIII) : 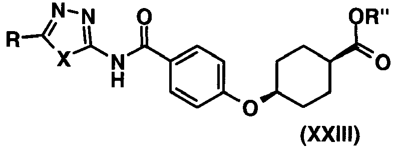
in which R represents a group -C(R1R2)n-Y and X, Y, R1, R2 and n are as defined in Claim 1 and R'' represents a protecting group,
is deprotected.

14. Preparation process according to Claim 13, **characterized in that** the production of the compound of formula (V), is performed by reacting (i) a compound of formula (II): 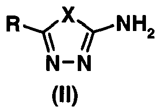
in which R represents a group -C(R1R2)n-Y and X, Y, R1, R2 and n are as defined in Claim 1,
with (ii) a compound of formula (III) 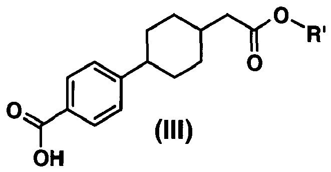
in which R' represents a protecting group.

15. Process for preparing a compound of formula (I) according to Claim 13, **characterized in that** the production of the compound of formula (XXIII), is performed by reacting (i) a compound of formula (II) : 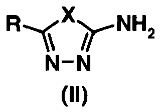
in which R represents a group -C(R1R2)n-Y and X, Y, R1, R2 and n are as defined in Claim 1,
with (ii) a compound of formula (XXI) 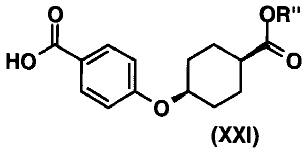
in which R'' represents a protecting group.

16. Compounds of formula (III): 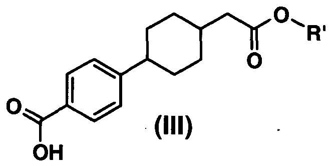
in which R' represents a protecting group.

17. Compounds of formula (V): 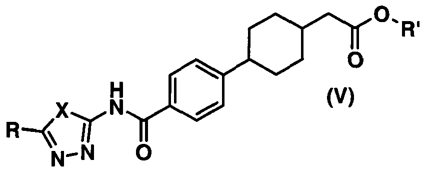
in which R represents a group -C(R1R2)n-Y with Y, R1, R2, n and X as defined in Claim 1 and R' representing a protecting group.

18. Compounds of formula (XXIII) : 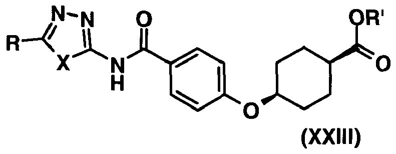
in which R represents a group -C(R1R2)n-Y with Y, R1, R2, n and X as defined in Claim 1 and R' representing a protecting group.

19. Compounds of formula (XXI) : 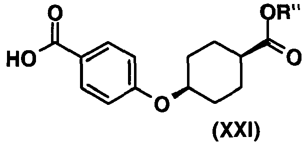
in which R' represents a protecting group, with the exclusion of the compound 4-(4-ethoxycarbonylcyclo-hexyloxy)benzoic acid.

20. Medicament, **characterized in that** it comprises a compound of formula (I) according to any one of Claims 1 to 12 or an addition salt of this compound with a pharmaceutically acceptable acid or base of the compound of formula (I).

21. Pharmaceutical composition, **characterized in that** it comprises a compound of formula (I) according to any one of Claims 1 to 12 or a pharmaceutically acceptable salt of this compound, and also at least one pharmaceutically acceptable excipient.

22. Use of a compound of formula (I) according to any one of Claims 1 to 12, for the preparation of a medicament for treating and/or preventing obesity, dyslipidaemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome, coronary diseases, hypertension, skin diseases, Alzheimer's disease, immunomodulatory diseases, infection with HIV, irritable bowel syndrome and certain cancers, and advantageously for the preparation of a medicament for treating or preventing obesity, dyslipidaemia, fasted glucose impairment conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), and metabolic syndrome.
